# EUROPEAN PATENT SPECIFICATION

(11) **EP 2 259 787 B1**
(45) Date of publication and mention of the grant of the patent: **23.12.2015**
(21) Application number: 09725853.7
(22) Date of filing: 25.03.2009
(51) Int. Cl.: A61K 31/4184, A61P 3/04, A61K 39/395, A61K 31/7088, A61K 31/7105, G01N 33/68, A61K 48/00

(54) **IKKI INHIBITOR THERAPIES AND SCREENING METHODS, AND RELATED IKKI DIAGNOSTICS**
IKKI-HEMMER-THERAPIEN UND -SCREENINGVERFAHREN SOWIE ENTSPRECHENDE IKKI-DIAGNOSEVERFAHREN
THÉRAPIES INHIBITRICES D'IKKI ET PROCÉDÉS DE CRIBLAGE, ET DIAGNOSTICS D'IKKI APPARENTÉS

(30) Priority: 25.03.2008 US 39295
(43) Date of publication of application: 15.12.2010
(73) Proprietor: The Regents of the University of Michigan, Ann Arbor, MI 48104-2592 (US)
(72) Inventor: SALTIEL, Alan, Ann Arbor MI 48105 (US); BAZUINE, Merlijn, Ann Arbor, MI 48105 (US); CHIANG, Shian-Huey, Ann Arbor MI 48105 (US); LUMENG, Carey, Ann Arbor MI 48105 (US)
(74) Representative: Witte, Weller & Partner Patentanwälte mbB
(86) International application number: PCT/US2009/038287
(87) International publication number: WO 2009/120801

(56) References cited:
- WO-A2-2004/097009
- US-A1- 2001 036 625
- US-A1- 2007 149 519
- WERNER ERIC D ET AL: "Disruptive yeast tri-hybrid identifies inducible IKK (IKKi) as a new insulin resistance kinase", DIABETES, AMERICAN DIABETES ASSOCIATION, US, vol. 53, no. Suppl.2, 8 June 2004 (2004-06-08), page A37, XP009150623, ISSN: 0012-1797
- YUAN M ET AL: "REVERSAL OF OBESITY- AND DIET-INDUCED INSULIN RESISTANCE WITH SALICYLATES OR TARGETED DISRUPTION OF IKKBETA", SCIENCE, AMERICAN ASSOCIATION FOR THE ADVANCEMENT OF SCIENCE, WASHINGTON, DC; US, vol. 293, no. 5535, 31 August 2001 (2001-08-31), pages 1673-1677, XP001018090, ISSN: 0036-8075, DOI: DOI:10.1126/SCIENCE.1061620
- BAMBOROUGH P ET AL: "5-(1H-Benzimidazol-1-yl)-3-alkoxy-2-thiop henecarbonitriles as potent, selective, inhibitors of IKK-epsilon kinase", BIOORGANIC & MEDICINAL CHEMISTRY LETTERS, PERGAMON, ELSEVIER SCIENCE, GB, vol. 16, no. 24, 15 December 2006 (2006-12-15), pages 6236-6240, XP025106628, ISSN: 0960-894X, DOI: DOI:10.1016/J.BMCL.2006.09.018 [retrieved on 2006-12-15]
- GAO Z ET AL.: 'Serine phosphorylation of insulin receptor substrate 1 by inhibitor kappa B kinase complex.' JBIOL CHEM. vol. 277, no. 50, 13 December 2002, pages 48115 - 48121
- VLADIMIR V. ET AL.: 'IKKi/IKKe plays a key role in integrating signals induced by pro- inflammatory stimuli.' J. BIOL. CHEM. vol. 278, no. 29, 2003, pages 26612 - 26619
- SHRESTHA S ET AL.: 'PTPIB inhibitor Ertiprotafib is also a potent inhibitor ofIkappaB kinase beta (IKK-beta).' BIOORG MED CHEM LETT. vol. 17, no. 10, 15 May 2007, pages 2728 - 30
- LALLI CA ET AL.: 'Statin modulates insulin signaling and insulin resistance in liver and muscle of rats fed a high-fat diet.' METABOLISM CLINICAL AND EXPERIMENTAL. vol. 57, no. 1, January 2008, pages 57 - 65
- NANDINI KISHORE ET AL: "IKK-i and TBK-1 are enzymatically distinct from the homologous enzyme IKK-2: Comparative analysis of recombinant human IKK-i, TBK-1, and IKK-2", JOURNAL OF BIOLOGICAL CHEMISTRY, AMERICAN SOCIETY FOR BIOCHEMISTRY AND MOLECULAR BIOLOGY, vol. 277, no. 16, 11 February 2002 (2002-02-11), pages 13840-13847, XP009150760, ISSN: 0021-9258, DOI: 10.1074/JBC.M110474200

## Description

### FIELD OF THE INVENTION

The present invention relates to diagnostics, screening methods, and treatment methods related to obesity, insulin resistance, diabetes, weight loss, and related disorders. In particular, the present invention provides methods of treating such conditions with IKKi inhibitors, methods of diagnosing such conditions based on IKKi status, and methods of screening candidate IKKi inhibitors.

### BACKGROUND

Generally, obesity is defined as an excess of adipose tissue. Clinically, it is generally defined as that amount of adiposity that imparts a health risk. Even mild obesity, at 20% over desirable weight according to standard height-weight charts, may increase the risk for disease and premature death. While the etiology of obesity and diabetes is not entirely overlapping, it is now amply clear that both share appreciable biochemical and physiological components.

The incidence of the metabolic disorders of diabetes and obesity has reached epidemic levels. It has been estimated that over 120 million Americans are clinically over-weight and more than ten million Americans are diagnosed with diabetes every year. Moreover, obesity and diabetes can cause or contribute to the development of, or at least affect the treatment of, other diseases and disorders such as cardiovascular diseases, stroke, hypertension, and kidney failure. The combined economic burden of diabetes and obesity and the co-morbidities associated with these disorders is estimated to be over $100 billion a year. Obesity and diabetes have a major impact on human health and the various national healthcare systems all over the world.

Recently launched weight-loss drugs have failed or have demonstrated limited efficacy and undesirable side effects. Similarly, despite a tremendous medical need, the pharmaceutical industry has realized only limited success developing therapeutics to manage diabetes. The most common therapeutics (sulfonylureas) are not effective and the most promising new drugs (thiazolidinediones) have demonstrated rare but fatal side effects. Thus, there is an urgent need for a more comprehensive understanding of the molecular basis of obesity and diabetes, for diagnosis tests that allow early detection of predispositions to the disorders, and for more effective pharmaceuticals for preventing and treating the diseases without undesirable side effects.

The WO 2004/097009 discloses methods for the use of agents that increase or decrease the expression or activity of IKKi to regulate specific cellular processes.

The US 2007/0149519 discloses compounds and compositions for a use in the treatment of diseases associated with inappropriate IKK3 activity.

The US 2001/0036625 discloses a method of identifying a compound capable to bind IKK-β or modulate IKK-β activity.

Gao et al. (2002), JBC, Vol. 277, No. 50, pp. 48115-48121, deals with the serine phosphorylation of insuline receptor substrate 1 by inhibitor κB kinase complex.

Kravchenko et al. (2003), JBC, Vol. 278, No. 29, pp. 26612-26619, concerns the role of IKKi/IKKε in integrating signals induced by pro-inflammatory stimuli.

Shrestha et al. (2007), Bioorg. Med. Chem. Lett., Vol. 17, pp. 2728-2730 disclose the PTP1B inhibitor Ertiprotafib as an inhibitor of IkB kinase β.

Lalli et al. (2008), Metabol. Clin. Exp., Vol. 57, pp. 57-65 disclose the modulation of insulin signaling and resistance in rats by statin.

Werner et al. (2004), Diabetes, Vol. 53, Suppl. 2, p. A37 disclose IKKi as an insuline resistance kinase.

Yuan et al. (2001), Science, Vol. 293, pp. 1673-1676 disclose the reversal of obesity-and diet-induced insulin resistance with salicylates or targeted disruption of IKKβ**.**

Bamborough et al. (2006), Bioorg. Med. Chem. Lett. disclose specific IKK-ε inhibitors.

Kishore et al. (2002), JBC, Vol. 277, No. 16, disclose the results of a comparative analysis of recombinant human IKK-i, TBK-1, and IKK-2.

### SUMMARY OF THE INVENTION

Disclosed are diagnostics, screening methods, and treatment methods related to obesity, insulin resistance, diabetes, weight loss, and related disorders. In particular, the present invention provides methods of treating obese body composition with IKKi inhibitors, methods of diagnosing an elevated risk for a condition associated with impaired insulin receptor signalling based on IKKi status, and methods of screening candidate IKKi inhibitors.

Disclosed are methods of treatment comprising: administering an IKKi inhibitor to a subject with a condition associated with impaired insulin receptor signaling, wherein the administering causes a reduction in one or more symptoms of the condition.

The impaired insulin receptor signaling might be in the subject's adipocyte cells or adipose tissue macrophage cells, or liver or muscle cells. The impaired insulin receptor signaling might cause the subject to have impaired glucose metabolism. The administering might cause an increase in glucose metabolism by adipocytes and adipose tissue macrophages of the subject. The increase in glucose metabolism might be caused by increased insulin receptor signaling in response to insulin. The administering might cause a reduction of body fat in the subject (e.g., the size of adipocytes in the subject are reduced). In certain embodiments, the administration causes the patient to lose at least 10 pounds (e.g., 10 ... 15 ... 20 ... 35 ... 60 ... 100 ... or 200 or more pounds). The administration might cause at least a 5% reduction in the patient's body weight (e.g., at least 7% ... 10% ... 20% ... 30% ... 50% ... 75% reduction or more).

The condition treated might be obesity. The condition treated might be diabetes (e.g., type I, or type II, or both types I and II). The condition treated might be insulin resistance. The subject might not have diabetes type I or type II. The IKKi inhibitor might inhibit the insulin receptor phosphorylation activity of IKKi. The IKKi inhibitor might inhibit the insulin receptor phosphorylation activity of IKKi at the serine in the human insulin receptor sequence VKTVNES (SEQ ID NO: 15) or at the corresponding serine in the insulin receptor sequences of another species (e.g., mouse, cat, dog, rat, horse, cow, etc.) which can be located, for example, by performing a sequence alignment. The IKKi inhibitor might inhibit the insulin receptor phosphorylation activity of IKKi at the serine in the human insulin receptor sequence VKTVNES (SEQ ID NO: 15), or related species, but does not inhibit one or more other activities of IKKi (e.g., phosphorylation of one or more of the IkB proteins). The IKKi inhibitor might inhibit the phosphorylations of other proteins that directly or indirectly contribute to disregulation of glucose or lipid homeostasis.

The IKKi inhibitor might comprise a benzimidazol substituted thiopene derivative. The IKKi inhibitor might operate through RNA interference. The IKKi inhibitor might be an siRNA or antisense oligonucleotide. The IKKi inhibitor might be an anti-IKKi antibody (e.g., monoclonal antibody or antibody fragment). The IKKi inhibitor might be an anti-IKKi antibody specific for the TLR4 phosphorylated form of IKKi.

Disclosed are methods of reducing body fat of a subject comprising: administering an IKKi inhibitor to a subject under conditions such that there is a reduction in body fat of the subject. The reduction in body fat might be a result of increased glucose metabolism caused by the IKKi inhibitor. The reduction in body fat might be caused by increased insulin receptor signaling.

The present invention provides diagnostic methods comprising: a) measuring the IKKi protein or mRNA expression level in a sample from a subject, and b) determining if the subject has, or has an elevated risk for, a condition associated with impaired insulin receptor signaling, wherein an elevated IKKi protein or mRNA expression level indicates that the subject has, or is at elevated risk for, the condition.

The sample might comprise adipocytes, adipose tissue macrophages, or adipose tissue from the subject. In further embodiments, the measuring comprises the use of an anti-IKKi antibody or antibody fragment. In particular embodiments, the measuring comprises the use of an IKKi nucleic acid probe (e.g., at least a portion of SEQ ID NO: 13). In further embodiments, the condition diagnosed is obesity or predisposition to obesity. In further embodiments, the condition diagnosed is diabetes type I or type II or both. In some embodiments, the condition diagnosed is insulin resistance.

Disclosed are diagnostic methods comprising: a) determining the level of insulin receptor phosphorylation in a sample from a subject, and b) determining if the subject has, or has an elevated risk for, a condition associated with impaired insulin receptor signaling, wherein an elevated level of insulin receptor phosphorylation in the sample indicates that the subject has, or is at elevated risk for, the condition.

In some embodiments, the level of insulin receptor phosphorylation is compared to a standard, wherein the standard is either known to be associated with the condition or is from a healthy individual without the condition. The sample might comprise adipocytes, adipose tissue macrophages, or adipose tissue from the subject. In further embodiments, the condition diagnosed is obesity. The condition diagnosed might be diabetes type I or type II or both. The condition diagnosed might be insulin resistance.

Further disclosed are diagnostic methods comprising: a) determining the level of TRL4 mediated IKKi phosphorylation in a sample from a subject, and b) determining if the subject has, or has an elevated risk for, a condition associated with impaired insulin receptor signaling, wherein an elevated level of TRL4 mediated IKKi phosphorylation of IKKi in the sample indicates that the subject has, or is at elevated risk for, the condition.

The present invention provides cell-free methods of screening a candidate agent comprising: a) combining IKKi (e.g., full protein or active peptide fragments), an insulin receptor, labeled phosphorous atoms, and a candidate IKKi inhibitor under conditions such that the IKKi can transfer the labeled phosphorous atoms onto the insulin receptor if not inhibited by the candidate IKKi inhibitor; and b) determining if the candidate IKKi inhibitor inhibits the IKKi from phosphorylating the insulin receptor.

The determining might comprise evaluating whether the candidate IKKi inhibitor inhibits the IKKi from phosphorylating the serine in the insulin receptor in the sequence VKTVNES (SEQ ID NO: 15) or at the serine in related non-human insulin sequences. The methods may further comprise step c) administering the candidate IKKi inhibitor to an animal and determining if the IKKi inhibitor promotes glucose metabolism in the animal. The animal might be a model for obesity, diabetes, or insulin resistance. In other embodiments, the determining comprising weighing the animal before and after treatment.

The IKKi inhibitor may comprise a benzimidazol substituted thiopene derivative. In further embodiments, the IKKi inhibitor operates through RNA interference. The IKKi inhibitor might be an siRNA or antisense oligonucleotide. The IKKi inhibitor might be an anti-IKKi antibody (e.g., monoclonal antibody or antibody fragment). The IKKi inhibitor might be an anti-IKKi antibody specific for the TLR4 phosphorylated form of IKKi.

Disclosed are methods of screening a candidate agent comprising: a) contacting a cell (e.g., adipoctye, macrophage, or 3T3-L1 fibroblast or other adipocyte cell culture line) with a candidate IKKi inhibitor, wherein the cell comprises insulin receptors; and b) determining if the candidate IKKi inhibitor prevents, or reduces the level of, phosphorylation of the insulin receptors in the cell. The methods may further comprise a step of lysing the cell to generate a cell lysate prior to the determining step. The determining may comprise examining the level of phosphorylation at the serine in the insulin receptor sequence VKTVNES (SEQ ID NO: 15). The cell might be an adipocyte or adipose tissue macrophage.

The determining may comprise the use of an antibody, or antibody fragment, that recognizes the phosphorylated form, or the un-phosphorylated forms, of the insulin receptors. The determining may comprise the use of an antibody, or antibody fragment, that recognizes the form of the insulin receptor that is phosphorylated at the serine in the insulin receptor sequence VKTVNES (SEQ ID NO: 15) or at corresponding serine in non-human insulin receptor sequences. The determining may comprise the use of an antibody or antibody fragment that recognizes the form of the insulin receptors that is not phosphorylated at the serine in the insulin receptor sequence VKTVNES (SEQ ID NO: 15) or at the corresponding serine in non-human insulin receptor sequences.

The methods may further comprise step c) administering the candidate IKKi inhibitor to an animal and determining if the IKKi inhibitor promotes glucose metabolism in the animal. The animal may be a model for obesity, diabetes, or insulin resistance. The determining may comprise weighing the animal before and after treatment.

In certain embodiments, the candidate IKKi inhibitor comprises a benzimidazol substituted thiopene derivative. In further embodiments, the candidate IKKi inhibitor operates through RNA interference. In other embodiments, the IKKi inhibitor is an siRNA or antisense oligonucleotide. In additional embodiments, the candidate IKKi inhibitor is an anti-IKKi antibody. The cell may be treated with an IKKi inducer prior to the contacting step. The IKKi inducer may be selected from the group consisting of: tumor necrosis factor (TNF), lipopolysaccharide (LPS), interleukin-1 (IL-I), interleukin-6 (IL-6), interferon-gamma, and phorbol myristate.

Disclosed are methods of screening a candidate agent comprising: a) contacting a cell with a candidate IKKi inhibitor, wherein the cell is an adipocyte or adipose tissue macrophage, and wherein the cell comprises activated IKKi proteins; and b) determining if the IKKi inhibitor promotes glucose metabolism in the cell. In particular embodiments, prior to the contacting step, the cell is first contacted with an IKKi inducer. The IKKi inducer may be selected from the group consisting of: LPS, IL-I, IL-6, interferon-gamma, and phorbol myristate.

The determining if the IKKi inhibitor promotes glucose metabolism in the cell may comprise measuring the uptake of glucose by the cell. The determining if the IKKi inhibitor promotes glucose metabolism in the cell may comprise measuring the state of phosphorylation of insulin receptors in the cell. The determining if the IKKi inhibitor promotes glucose metabolism in the cells may comprise measuring the state of phosphorylation of a protein selected from the group consisting of: Aps, Cbl, and TCl0.

The determining if the IKKi inhibitor promotes glucose metabolism in the cell may comprise measuring the ability of GLUT4 to transport glucose. The determining if the IKKi inhibitor promotes glucose metabolism in the cells may comprise measuring the size of the cell compared to a control cell. The methods further comprise step c) administering the candidate IKKi inhibitor to an animal and determining if the IKKi inhibitor promotes glucose metabolism in the animal. The animal is a model for obesity, diabetes, or insulin resistance. The determining comprising weighing the animal before and after treatment.

Disclosed is a method of treating conditions associated with impaired insulin, comprising: providing a subject experiencing or at risk for impaired insulin signaling and administering to the subject a therapeutically effective dose of an IKKi-inhibiting agent, wherein the administration results in improved insulin signaling in the subject. The impaired insulin signaling might occur in such as adipocyte cells, adipose tissue macrophage cells, adipose tissue, liver cells, and liver tissue. The subject might experience or is at risk of experiencing a condition such as obesity, diabetes, and insulin resistance. The administering of an IKKi-inhibiting agent might result in an outcome of increased glucose metabolism, reduction in body fat, lack of increase in body fat, increased insulin receptor signaling, decreased level of insulin receptor phosphorylation, reduction in or prevention of chronic inflammation in liver, reduction in or prevention of chronic inflammation in adipose tissue, reduction in or prevention of hepatic steatosis, promotion of metabolic energy expenditure, reduction in circulating free fatty acids, and/or reduction in cholesterol. The decreased level of insulin receptor phosphorylation might occur at the serine residue of insulin receptor sequence VKTVNES (SEQ ID NO: 15). The IKKi-mediated phosphorylation of IκB in the subject might be unaffected by the IKKi-inhibiting agent. The IKKi inhibitor might comprise an agent such as a benzimidazol-substituted thiopene derivative, an siRNA, an antisense oligonucleotide, a non-phospho-specific anti-IKKi antibody, and a phospho-specific anti-IKKi antibody.

Also disclosed is a method of reducing body fat or preventing increase in body fat in a subject, comprising: providing a subject experiencing or at risk of overweight or obese body composition, and administering to the subject a therapeutically effective dose of an IKKi-inhibiting agent, wherein the administration results in reduction of or prevention of increase in body fat in the subject. The subject might be experiencing or is at risk of experiencing a condition such as diabetes and insulin resistance. The administering of an IKKi-inhibiting agent might result in an outcome such as increased glucose metabolism, increased insulin receptor signaling, decreased level of insulin receptor phosphorylation, reduction in or prevention of chronic inflammation in liver, reduction in or prevention of chronic inflammation in adipose tissue, reduction in or prevention of hepatic steatosis, promotion of metabolic energy expenditure, reduction in circulating free fatty acids, and/or reduction in cholesterol. The decreased level of insulin receptor phosphorylation might occur at the Ser of insulin receptor sequence VKTVNES (SEQ ID NO: 15). The IKKi-mediated phosphorylation of IkB in the subject is unaffected by the IKKi-inhibiting agent. The IKKi inhibitor might comprise an agent such as a benzimidazol-substituted thiopene derivative, an siRNA, an antisense oligonucleotide, a non-phospho-specific anti-IKKi antibody, and a phosphor-specific anti-IKKi antibody.

Further disclosed is a diagnostic method, comprising: providing a sample from a subject, and measuring the level in the sample of a molecule such as IKKi protein, IKKi transcript, phosphorylated insulin receptor, and phosphorylated IKKi wherein the IKKi phosphorylation is mediated by TLR4, and determining if the subject has or has an elevated risk for a condition associated with impaired insulin receptor signaling, wherein an elevated level of said molecule indicates that said subject has, or is at elevated risk for, a condition associated with impaired insulin receptor signaling. The sample might comprise adipocytes, adipose tissue macrophages, adipose tissue, liver cells, or liver tissue. The measuring might comprise the use of an agent specific to the molecule. This agent may include a nucleic acid probe, a non-phospho-specific antibody, and/or a phospho-specific antibody. The level of the molecule might be compared to a standard, wherein the standard is either known to be associated with the condition or is from a healthy individual without the condition or from the subject at a prior time period. The condition might be obesity, diabetes, and/or insulin resistance.

Disclosed is a method of identifying an IKKi-inhibiting agent, comprising: combining a polypeptide comprising IKKi, a polypeptide comprising an insulin receptor, labeled phosphorous atoms, and a candidate IKKi inhibitor under conditions sufficient to promote phosphorylation of said insulin receptor by the IKKi polypeptide in absence of the candidate inhibitor, and determining the activity of the IKKi polypeptide with regard to phosphorylation of the insulin receptor. The decreased level of insulin receptor phosphorylation might occur at the serine residue of insulin receptor sequence VKTVNES (SEQ ID NO: 15). The method may further comprise a step of administering the candidate IKKi inhibitor to an animal and determining whether the candidate IKKi inhibitor promotes glucose metabolism in the animal.

Also disclosed is a method of identifying an IKKi-inhibiting agent, comprising: providing a cell or cell Iy sate comprising insulin receptors, contacting the cell with a candidate IKKi inhibitor, and determining whether the candidate IKKi inhibitor affected a property such as the rate of glucose metabolism and/or the level of phosphorylation of said insulin receptors. The determination of whether the IKKi inhibitor may affect the rate of glucose metabolism comprises measuring a feature such as uptake of glucose by the cell, the phosphorylation state of insulin receptors, the phosphorylation state of APS, the phosphorylation state of CbI, the phosphorylation state of TC 10, the ability of GLUT4 to transport glucose, the translocation of GLUT4 to the plasma membrane, and/or the size of the cell relative to a control cell. The IKKi inhibitor may comprise an agent such as a benzimidazol-substituted thiopene derivative, an siRNA, an antisense oligonucleotide, a non-phospho-specif[iota]c anti-IKKi antibody, and a phospho-specific anti-IKKi antibody. The cell may be treated with an IKKi-inducing agent prior to the contacting step. The method may further comprise the step of administering the candidate IECKi inhibitor to an animal and determining whether the candidate IBCKi inhibitor promotes glucose metabolism in the animal.

Conditions and disease states which may be treated by methods and compositions of the present invention include but are not limited to diabetes mellitus, type I diabetes, type II diabetes, gestational diabetes, metabolic syndrome, metabolic syndrome X, syndrome X, insulin resistance syndrome, Reaven's syndrome, CHAOS, and malnutrition-related diabetes mellitus.

Lipid metabolic conditions and disease states which may be treated using methods and compositions of the present invention include but are not limited to lipodystrophy, congenital generalized lipodystrophy (Beradinelli-Seip syndrome), familial partial lipodystrophy, acquired partial lipodystrophy (Barraquer-Simons syndrome), acquired generalized lipodystrophy, centrifugal abdominal lipodystrophy (Lipodystrophia centrifugalis abdominalis infantilis), lipoatrophia annularis (Ferreira-Marques lipoatrophia), localized lipodystrophy, HIV-associated lipodystrophy, hypercholesterolemia, hyperlipidemia, obesity, hypertriglyceridemia. Lipid metabolic conditions may occur in concert with or in absence of conditions such as vascular disease, hypertension, atherosclerosis, arteriosclerosis, peripheral vascular disease (PVD), peripheral arterial disease (also known as peripheral artery disease or PAD), claudication, intermittent claudication, vascular diseases, peripheral arterial occlusive disease (PAOD), coronary artery disease (CAD), cardiovascular disease, obesity, metabolic syndrome, and critical limb ischemia.

The methods and treatments find use in the treatment of high total cholesterol (hypercholesterolemia). Primary causes of hypercholesterolemia include but are not limited to high-fat diet, smoking or tobacco use, hypothyroidism, renal disease, liver disease, use of progestins, use of anabolic steroids, and use of glucocorticoids. Hypercholesterolemia may be polygenic or familial. Known familial hypercholesterolemia diseases include but are not limited to familial ligand defective apoB-100 (FLDB) and autosomal recessive hypercholesterolemia.

The methods and compositions find use in the treatment of hepatic steatosis disease, also referred to as fatty liver disease. Fatty liver disease can range from fatty liver alone (steatosis) to fatty liver associated with inflammation (steatohepatitis). This condition can occur with the use of alcohol (alcohol-related fatty liver) or in the absence of alcohol (nonalcoholic fatty liver disease [NAFLD]). Other factors that may lead to fatty liver disease include but are not limited to drugs (eg, amiodarone, tamoxifen, methotrexate), alcohol, metabolic abnormalities (eg, galactosemia, glycogen storage diseases, homocystinuria, tyrosemia), nutritional status (e.g., overnutrition, severe malnutrition, total parenteral nutrition [TPN], starvation diet), or other health problems (eg, celiac sprue, Wilson disease). Individuals genetically predisposed to fatty liver disease may exhibit normal or underweight body composition.

The present invention finds use in the treatment or prevention of overweight and obesity. The most widely accepted clinical definition of obesity is the World Health Organization (WHO) criteria based on BMI. Under this convention for adults, grade 1 overweight (commonly and simply called overweight) is a BMI of 25-29.9 kg/m². Grade 2 overweight (commonly called obesity) is a BMI of 30-39.9 kg/m². Grade 3 overweight (commonly called severe or morbid obesity) is a BMI greater than or equal to 40 kg/m². The surgical literature often uses a different classification to recognize particularly severe obesity. In this setting, a BMI greater than 40 kg/m² is described as severe obesity, a BMI of 40-50 kg/m² is termed morbid obesity, and a BMI greater than 50 kg/m² is termed super obese. The definition of obesity in children involves BMIs greater than the 85th (commonly used to define overweight) or the 95th (commonly used to define obesity) percentile, respectively, for age-matched and sex-matched control subjects. Secondary causes of obesity include but are not limited to hypothyroidism, Cushing syndrome, insulinoma, hypothalamic obesity, polycystic ovarian syndrome, genetic syndromes (eg, Prader-Willi syndrome, Alström syndrome, Bardet-Biedl syndrome, Cohen syndrome, Börjeson-Forssman-Lehmann syndrome, Fröhlich syndrome), growth hormone deficiency, oral contraceptive use, medication-induced obesity (e.g., phenothiazines, sodium valproate, carbamazepine, tricyclic antidepressants, lithium, glucocorticoids, megestrol acetate, thiazolidine diones, sulphonylureas, insulin, adrenergic antagonists, serotonin antagonists [especially cyproheptadine]), eating disorders (especially binge-eating disorder, bulimia nervosa, night-eating disorder), hypogonadism, pseudohypoparathyroidism, and obesity related to tube feeding.

The methods and compositions may be used to treat subjects having one or more of the above diseases or conditions, but lacking at least one of the following diseases or conditions: asthma, bronchitis, lung inflammation, osteoarthritis, juvenile arthritis, rheumatoid arthritis, spondylo arthopathies, gouty arthritis, chronic granulomatous diseases such as tuberculosis, leprosy, sarcoidosis, and silicosis, nephritis, amyloidosis, ankylosing spondylitis, chronic bronchitis, scleroderma, systemic lupus erythematosus, polymyositis, appendicitis, inflammatory bowel disease, Crohn's disease, gastritis, irritable bowel syndrome, ulcerative colitis, colorectal cancer, Sjorgen's syndrome, Reiter's syndrome, psoriasis, pelvic inflammatory disease, orbital inflammatory disease, thrombotic disease, menstrual cramps, tendinitis, bursitis, psoriasis, eczema, bums, dermatitis and inappropriate allergic responses to environmental stimuli such as poison ivy, pollen, insect stings and certain foods, including atopic dermatitis and contact dermatitis, migraine headaches, periarteritis nodosa, thyroiditis, aplastic anemia, Hodgkin's disease, sclerodoma, rheumatic fever, myasthenia gravis, sarcoidosis, nephrotic syndrome, Behcet's syndrome, polymyositis, gingivitis, hypersensitivity, conjunctivitis, swelling occurring after injury, lipopolysaccharide-induced septic shock, tissue regeneration, neurodegenerative disease (e.g., Alzheimer's Disease), tissue rejection, osteoporosis, cachexia, and neurodegeneration. In some embodiments, methods and compositions of the present invention are used to treat subjects not in need of tissue regeneration. In some embodiments, methods and compositions of the present invention are used to treat subjects lacking cell proliferative disorders such as, for instance, benign prostate hyperplasia, familial adenomatosis, polyposis, neurofibromatosis, psoriasis, pulmonary fibrosis, and arthritis glomerulonephritis.

The methods and compositions may be used to treat subjects lacking at least one of the following cancers: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; hematopoietic tumors of lymphoid lineage, including leukemia, acute lymphocytic leukemia, acute lymphoblastic leukemia, B-cell lymphoma, T- cell-lymphoma, Hodgkin's lymphoma, non- Hodgkin's lymphoma, hairy cell lymphoma and Burkett's lymphoma ; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuro-blastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.

In some embodiments, methods of the present invention comprise testing a subject for a disease or condition such as impaired insulin signaling, obesity, diabetes, insulin resistance, high cholesterol, metabolic syndrome, hepatic stenosis, chronic inflammation in liver, and chronic inflammation in adipose tissue, followed by administering an IKKi-inhibiting agent. In some embodiments, methods of the present invention comprise administering to a subject an IKKi- inhibiting agent, followed by testing the subject for a disease or a condition such as impaired insulin signaling, obesity, diabetes, insulin resistance, high cholesterol, metabolic syndrome, hepatic stenosis, chronic inflammation in liver, and chronic inflammation in adipose tissue. In some embodiments, methods of the present invention comprise testing a subject for a disease or condition such as impaired insulin signaling, obesity, diabetes, insulin resistance, high cholesterol, metabolic syndrome, hepatic stenosis, chronic inflammation in liver, and chronic inflammation in adipose tissue, followed by administering an IKKi-inhibiting agent, followed by a second round of testing for a disease or condition such as impaired insulin signaling, obesity, diabetes, insulin resistance, high cholesterol, metabolic syndrome, hepatic stenosis, chronic inflammation in liver, and chronic inflammation in adipose tissue (e.g., to monitor the effect of the treatment). In some embodiments, methods of the present invention comprise testing a subject for a disease or condition such as impaired insulin signaling, obesity, diabetes, insulin resistance, high cholesterol, metabolic syndrome, hepatic stenosis, chronic inflammation in liver, and chronic inflammation in adipose tissue, followed by administering an IKKi-inhibiting agent, followed by a second round of testing for a disease or condition such as impaired insulin signaling, obesity, diabetes, insulin resistance, high cholesterol, metabolic syndrome, hepatic stenosis, chronic inflammation in liver, and chronic inflammation in adipose tissue, and a second administration of IKKi-inhibiting agent, with this second administration being modified in dose, duration, frequency, or administration route in a manner dependent upon the results of the prior testing.

Disclosed is further the use of an IKKi-inhibiting agent in the manufacture of a medicament for the treatment of a condition such as impaired insulin signaling, obesity, diabetes, insulin resistance, high cholesterol, metabolic syndrome, hepatic stenosis, chronic inflammation in liver, and chronic inflammation in adipose tissue.

### BRIEF DESCRIPTION OF THE DRAWINGS

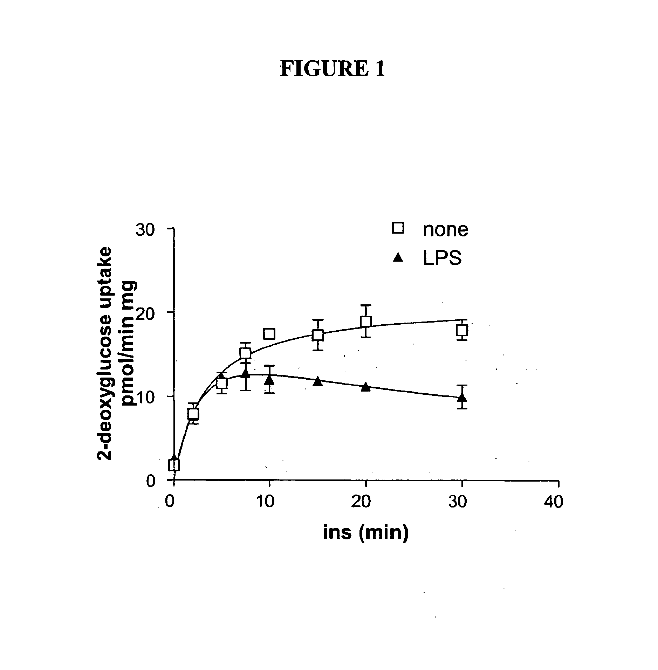

Figure 1 shows that LPS blocks insulin-stimulated glucose uptake in 3T3L1 adipocytes. Adipocytes were pretreated with or without LPS, and then treated with insulin and 2-deoxyglucose uptake was assessed at various times as indicated in the Figure.

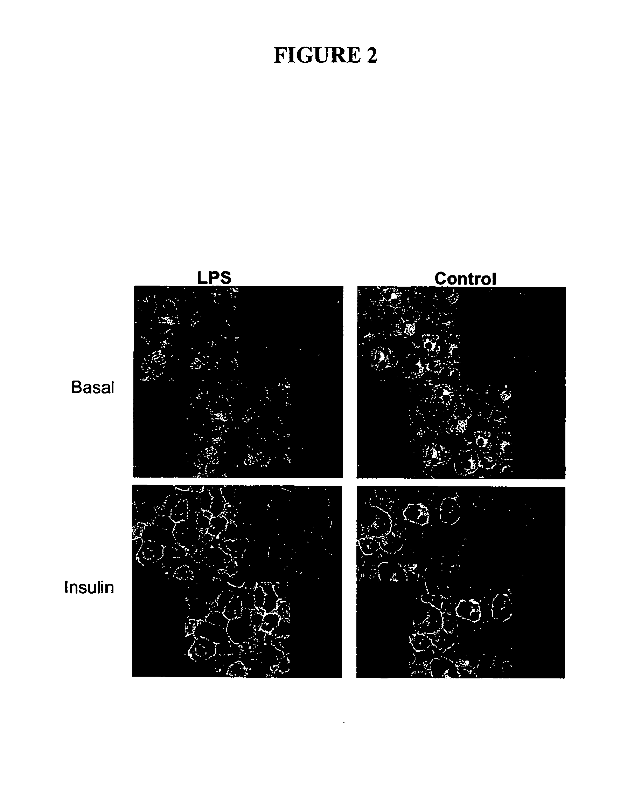

Figure 2 shows that LPS blocks insulin-stimulated translocation of the glucose transporter Glut4. Cells were transfected with a Glut4-eGFP/Myc reporter gene, to monitor the translocation of the protein to the cell surface in response to insulin. Preincubation with LPS attenuated insulin action.

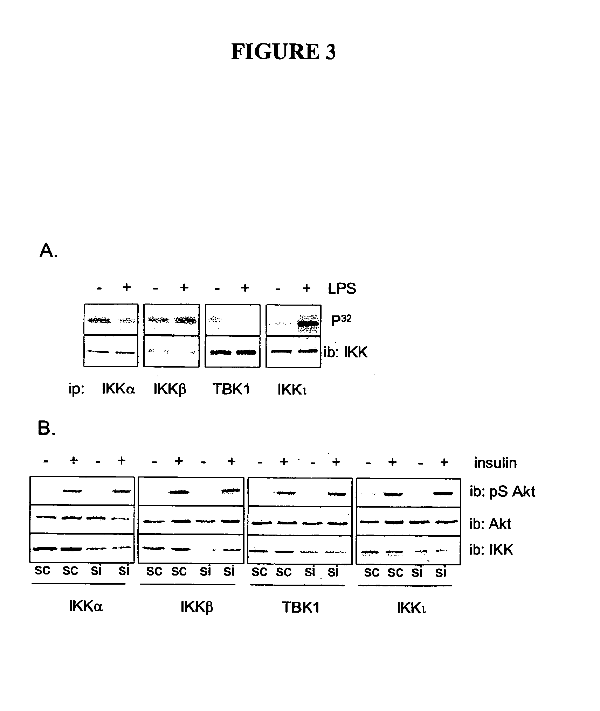

**Figure 3** shows the role of IKK isoforms in LPS action. Figure 3A shows LPS increases the phosphorylation of IKK isoforms α, β, and i. Figure 3B shows knockdown of IKK isoforms by siRNA does not affect activation of Akt by insulin.

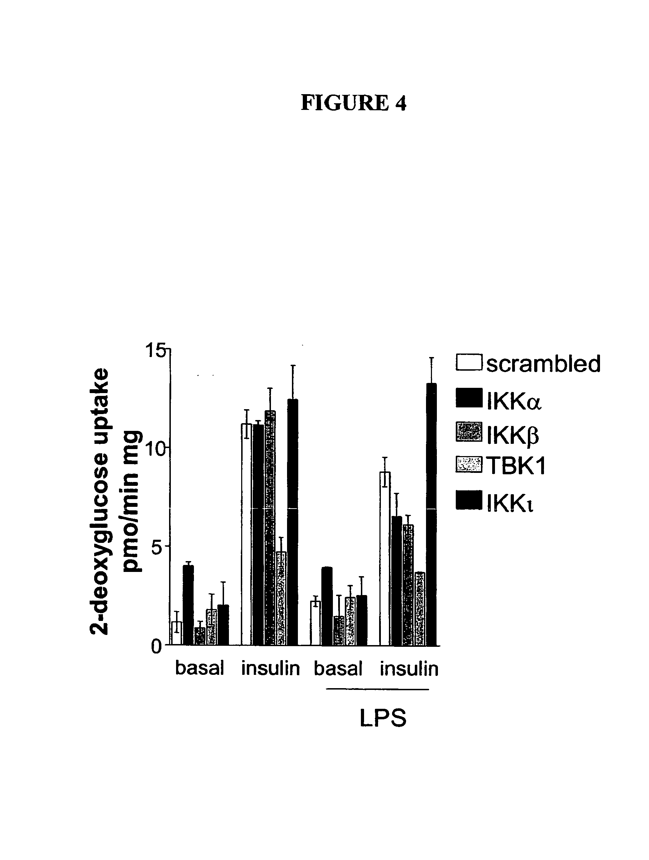

**Figure 4** shows inhibition of insulin-stimulated glucose uptake by LPS is prevented by knockdown of Ikki. 3T3L1 adiopocytes were transfected with the indicated siRNA oligos, and the effect of LPS on insulin-stimulated glucose transport was assessed.

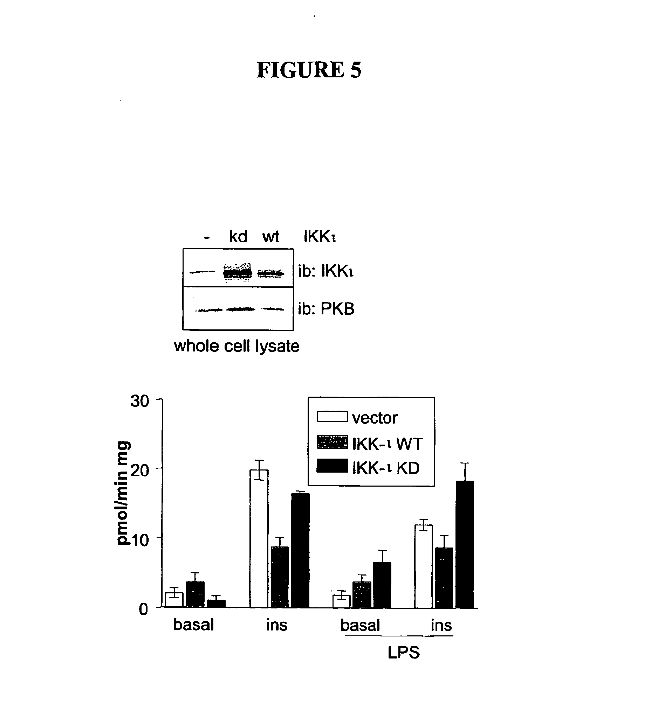

**Figure 5** shows overexpression of dominant negative Ikki blocks the inhibitory effects of LPS. 3T3L1 adipocytes were transfected with wt or kinase-inactive Ikki and the inhibitory effects of LPS were assessed on insulin-stimulated glucose uptake.

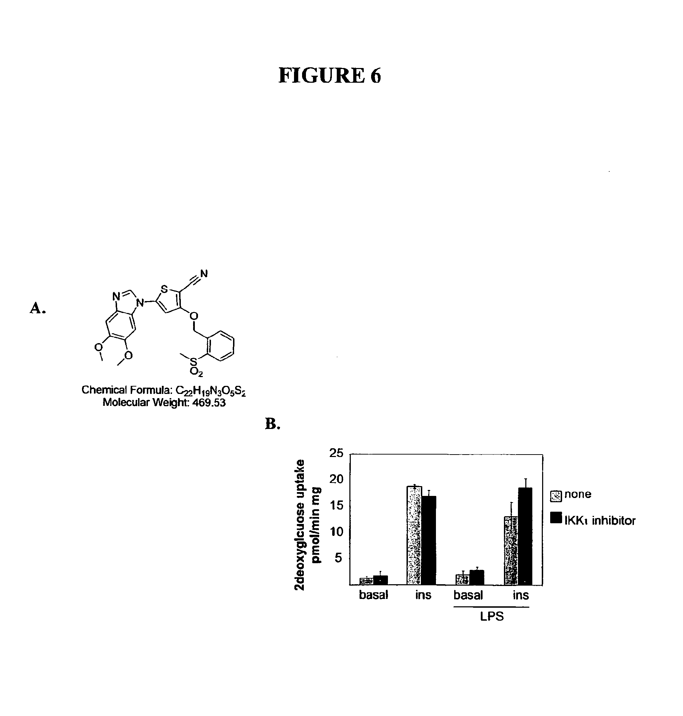

**Figure 6a** shows the IKKi inhibitor 5-(5,6-Dimethoxy-1*H-*benzimidazol-1-yl)-3-[[2-(methylsulfonyl)phenyl]methoxy]-2-thiophenecarbonitrile. **Figure 6b** shows that an inhibitor of IKKi prevents LPS decreases in insulin stimulated glucose uptake. In particular, 3T3L1 adipocytes were treated with 50 nM of 5-(5,6-Dimethoxy-1*H*-benzimidazol-1-yl)-3-[[2-(methylsulfonyl)phenyl]methoxy]-2-thiophenecarbonitrile prior to treatment with LPS and insulin.

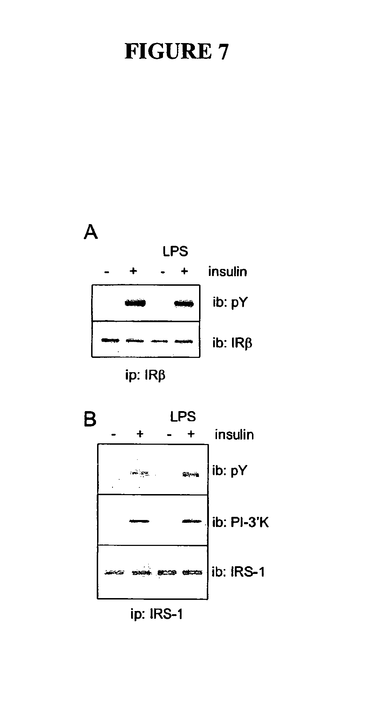

**Figure 7** shows that treatment of adipocytes with LPS had no effect on insulin-stimulated tyrosine phosphorylation of the insulin receptor (InsR) or IRS-1 (Figure 7A), as detected by anti-phosphotyrosine immunoblotting, nor was there a reduction in the amount of PI-3' kinase that co-immunoprecipitated with IRS-1 after insulin stimulation (Figure 7B).

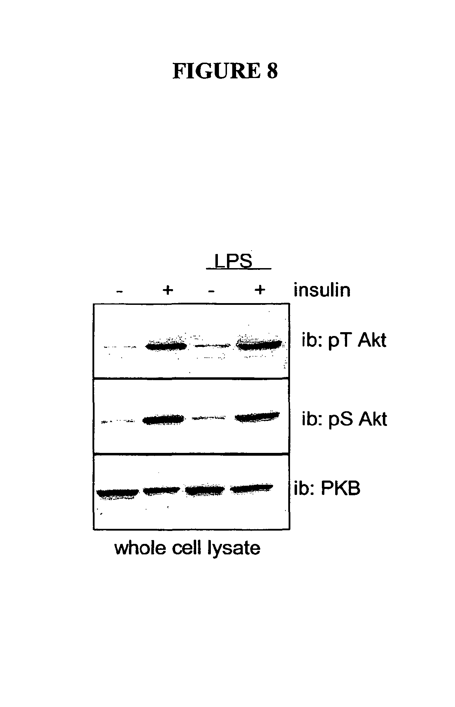

**Figure 8** shows a Western blot from Example 1 that shows activation of the protein kinase Akt by insulin was not affected by LPS pre-treatment of cells.

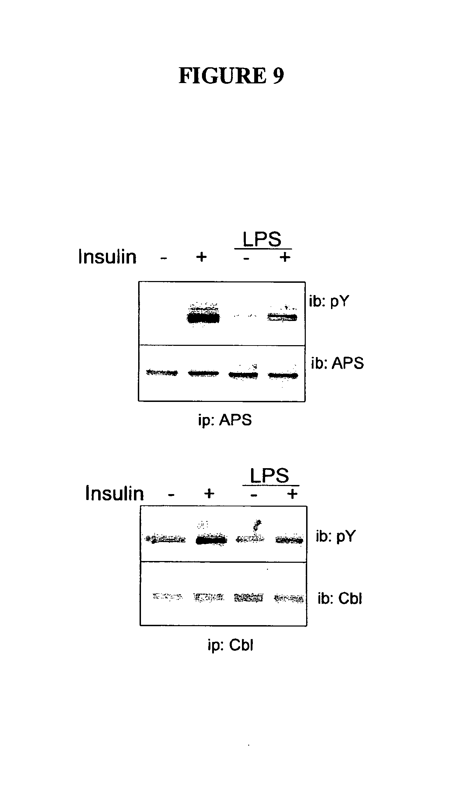

**Figure 9** shows that LPS attenuate the insulin-stimulated tyrosine phosporylation of the adapter proteins APS and Cbl. 3T3L1 adipocytes were pretreated with LPS prior to treatment with insulin. Cells were lysed and APS and C-Cbl tyrosine phosphorylation was assessed by Western blotting as shown in this Figure.

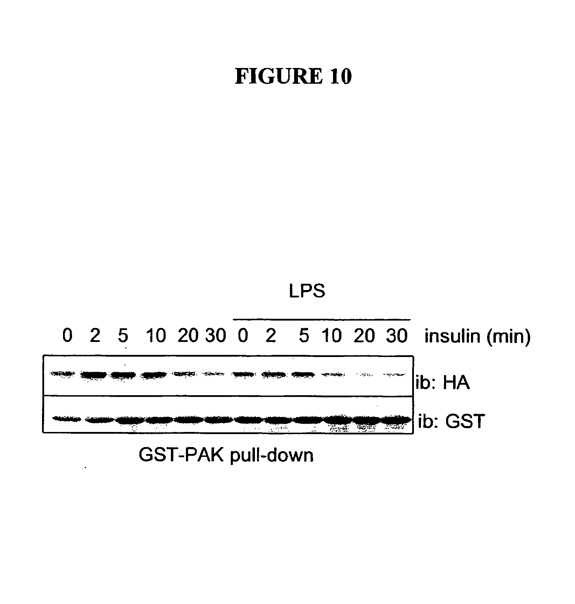

**Figure 10** shows a Western blot from Example 1 that shows that LPS treatment reduces the activation of TC10 by insulin.

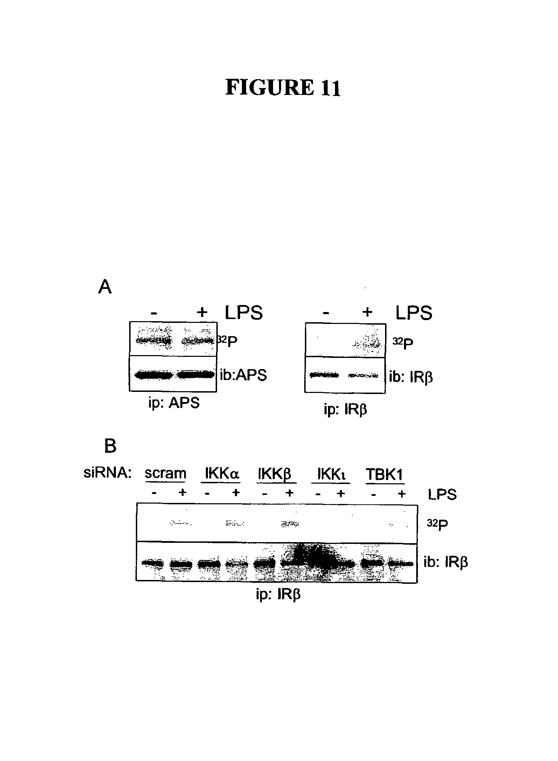

**Figure 11** shows that LPS stimulates the phosphorylation of the insulin receptor via activation of IKKi. Figure 11a: 3T3L1 cells were incubated with ³²P-orthophosphate, and stimulated with or without LPS. InsR and APS were immunoprecipitated and subject to autoradiography. Figure 11b: cells were subject to knock down with the indicated Ikki siRNA oligos prior to ³²P labelling and immunoprecipitation.

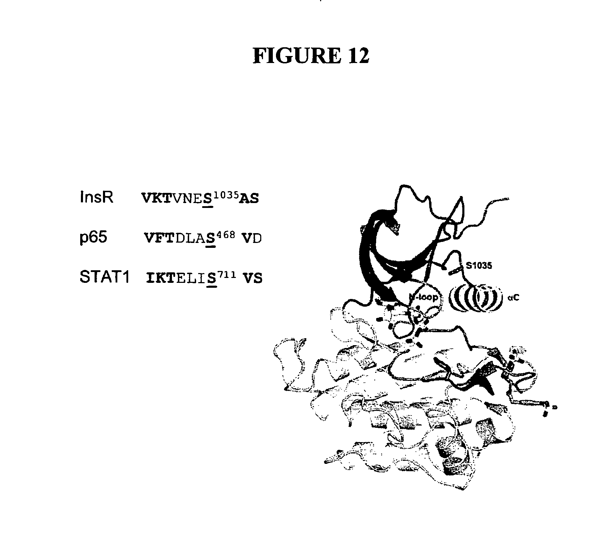

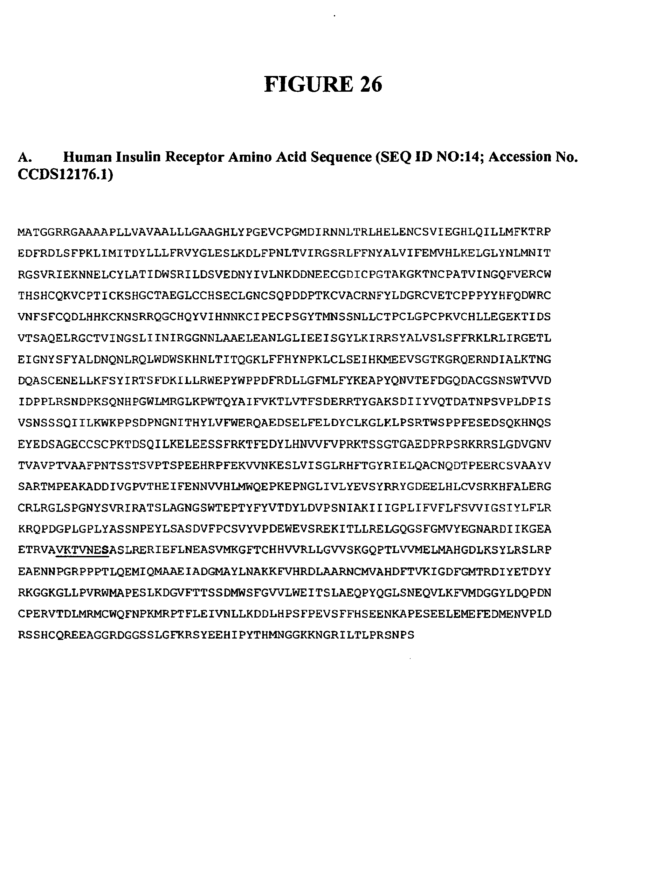

**Figure 12** shows contemplated phosphorylation sites on the insulin receptor by IKKi, based on known IKKi consensus sites. The following sequences are shown in this Figure: InsR: VKTVNES¹⁰³⁵ AS (SEQ ID NO:9); p65: VFTDLAS⁴⁶⁸VD (SEQ ID NO:7) and STAT1: IKTELIS⁷¹¹VS (SEQ ID NO:8). It is noted that the phosphorylated serine at position 1035 in the insulin receptor, is position 1062 in the an alternatively spliced version of the insulin receptor (which is shown at Figure 26).

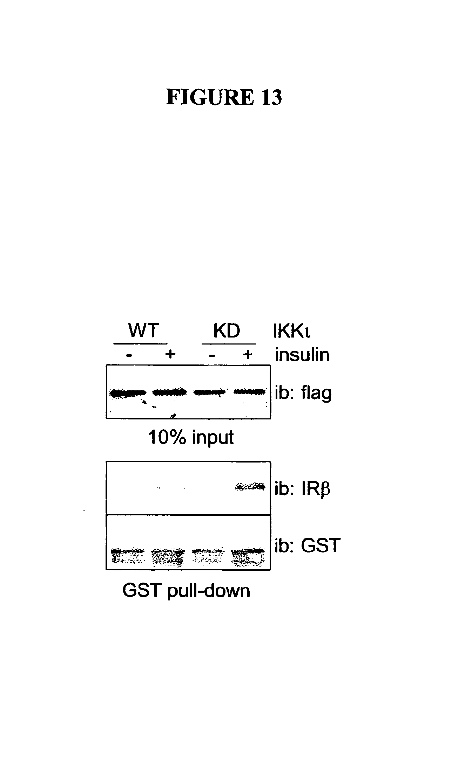

**Figure 13** shows overexpression of kinsase-inactive IKKi increases the binding of the insulin receptor to APS. COS cells were transfected with wildtype or kinase-inactive IKKi. Cells were stimulated with or without insulin, lysed and lysates pulled down with GST-APS SH2 domain.

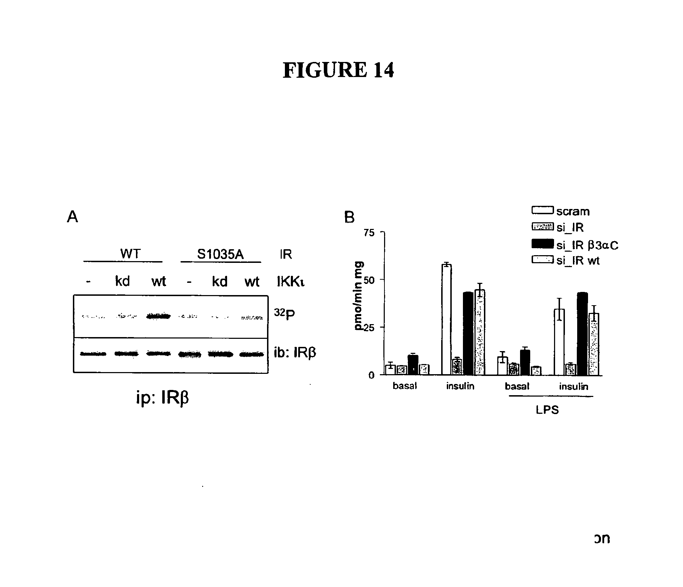

**Figure 14** shows mutation of Ser¹⁰³⁵ in the insulin receptor blocks the inhibitory effect of LPS on insulin-stimulated glucose uptake. 3T3L1 adipocytes were subject to siRNA knockdown of the insulin receptor, followed by transfection with a vector containing wildtype or ser¹⁰³⁵ Ala mutant receptor. Cells were pretreated with LPS followed by insulin, and A) insulin receptor phosphorylation by ³²P incorporation or B) glucose transport was assayed.

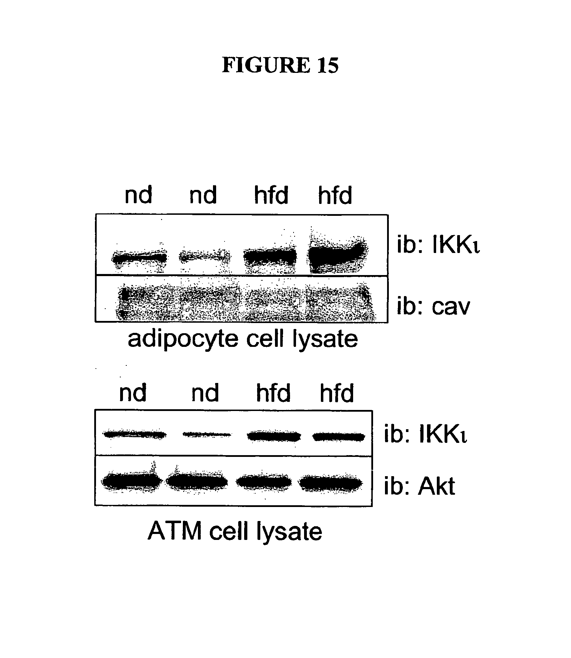

**Figure 15** shows IKKi is upregulated in adipose tissue after high fat feeding of mice. Mice were fed a normal chow or high fight diet for 8 weeks, and adipose tissue was excised and subject to differential centrifugation to separate adipocytes and a stromal vascular fraction that contains adipose tissue macrophages (ATM). Cells were lysed and subject to western blotting with anti-IKKi antibodies.

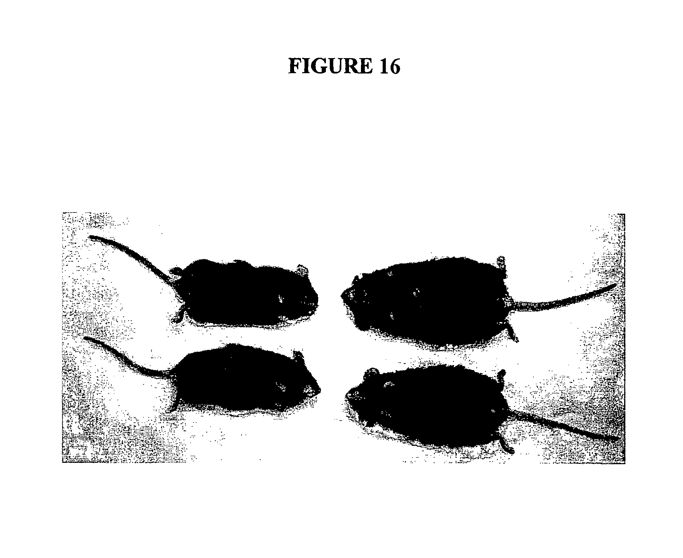

**Figure 16** shows that genetic ablation of Ikki prevents weight gain on a high fat dies. IKKiKo mice are shown on the left, while wildtype mice are on the right.

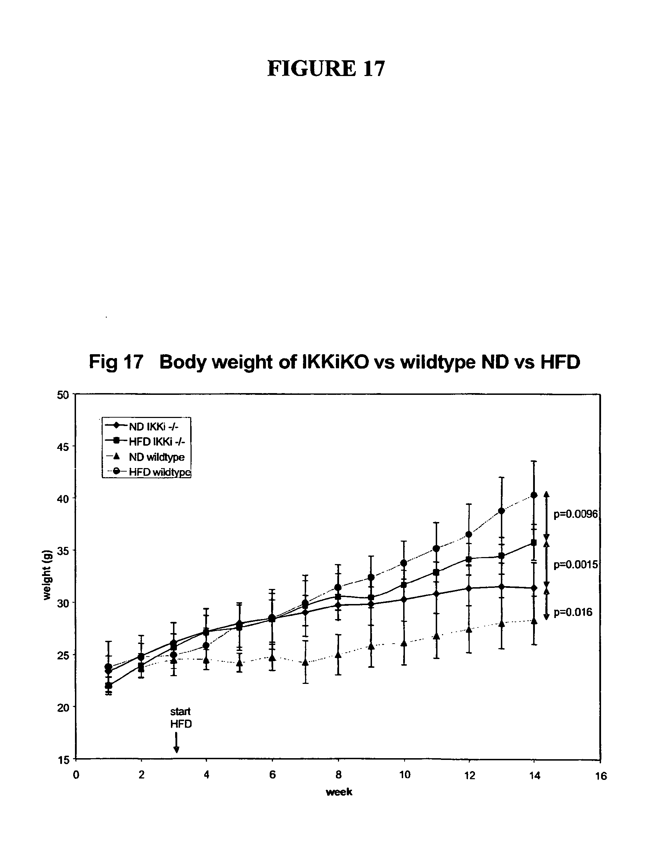

**Figure 17** shows that IKKi knockout mice (IKKiKO) gained significantly less weight than did their wildtype littermates on high fat and normal chow diets, with quantitation of this data indicating that this reduction in weight gain was statistically significant, with a P<0.01.

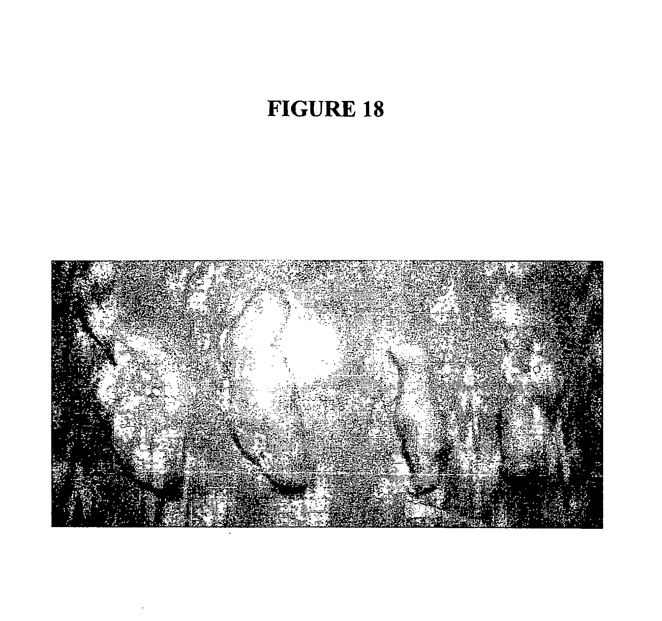

**Figure 18** shows that genetic ablation of IKKi prevents adipose tissue expansion after high fat feeding. Epididymal fat pads were excised from wildtype (left) or IKKiKO (right) mice fed a high fat diet for 8 weeks.

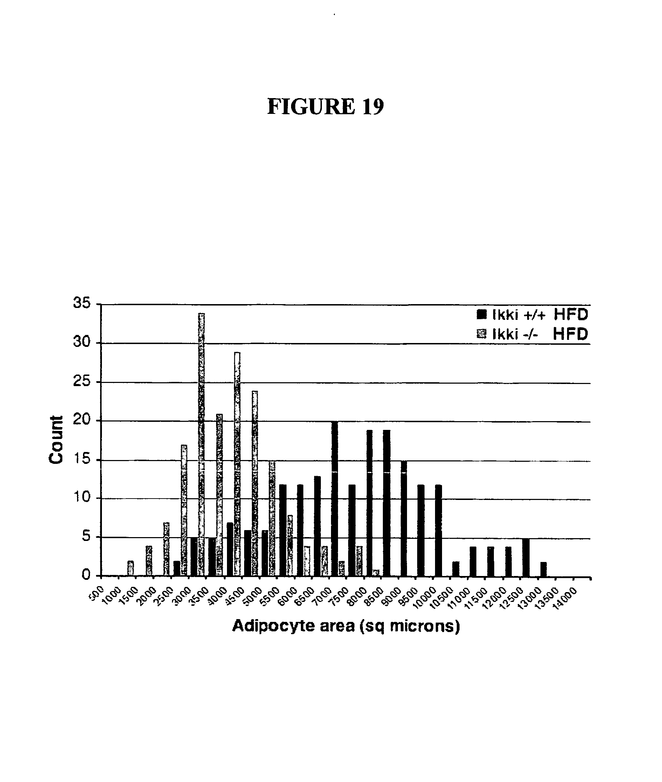

**Figure 19** shows that the reduction in weight in the IKKiKO mice was due to small fat cells.

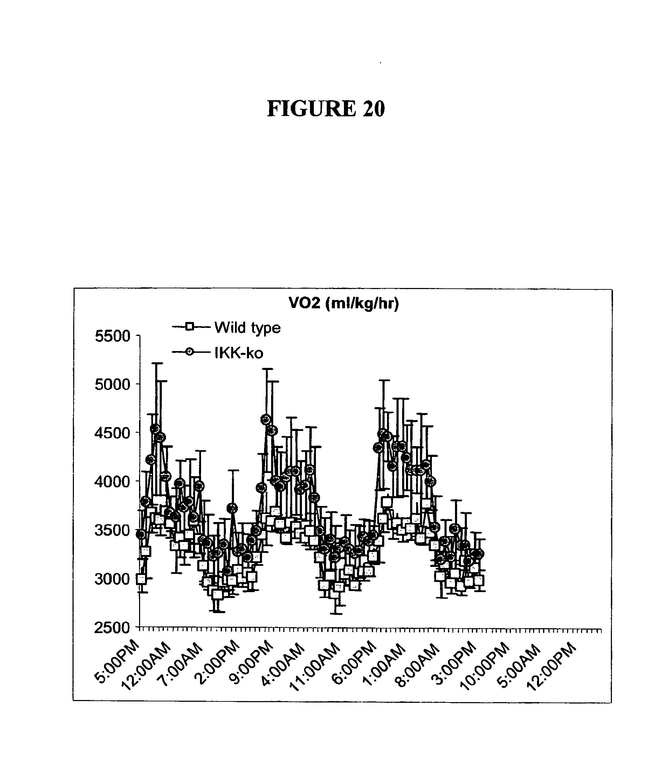

**Figure 20** shows that genetic ablation of IKKi increases respiration in mice.

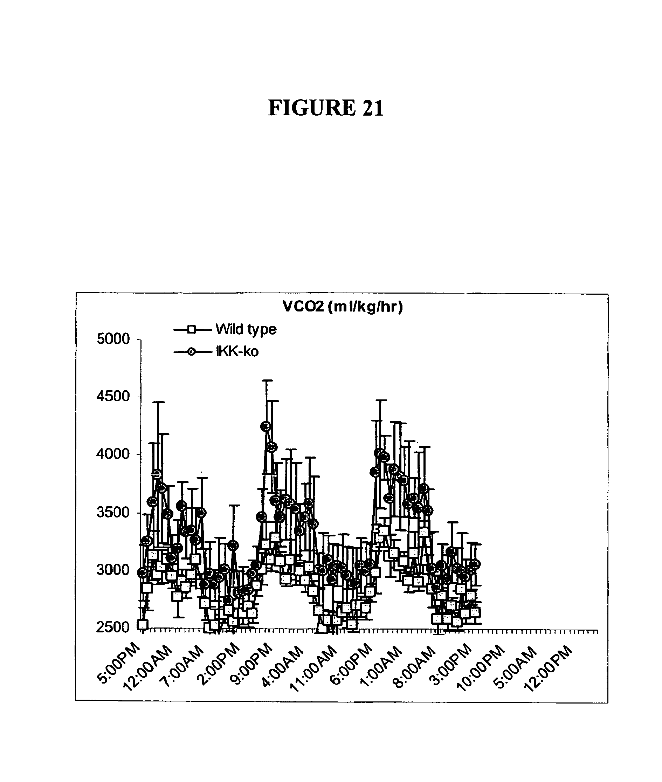

**Figure 21** shows that genetic ablation of IKKi increases respiration in mice.

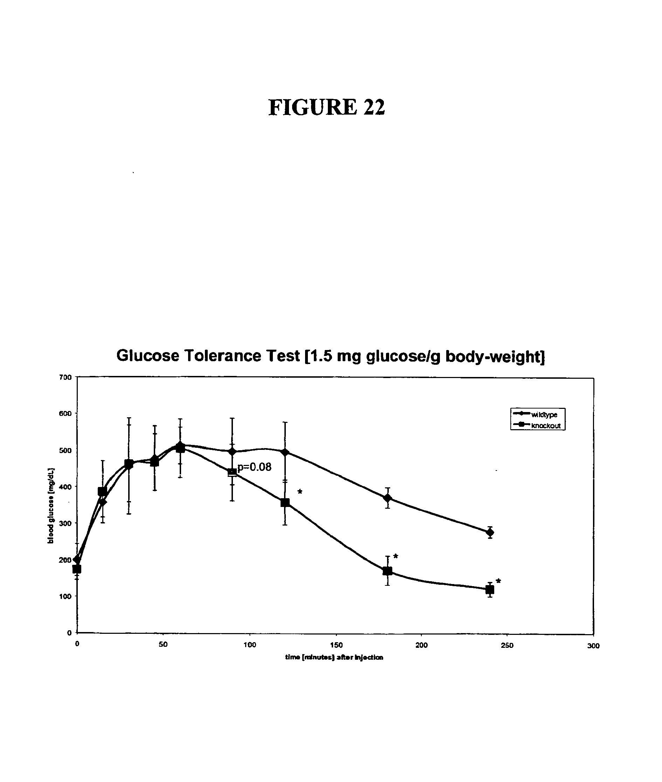

**Figure 22** shows that IKKiKo mice remain glucose tolerant on a high fat diet.

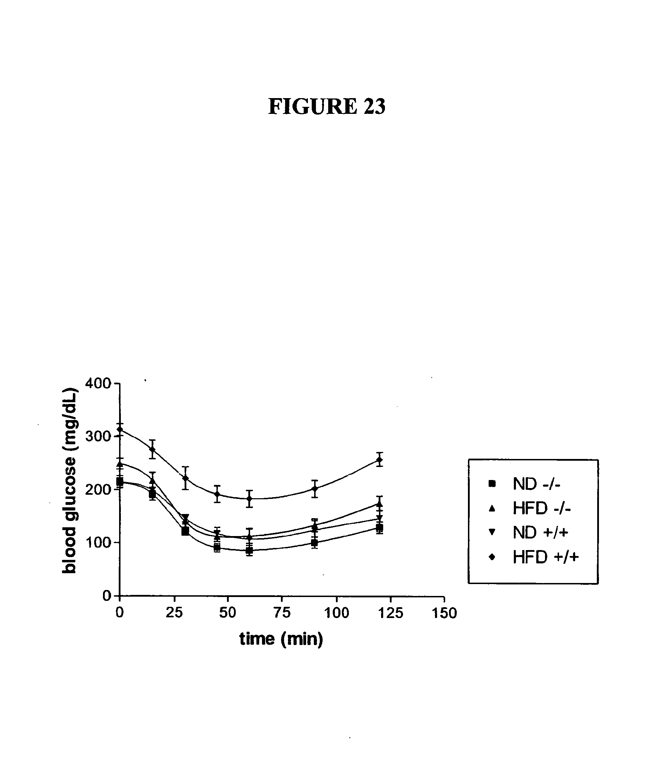

**Figure 23** shows insulin tolerance in IKKi knock out mice after 3 months on a high fat diet.

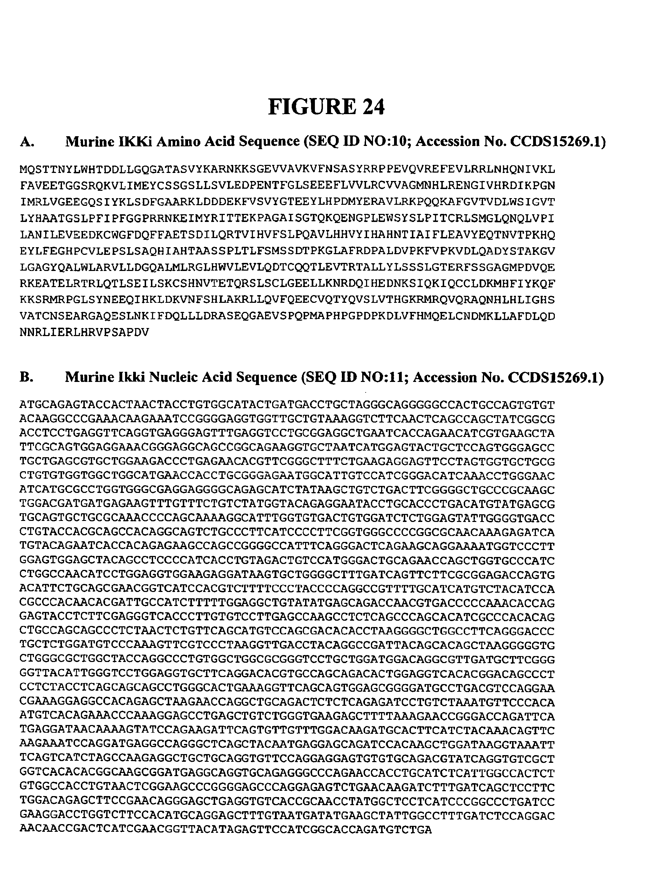

**Figure 24A** shows the consensus sequence for the murine IKKi amino acid sequence (SEQ ID NO: 10), and **Figure 24B** shows the consensus sequence for the murine Ikki nucleic acid sequence.

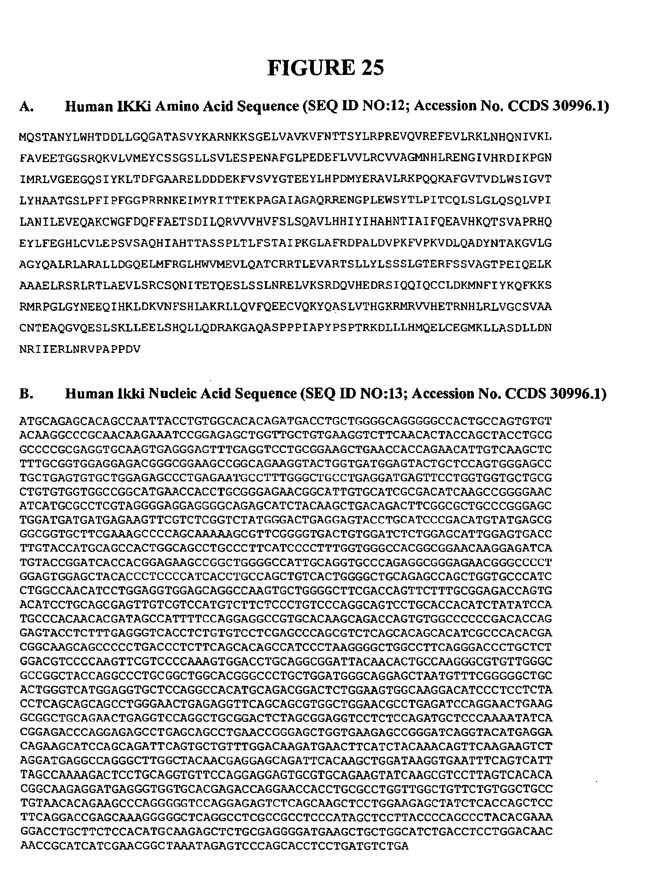

**Figure 25A** shows the consensus sequence for the human IKKi amino acid sequence (SEQ ID NO:10), and **Figure 25B** shows the consensus sequence for the human Ikki nucleic acid sequence.
**Figure 26** shows the consensus amino acid sequence of one of the two alternative splicing forms of human Insulin Receptor (SEQ ID NO: 14). Underlined in this Figure is VKTVNES (SEQ ID NO:15). The serine in this sequence (shown in bold) is the site of phosphorylation of the insulin receptor by IKKi.

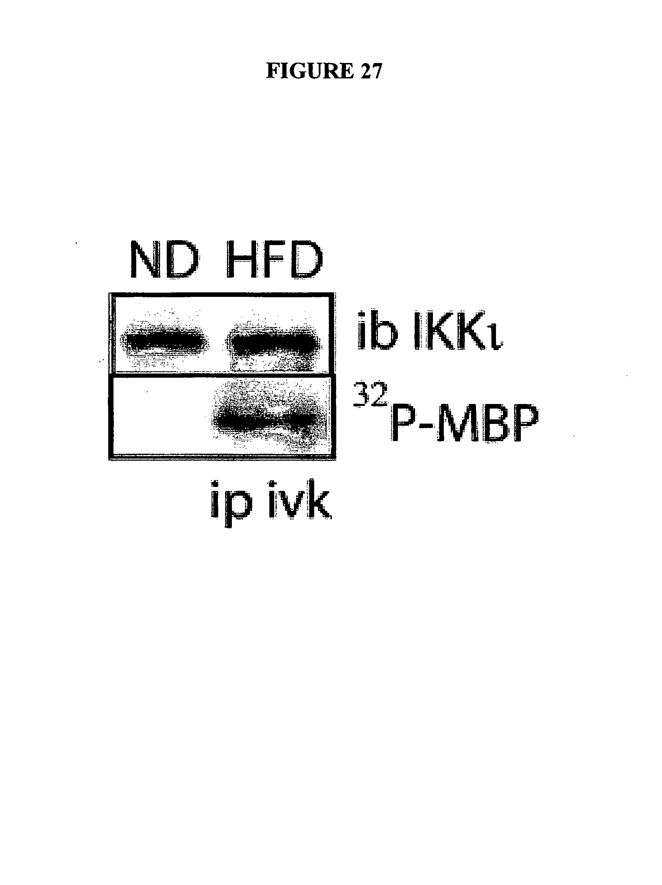

**Figure 27** shows that IKKi enzyme activity is markedly elevated in adipose tissue derived from high fat fed mice compared to control mice.

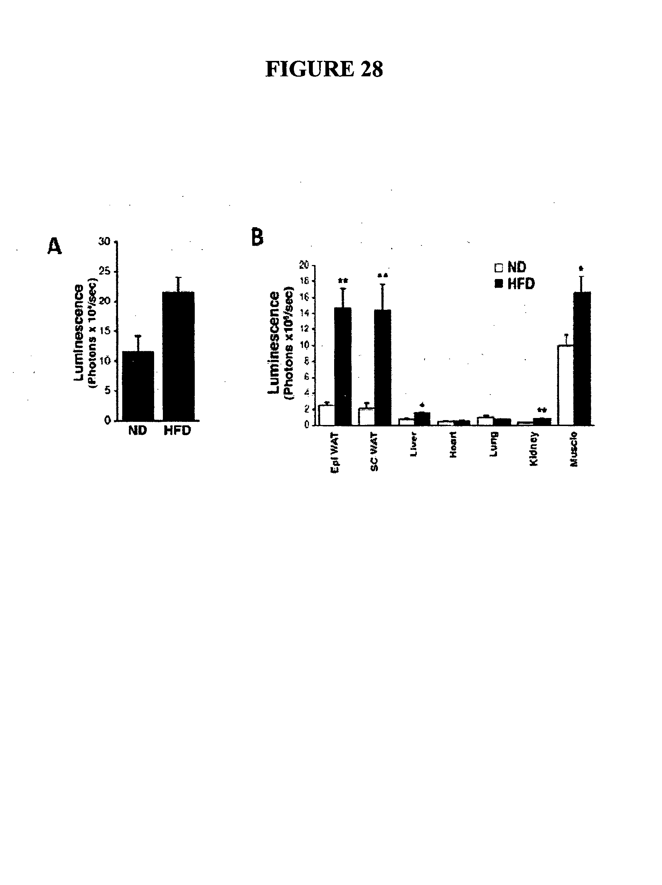

**Figure 28** shows that high-fat diet (HFD) increases NFκB activity in adipose tissue as measured by *in vivo* bioluminescence in live mice. A, Male HLL mice on normal diet (ND) and HFD were assessed for bioluminescence after injection of luciferin. Quantitation of luminescence collected over the abdominal cavity is presented for ND and HFD HLL mice.
(n=7 per group). **p*-value<0.05. B, Tissues from HLL mice were dissected and assessed *ex vivo* for luminescence. Quantitation of absolute tissue luminescence from HLL mice. n=7 mice per group. Data was collected serially after dissection to ensure plateau of luminescent signal.

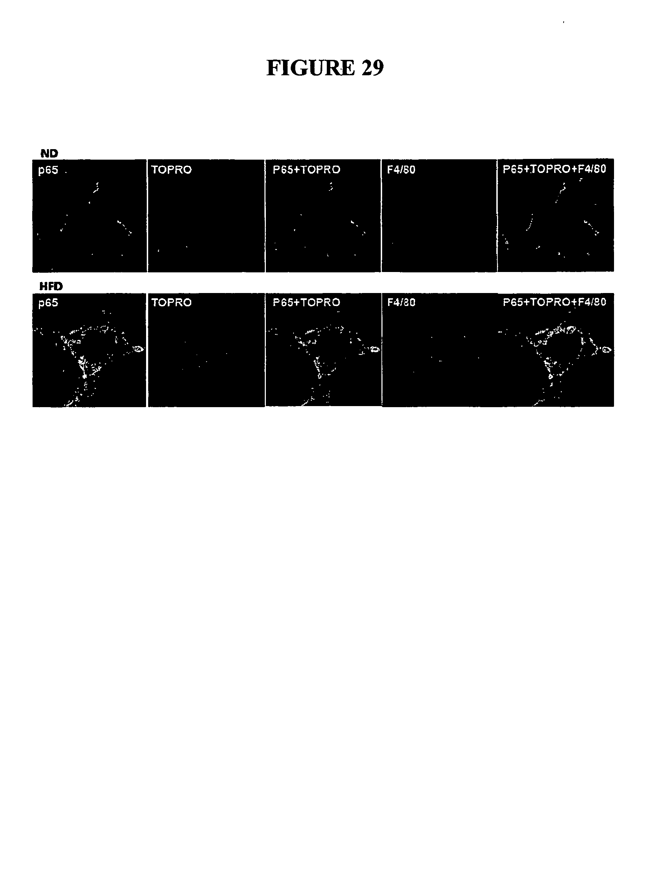

**Figure 29** shows induction of NFκB expression in adipose tissue macrophage (ATM) clusters in obese mice. Epididymal fat pads from ND or HFD fed male C57Bl/6 mice were analyzed for p65/RelA expression by immunofluorescence showing maximal signal in ATM clusters and localization of p65 in ATM nuclei (TOPRO3 co-stain).

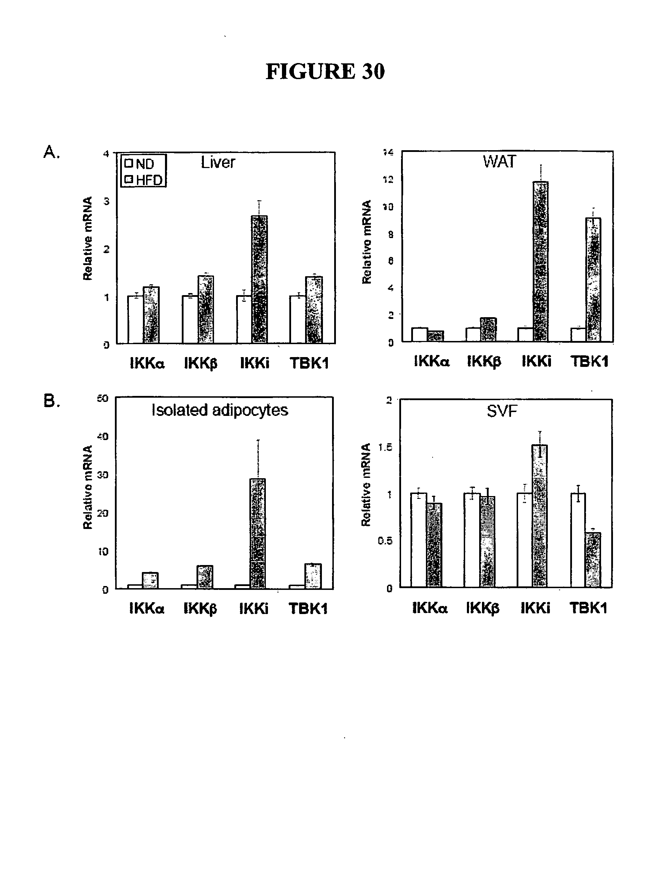

**Figure 30** shows that high fat diet increases IKKi expression in white adipose tissue and liver. A, Quantitative qPCR analysis on the expression of genes encoding IKK family members in liver and white adipose tissue. White bars, wild-type mice, normal diet (ND) (n=6); gray bar, wild-type mice, high fat diet (HFD) for 4 months (n=6). All data are presented as the average ±SEM normalized to *Rplp0* expression. Average of ND value was set as 1. B, Quantitative qPCR analysis on the expression of genes encoding IKK family members in isolated adipocytes and stromal vascular fraction. White bars, ND (n=6); gray bar, HFD for 4 months (n=6).

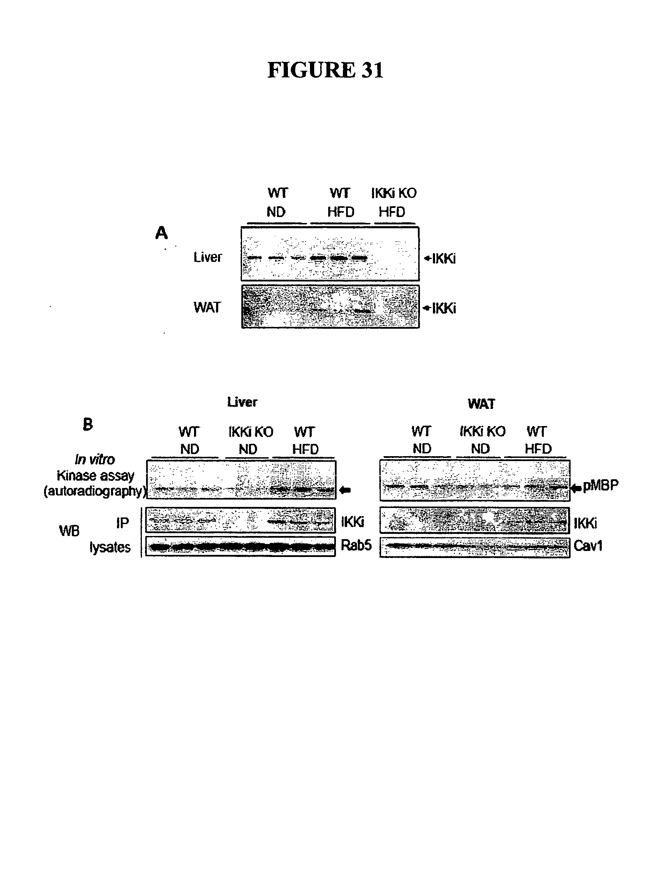

**Figure 31** shows additional analysis of high fat diet-induced increases IKKi expression in white adipose tissue and liver. A, Lysates from liver and white adipose tissue (WAT) of wild type (WT) and IKKi knockout mice (IKKi KO) fed with ND or HFD were immunoprecipitated with antibody against IKKi as indicated. The expression level of IKKi was determined by immunoblotting with same antibody against IKKi. B, Lysates from liver and WAT of WT and IKKi KO fed with ND or HFD were immunoprecipitated (IP) with antibody against IKKi and assayed for kinase activity against myelin basic protein (MBP) as substrate. The expression level of IKKi in IP was determined by immunoblotting with same antibody against IKKi. Lysates for IP were immunoblotted with antibodies against Rab5B and Caveolin 1 as a loading control.

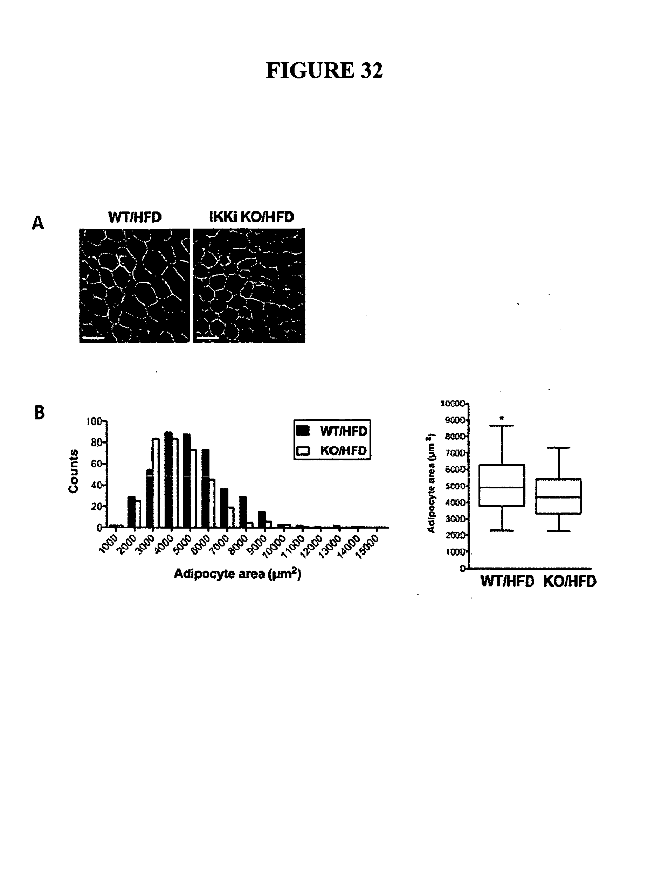

**Figure 32** shows that IKKi KO mice are protected from diet-induced weight gain by increasing energy expenditure. A, Representative confocal image of caveolin-stained epididymal adipose tissue from WT and KO mice fed with HFD. Bar=100µm. B, Whisker plot of adipocyte area from evaluation of <500 adipocytes from 3-4 independent mice. *p<0.0001 comparing mean adipocyte area.

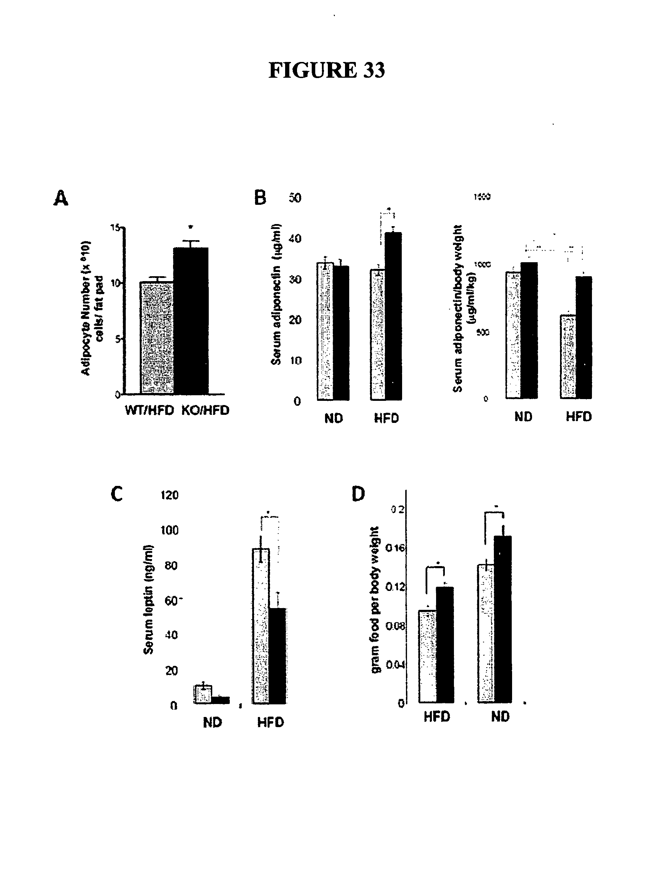

**Figure 33** shows additional analyses indicating that IKKi KO mice are protected from diet-induced weight gain by increasing energy expenditure. A, Adipocyte numbers in fat pads of WT (gray bar) and IKKi KO mice (black bar) fed with HFD. n=5 mice per genotype. *, *p*-value<0.005. B, (Left) adiponectin levels in serum from WT (gray bar) and KO (black bar) mice fed with ND or HFD as indicated. (Right) serum adiponectin normalized with body weight. n=12 mice per group. *, *p*-value<0.05; **, *p*-value<0.01. C, Leptin levels in serum from WT (gray bar) and KO (black bar) mice fed with ND or HFD as indicated. n=12 mice per group. *, *p*-value<0.05. D, Food intake was measured for WT (gray bar) and KO (black bar) mice fed with ND or HFD as indicated. n=8 mice per group. *, *p*-value<0.05.

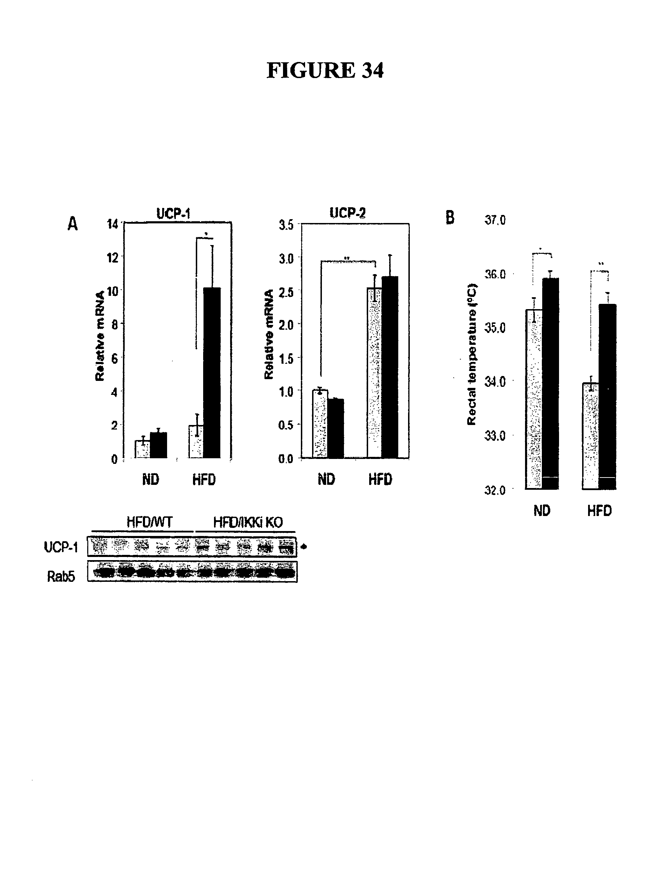

**Figure 34** shows additional analyses indicating that IKKi KO mice are protected from diet-induced weight gain by increasing energy expenditure. A, (Top) quantitative qPCR analysis on the expression of genes encoding UCP-1 and UCP-2 in WAT. Gray bars, wild-type mice (n=6); black bar, IKKi KO mice (n=6) fed with ND or HFD as indicated. Data are presented as the average ±SEM normalized to *Rplp0* expression. *, *p*-value<0.05. Average of WT fed with ND value was set as 1. (Bottom) Protein expression of UCP-1 in WAT, measured by immunoblotting with WAT lysates from WT and IKKi KO mice (5 mice in each group) fed with HFD as indicated. Rab5 was used as internal loading control. B, Rectal temperature measured for WT and KO mice fed with ND (3 months old) or HFD (5 months old with diet for 2 months). n=10 per group. *, *p*-value<0.05; **, *p*-value<0.01.

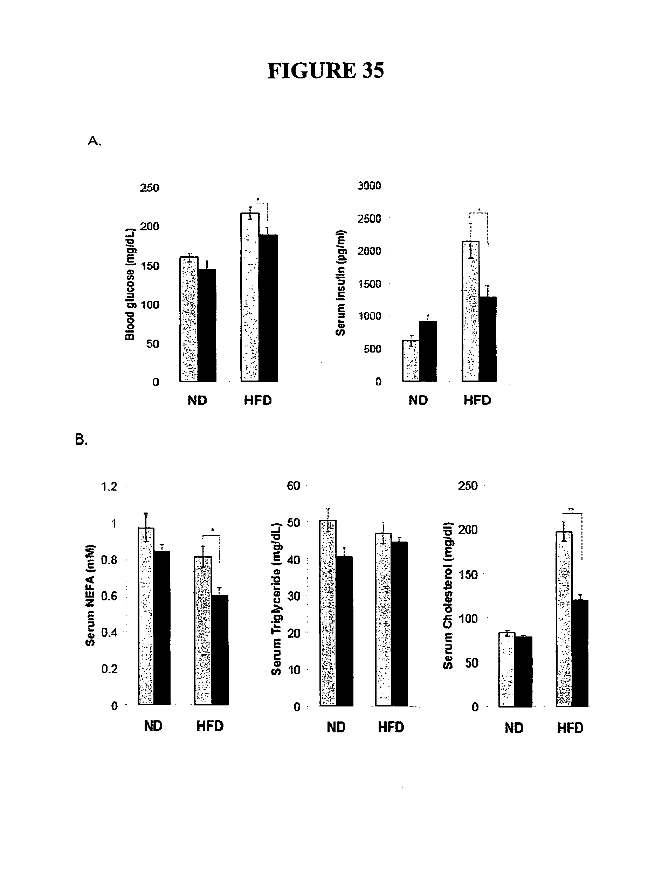

**Figure 35** shows IKKi KO mice display improved glucose and lipid homeostasis. A, Blood glucose and serum insulin levels measured for 18 hr fasting WT (gray bar) and KO (black bar) mice fed with ND or HFD as indicated. n=12 mice per group. B, Serum NEFA, triglyceride and total cholesterol levels measured for 18 hr-fasting WT (gray bar) and KO (black bar) mice fed with ND or HFD as indicated. n=12 mice per group. All data are presented as the average ±S.E.M. (*, *p*-value<0.05; **, *p*-value<0.01).

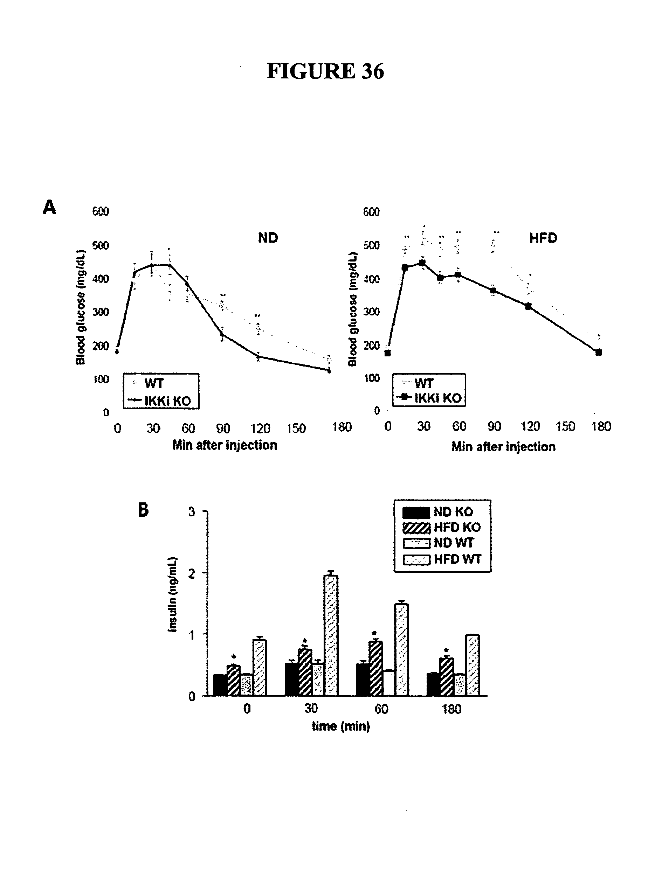

**Figure 36** shows additional data indicating that IKKi KO mice display improved glucose and lipid homeostasis. A, Glucose tolerance test (GTT) measured for 12 hr-fasting WT (gray) and KO (black) mice fed with ND (left panel) or HFD (right panel). n=12 mice per group. B, Serum insulin levels measured for mice during GTT shown in (C) at time points 0, 30, 60 and 180 min after injection. All data are presented as the average ±S.E.M. (*, *p*-value<0.05; **, *p*-value<0.01).

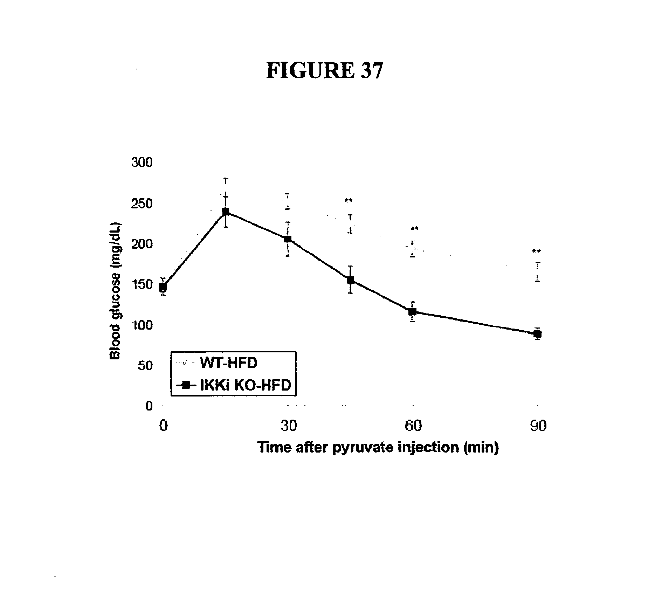

**Figure 37** shows additional data indicating that IKKi KO mice display improved glucose and lipid homeostasis. Pyruvate tolerance test (PTT) measured for 12 hr fasting WT (gray) and KO (black) mice fed with HFD. n=12 mice per group. All data are presented as the average ±S.E.M. (*, *p*-value<0.05; **, *p*-value<0.01).

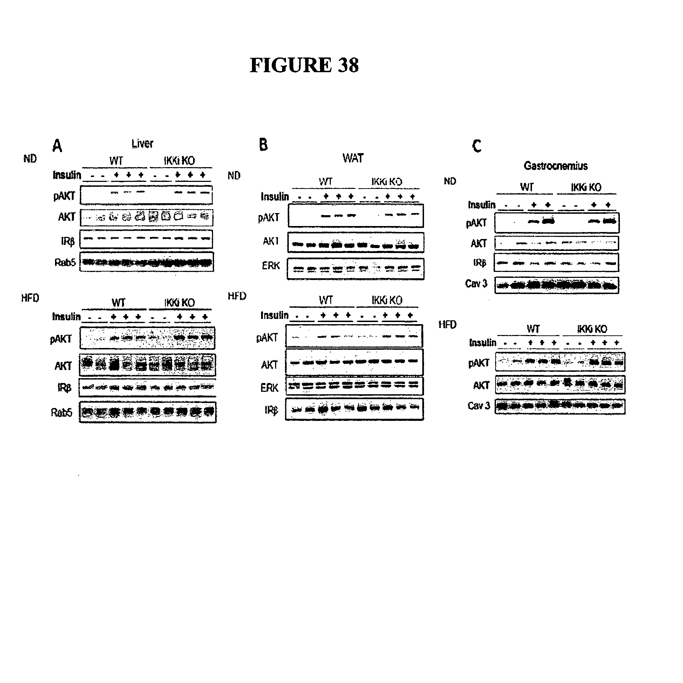

**Figure 38** shows that IKKi knock out preserves insulin signaling and insulin sensitivity in liver and adipose cells in mice fed a high fat diet. (A-C) Mice fasted for 18 hrs were IP injected with insulin (5mU/g) or saline. Lysates from liver (A), WAT (B) and gastrocnemius (C) of WT (duplicate per group) or IKKi KO mice (triplicate per group) fed with ND (top) or HFD (bottom) were immunoblotted with indicated antibodies.

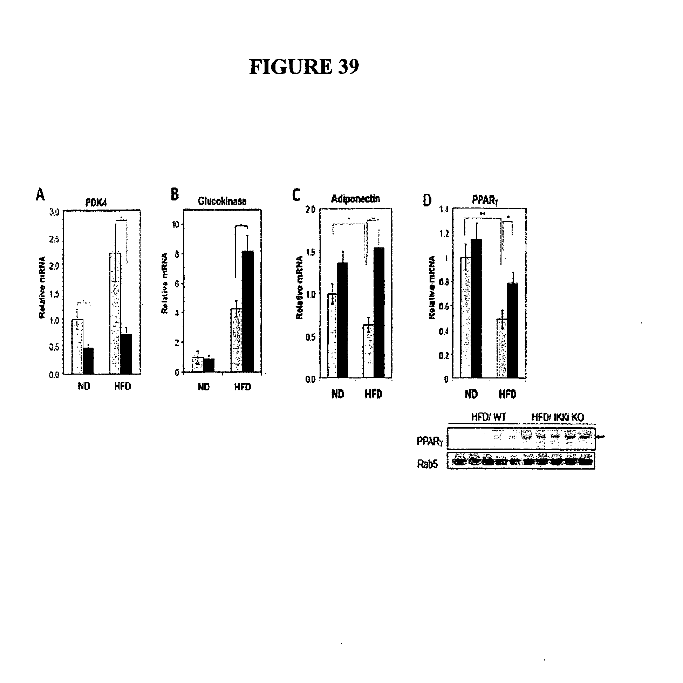

**Figure 39** shows additional data indicating that IKKi knock out preserves insulin signaling and insulin sensitivity in liver and adipose cells in mice fed a high fat diet. (A-B) Quantitative qPCR analysis on the expression of genes encoding PDK4 (A) and glucokinase (B) in liver of WT and IKKi KO mice fed with ND or HFD as indicated. Gray bars, wild-type mice (n=6); black bar, IKKi KO mice (n=6). (C- D) Quantitative qPCR analysis on the expression of genes encoding adiponectin (C) and PPARγ (D, Top) in WAT of WT and IKKi KO mice fed with ND or HFD as indicated. Gray bars, wild-type mice (n=6); black bar, IKKi KO mice (n=6). (D, Bottom) protein expression of PPARγ in WAT, measured by immunoblotting with WAT lysates from WT and IKKi KO mice (5 mice in each group) fed with HFD as indicated. Rab5 was used as internal loading control. All data are presented as the average ±S.E.M. (*, *p*-value<0.05; **, *p*-value<0.01).

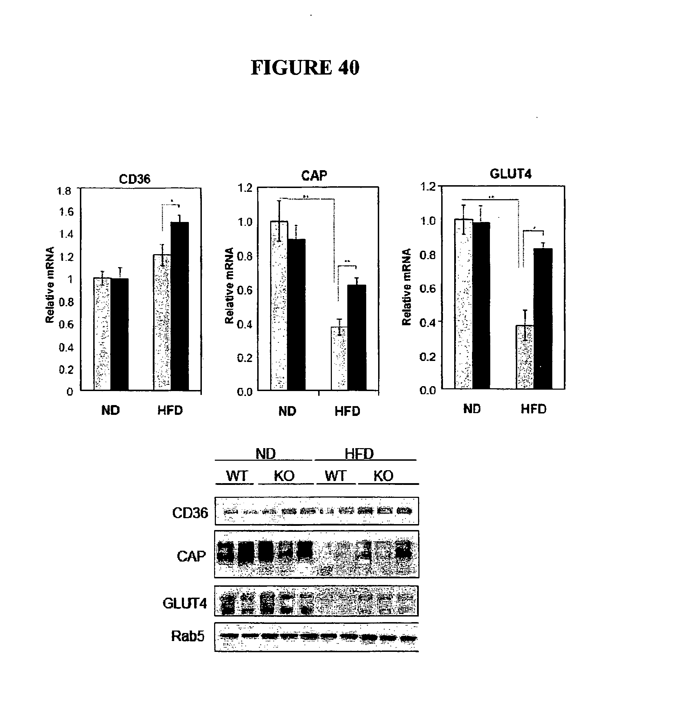

**Figure 40** shows additional data indicating that IKKi knock out preserves insulin signaling and insulin sensitivity in liver and adipose cells in mice fed a high fat diet. Top) quantitative qPCR analysis on the expression of genes encoding the PPARγ targets CD36, CAP, GLUT4 in WAT of WT and IKKi KO mice fed with ND or HFD as indicated. Gray bars, wild-type mice (n=6); black bar, IKKi KO mice (n=6). (Bottom) protein expression of CD36, CAP and GLUT4 in WAT, measured by immunoblotting with WAT lysates from WT (duplicate mice in each group) and IKKi KO mice (triplicate mice in each group) fed with ND or HFD as indicated. Rab5 was used as internal loading control. All data are presented as the average ±S.E.M. (*, *p*-value<0.05; **, *p*-value<0.01).

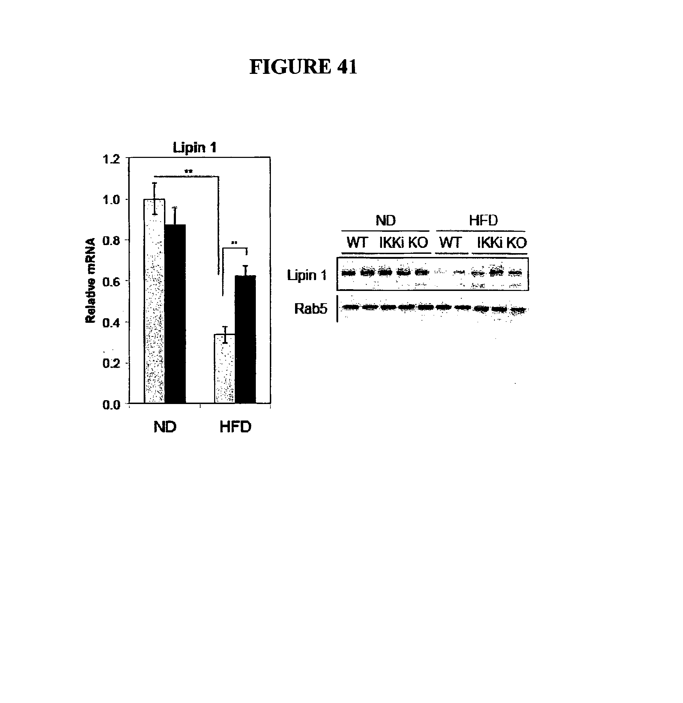

**Figure 41** shows additional data indicating that IKKi knock out preserves insulin signaling and insulin sensitivity in liver and adipose cells in mice fed a high fat diet. (Left) qPCR analysis on the expression of gene encoding Lipin1 in WAT of WT and IKKi KO mice fed with ND or HFD as indicated. Gray bars, wild type mice (n=6); black bar, IKKi KO mice (n=6). (Right) protein expression of lipin1 in WAT, measured by immunoblotting with WAT lysates from WT (duplicate mice in each group) and IKKi KO mice (triplicate mice in each group) fed with ND or HFD as indicated. Rab5 was used as internal loading control. All data are presented as the average ±S.E.M. (*, *p*-value<0.05; **, *p*-value<0.01).

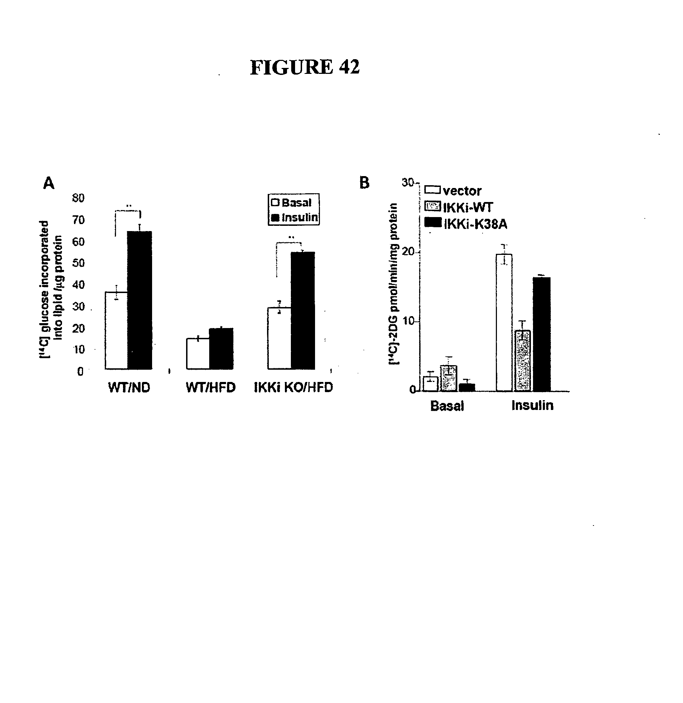

**Figure 42** shows additional data indicating that IKKi knock out preserves insulin signaling and insulin sensitivity in liver and adipose cells in mice fed a high fat diet. A, *Ex vivo* insulin-stimulated glucose incorporation into lipid in adipocytes isolated from WT and KO mice fed with ND or HFD as indicated. Cells were untreated (white bar) or treated with insulin for 30min (shaded bar). n=3 mice per condition. B, Insulin-stimulated glucose uptake in 3T3-L1 adipocytes. Differentiated adipocytes were electroporated with vector control (white bar), IKKi WT (gray bar) or IKKi kinase dead (K38A) (black bar) mutant expression constructs. Cells were untreated (basal) or treated with insulin for 30min (Insulin). Amount of ¹⁴C-2DG uptake in cells was normalized with total amount of protein. n=3 for each condition. All data are presented as the average ±S.E.M. (*, *p*-value<0.05; **, *p*-value<0.01).

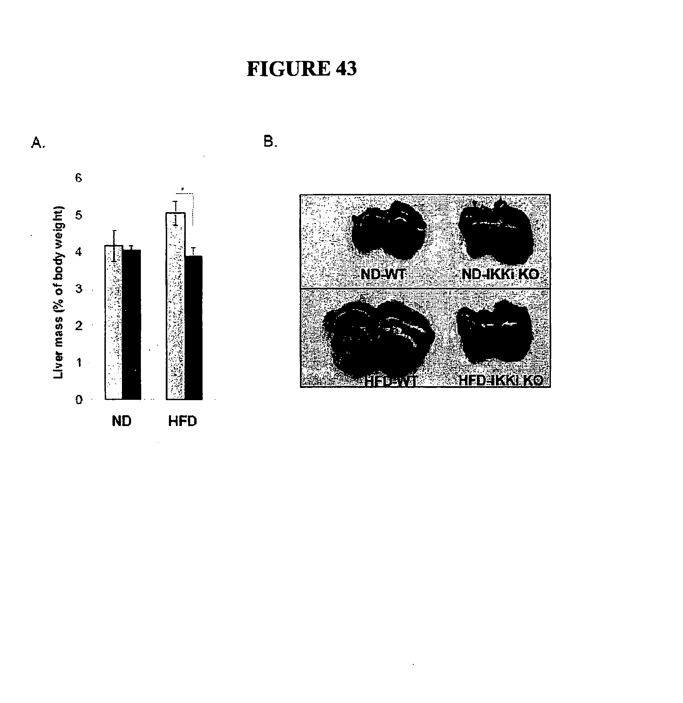

**Figure 43** shows that IKKi knockout mice are protected from diet-induced hepatic steatosis. A, Liver weight normalized with body weight was measured from WT (gray bar) and IKKi KO (black bar) mice fed with ND or HFD as indicated. n=8 for each group. B, Representative images of liver from WT and KO mice fed with ND or HFD as indicated.

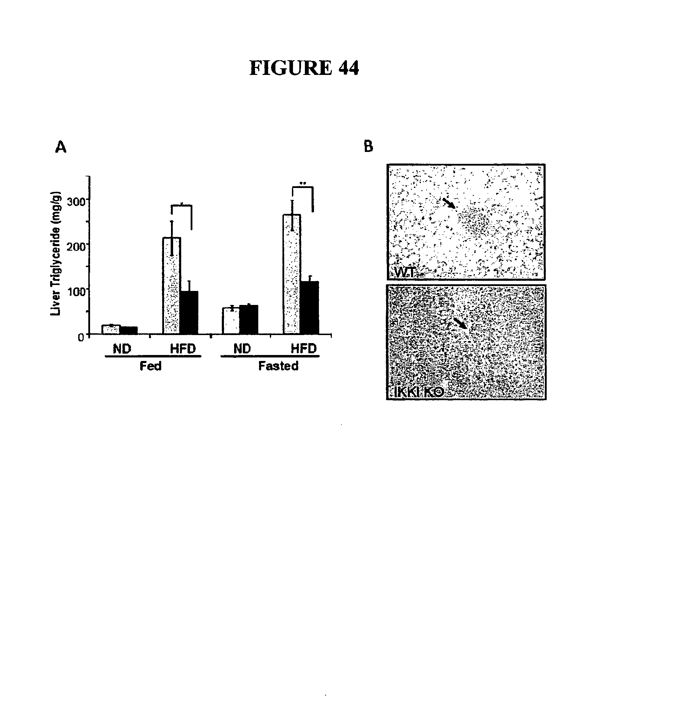

**Figure 44** shows additional data indicating that IKKi knockout mice are protected from diet-induced hepatic steatosis. A, Liver triglyceride content normalized with liver weight was measured from WT (gray bar) and IKKi KO (black bar) mice fed with ND or HFD in fed or fasted condition as indicated. n=8 for each group (*, p<0.05). B, Representative images of hematoxylin and eosin-stained section of liver from fasting WT or KO mice fed with HFD for 2 months. Arrows indicate central veins.

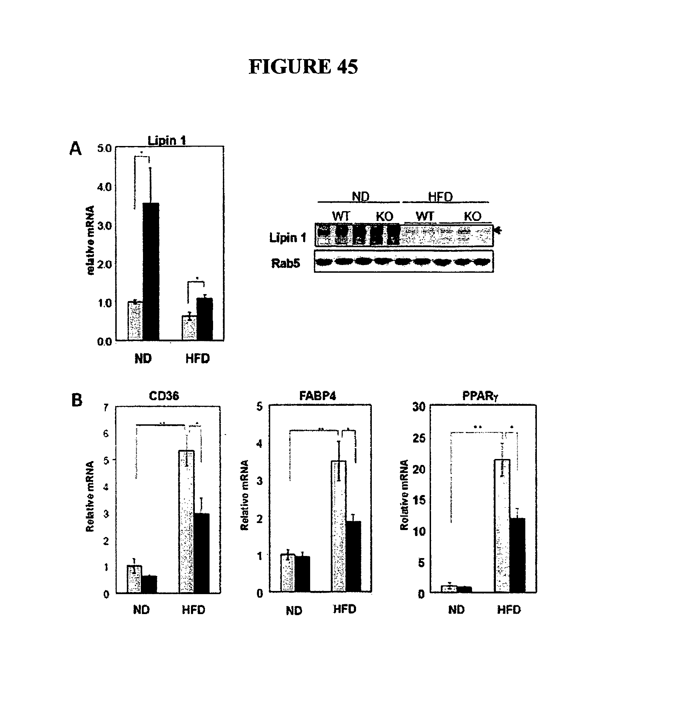

**Figure 45** shows additional data indicating that IKKi knockout mice are protected from diet-induced hepatic steatosis. A, (Left) quantitative qPCR analysis on the expression of the gene encoding Lipin1 in liver of WT and IKKi KO mice fed with ND or HFD as indicated. Gray bars, wild-type mice (n=6); black bar, IKKi KO mice (n=6). (Right) protein expression of lipin1 in liver, measured by immunoblotting with liver lysates from WT (duplicate mice in each group) and IKKi KO mice (triplicate mice in each group) fed with ND or HFD as indicated. Rab5 was used as an internal loading control. B, qPCR analysis on the expression of genes encoding CD36, FABP4, PPARγ in liver of WT and IKKi KO mice fed with ND or HFD as indicated. Gray bars, wild-type mice (n=6); black bar, IKKi KO mice (n=6).

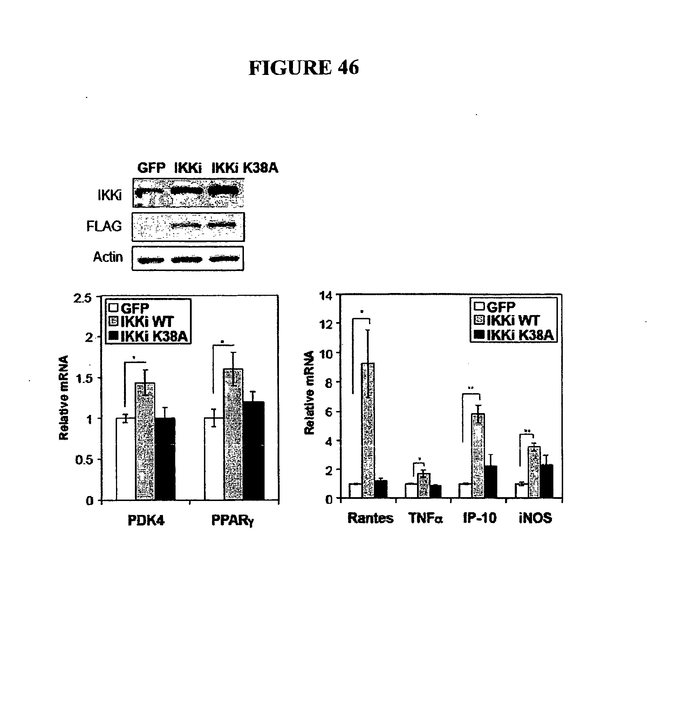

**Figure 46** shows additional data indicating that IKKi knockout mice are protected from diet-induced hepatic steatosis. (Top) Immunoblotting with an anti-FLAG antibody to detect the overexpression levels of IKKi WT and its kinase-dead mutant (K38A) in H2.35 hepatoma cells. (Bottom) qPCR analysis on the expression of the indicated genes. Gene expression was measured from cells transfected with vector control (white bar), IKKi WT (gray bar) or IKKi kinase dead (K38A) (black bar) mutant expression constructs.

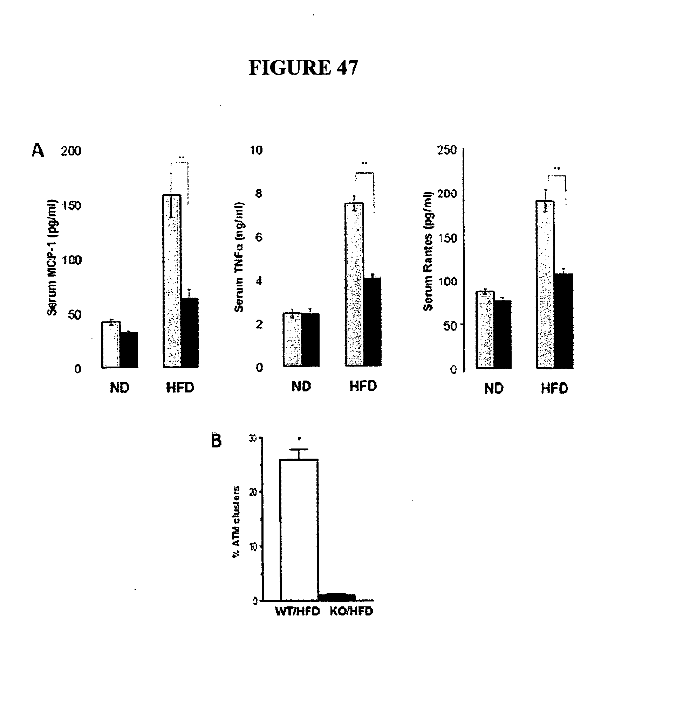

**Figure 47** shows that obesity-induced inflammation is attenuated in IKKi KO mice. A, Serum proinflammatory cytokines MCP-1, TNFα and Rantes secretion were measured in WT (gray bar) and IKKi KO mice (black bar) fed with ND or HFD as indicated. n=8. (**, *p-*value <0.01). B, Quantitation of F4/80⁺ crown-like structures. Confocal images were used to quantitate the percentage of crown-like structures. 3-5 low power fields analyzed for 3-4 mice per genotype (>1000 adipocyte examined per genotype, **p* value<0.001). All data are presented as the average ±S.E.M.

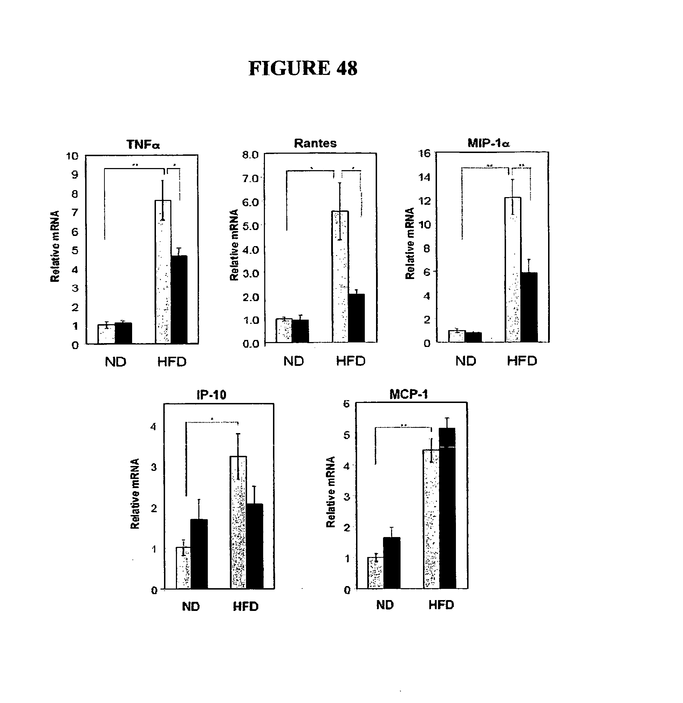

**Figure 48** shows additional data indicating that obesity-induced inflammation is attenuated in IKKi KO mice. Shown are qPCR analyses on the expression of genes encoding TNFα, Rantes, MIP-1 α, IP-10 and MCP-1 in WAT of WT and IKKi KO mice fed with ND or HFD as indicated. Gray bars, wild-type mice (n=6); black bar, IKKi KO mice (n=6). All data are presented as the average ±S.E.M. (*, *p-*value<0.05; **, *p*-value<0.01).

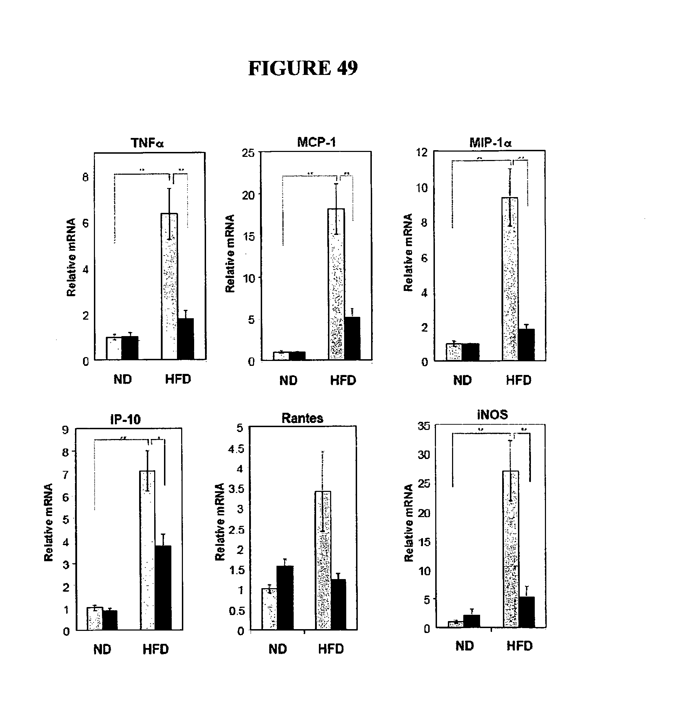

**Figure 49** shows additional data indicating that obesity-induced inflammation is attenuated in IKKi KO mice. Shown are qPCR analyses on the expression of genes encoding TNFα, MCP-1, MIP-1α, IP-10, Rantes, or iNOS in liver of WT and IKKi KO mice fed with ND or HFD as indicated. Gray bars, wild-type mice (n=6); black bar, IKKi KO mice (n=6). All data are presented as the average ±S.E.M. (*, *p*-value<0.05; **, *p*-value<0.01).

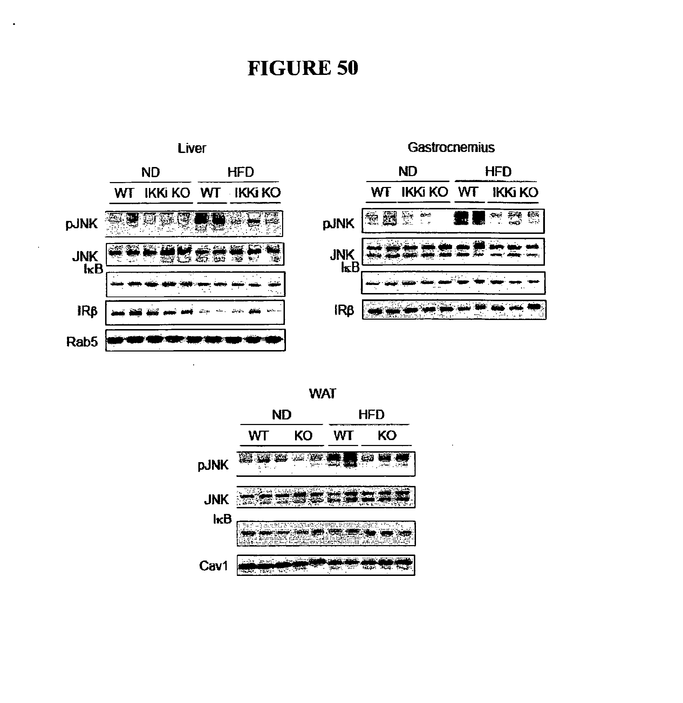

**Figure 50** shows additional data indicating that obesity-induced inflammation is attenuated in IKKi KO mice. Shown are protein levels of phospho-JNK, JNK, IκB were measured by immunoblotting with lysates from liver, gastrocnemius and WAT of WT (duplicate mice in each group) and IKKi KO mice (triplicate mice in each group) fed with ND or HFD as indicated. Rab5 and caveolin 1 were used as internal loading controls.

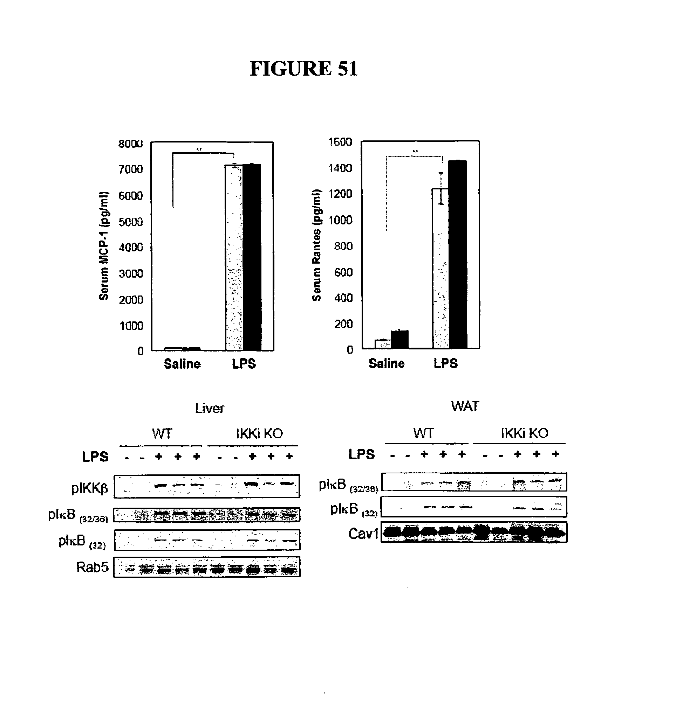

**Figure 51** shows additional data indicating that obesity-induced inflammation is attenuated in IKKi KO mice. (Top) serum proinflammatory cytokines MCP-1 and Rantes secretion were measured in WT (gray bar) and IKKi KO mice (black bar) injected with saline or LPS for 2.5 hrs as indicated. n=8. (Bottom) protein level of phospho-IKKβ, pIκB (serine 32 or serine 32/36) was measured by immunoblotting with lysates from liver and WAT of WT and IKKi KO mice injected with saline or LPS for 2.5 hrs as indicated. Rab5 and caveolin 1 were used as internal loading control. All data are presented as the average ±S.E.M. (*, *p*-value<0.05; **, *p*-value<0.01).

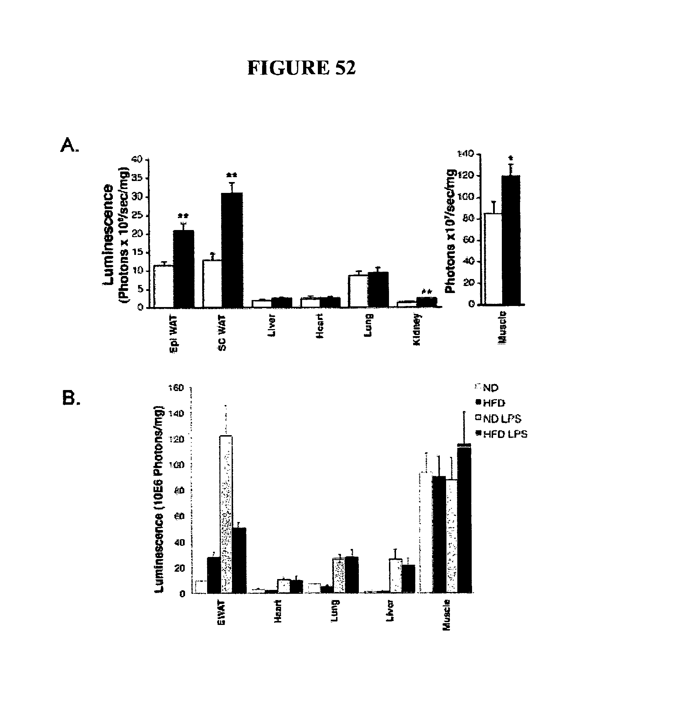

**Figure 52** shows activation of luciferase transgene in HLL mice with HFD. A, Quantitation of luciferase activity corrected for tissue weight. *, p-value<0.05; **, p value<0.01 B, HFD leads to submaximal activation of NFκB. Data from ND and HFD HLL mice were compared to tissue luminescence obtained 3 hours after IP injection of lipopolysaccharide (LPS).

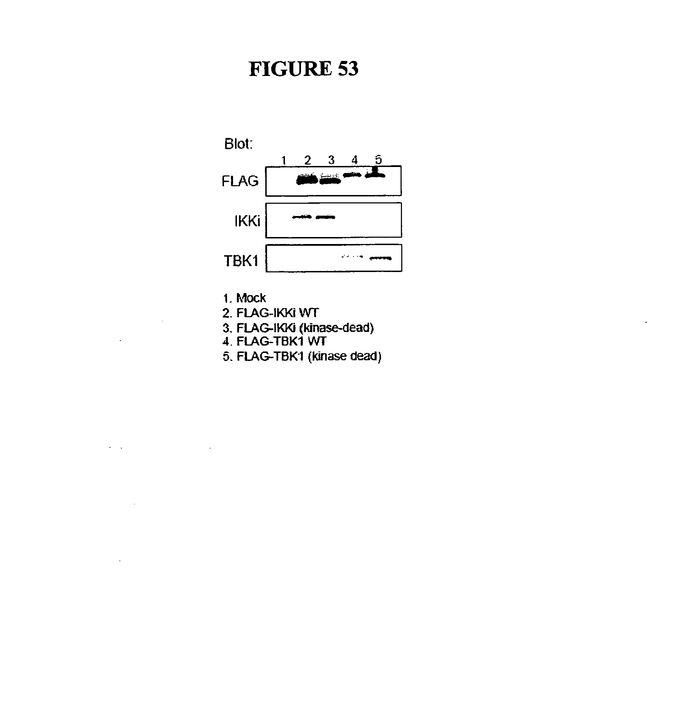

**Figure 53** shows characterization of the anti-IKKi antibody used in experiments conducted during the course of the present invention. FLAG-IKKi WT (lane 2), kinase-dead (K38A) (lane 3) and FLAG-TBK1 WT (Lane 4), kinase-dead (K38A) (lane 5) constructs were transfected into Cos cells. Lysates were immunoblotted with anti-FLAG, anti-IKKi and anti-TBK1 antibodies.

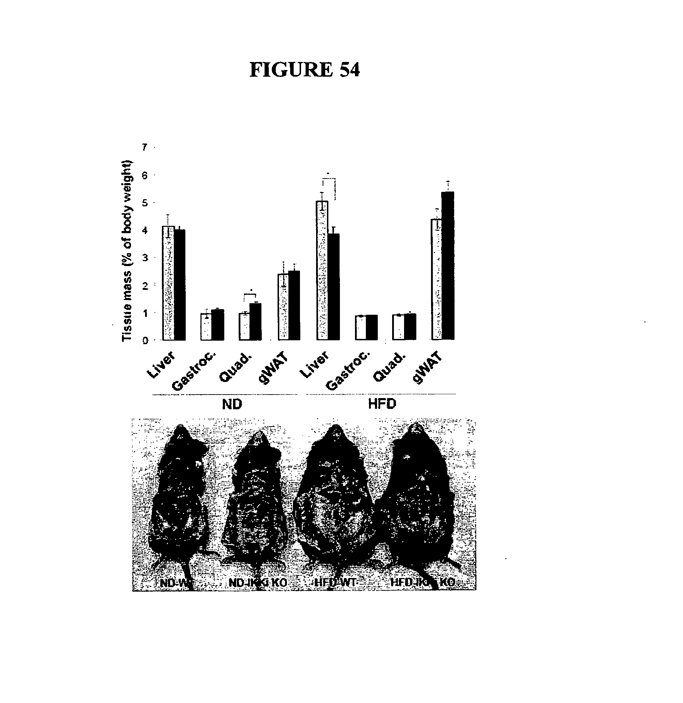

**Figure 54** shows measurement of tissue weight for WT and IKKi KO mice fed normal or high-fat diets. (Top) tissues weights normalized with body weight were measured for liver, gastrocnemius, quadriceps and gonadal WAT from WT (gray bar) and IKKi KO mice (black bar) fed with ND or HFD as indicated (*, *p*-value<0.05). (Bottom) Representative images of WT and KO mice fed with ND or HFD as indicated.

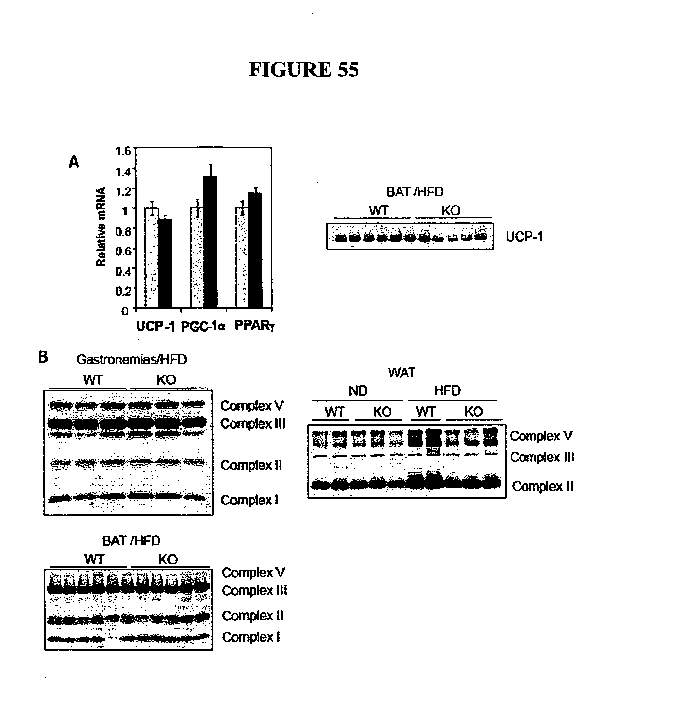

**Figure 55** shows thermogenesis gene expression in brown adipose tissue of WT and IKKi KO mice fed normal or high-fat diets. A, (Left) qPCR analysis of the expression of genes encoding UCP-1, PGC-1α and PPARγ in brown adipose tissue (BAT) of WT and IKKi KO mice fed with ND or HFD as indicated. Gray bars, wild-type mice (n=6); black bar, IKKi KO mice (n=6). (Right) protein level of UCP-1 was measured by immunoblotting with lysates from BAT of WT (n=5) and IKKi KO mice (n=5) fed with HFD. B, Protein levels of subunits of OXPHOS complex were immunoblotted with anti-OXOPHOS cocktail antibody with lysates from gastrocnemius, WAT and BAT of WT and KO mice fed with ND or HFD.

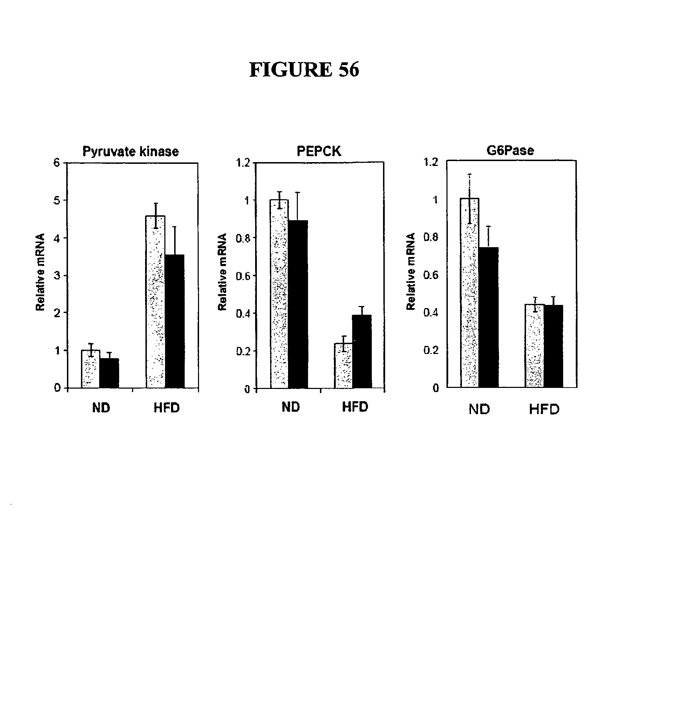

**Figure 56** shows glucose metabolic gene expression in liver of WT and IKKi KO mice fed normal or high-fat diets. Shown are qPCR analyses on the expression of glucose metabolic genes encoding pyruvate kinase, PEPCK, G6Pase in liver of WT and IKKi KO mice fed with ND or HFD as indicated. Gray bars, wild-type mice (n=6); black bar, IKKi KO mice (n=6).

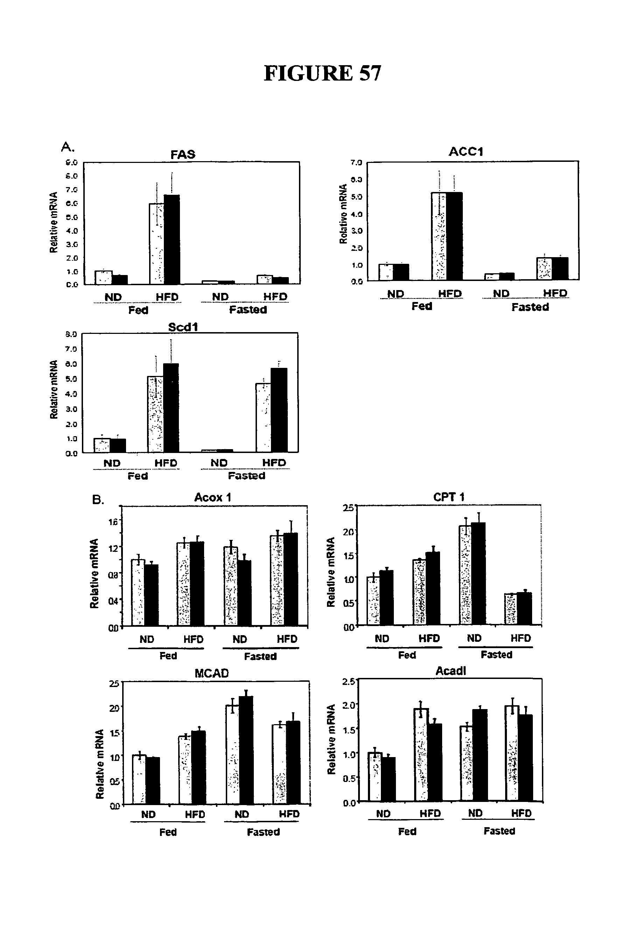

**Figure 57** shows lipid metabolic gene expression in liver of WT and IKKi KO mice fed normal or high-fat diets. A, qPCR analysis on the expression of fatty acid synthesis genes encoding FAS, ACC1 and SCD1 in liver of WT and IKKi KO mice fed with ND or HFD as indicated. Gray bars, wild-type mice (n=6); black bar, IKKi KO mice (n=6). B, qPCR analysis on the expression of β-oxidation genes encoding Acox1, CPT1, MCAD and Acad1 in liver of WT and IKKi KO mice fed with ND or HFD as indicated. Gray bars, wild-type mice (n=6); black bar, IKKi KO mice (n=6).

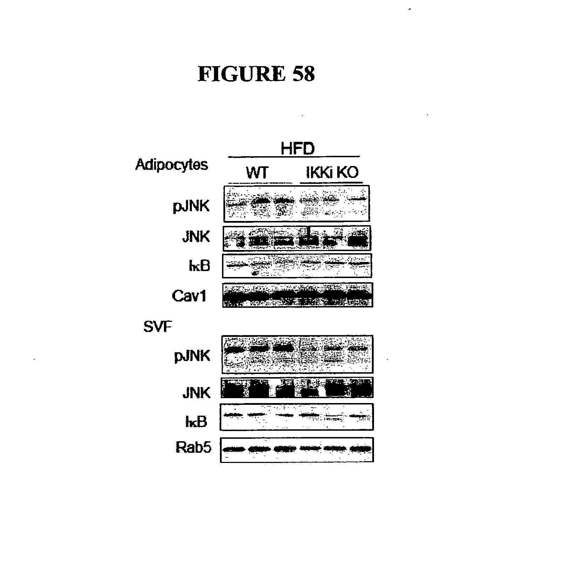

**Figure 58** shows Inflammatory signaling protein expression in isolated adipocytes and SVF from WT and IKKi KO mice fed normal or high-fat diets. Protein level of phospho-JNK, JNK, IκB was measured by immunoblotting with lysates from isolated adipocytes and SVF of WT (triplicate mice in each group) and IKKi KO mice (triplicate mice in each group) fed with HFD as indicated. Rab5 and caveolin 1 were used as internal loading controls.

### DEFINITIONS

To facilitate an understanding of the invention, a number of terms are defined below.

As used herein, the term "IKKi inhibitor" refers to any moiety (e.g., compound, nucleic acid sequence, antibody, etc.) that specifically inhibits the enzymatic activity of, or the expression of, IKKi.

As used herein, the terms "detect," "detecting" or "detection" may describe either the general act of discovering or discerning or the specific observation of a detectably labeled composition.

The term "RNA interference" or "RNAi" refers to the silencing or decreasing of gene expression by siNAs (e.g., "short interfering RNA", "siRNA", "short interfering nucleic acid molecule", "short interfering oligonucleotide molecule", or "chemically-modified short interfering nucleic acid molecule"). It is the process of sequence-specific, post-transcriptional gene silencing in animals and plants, initiated by siNA that is homologous in its duplex region to the sequence of the silenced gene. The gene (e.g., IKKi) may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

The term "short interfering nucleic acid," "siNA," "short interfering RNA," "siRNA," "short interfering nucleic acid molecule," "short interfering oligonucleotide molecule," or "chemically-modified short interfering nucleic acid molecule" as used herein refers to any nucleic acid molecule capable of inhibiting or down regulating gene expression or viral replication, for example by mediating RNA interference "RNAi" or gene silencing in a sequence-specific manner (see, e.g., Bass, 2001, Nature, 411, 428-429; Elbashir et al., 2001, Nature, 411, 494-498; and Kreutzer et al., International PCT Publication No. WO 00/44895; Zernicka-Goetz et al., International PCT Publication No. WO 01/36646; Fire, International PCT Publication No. WO 99/32619; Plaetinck et al., International PCT Publication No. WO 00/01846; Mello and Fire, International PCT Publication No. WO 01/29058; Deschamps-Depaillette, International PCT Publication No. WO 99/07409; and Li et al., International PCT Publication No. WO 00/44914; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237; Hutvagner and Zamore, 2002, Science, 297, 2056-60; McManus et al., 2002, RNA, 8, 842-850; Reinhart et al., 2002, Gene & Dev., 16, 1616-1626; and Reinhart & Bartel, 2002, Science, 297, 1831).

In some embodiments, the siNA can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The siNA can be assembled from two separate oligonucleotides, where one strand is the sense strand and the other is the antisense strand, wherein the antisense and sense strands are self-complementary (i.e., each strand comprises nucleotide sequence that is complementary to nucleotide sequence in the other strand; such as where the antisense strand and sense strand form a duplex or double stranded structure, for example wherein the double stranded region is about 19 base pairs); the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof. Alternatively, the siNA is assembled from a single oligonucleotide, where the self-complementary sense and antisense regions of the siNA are linked by means of a nucleic acid based or non-nucleic acid-based linker(s). The siNA can be a polynucleotide with a duplex, asymmetric duplex, hairpin or asymmetric hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. The siNA can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siNA molecule capable of mediating RNAi. The siNA can also comprise a single stranded polynucleotide having nucleotide sequence complementary to nucleotide sequence in a target nucleic acid molecule or a portion thereof (for example, where such siNA molecule does not require the presence within the siNA molecule of nucleotide sequence corresponding to the target nucleic acid sequence or a portion thereof), wherein the single stranded polynucleotide can further comprise a terminal phosphate group, such as a 5'-phosphate (see, e.g., Martinez et al., 2002, Cell., 110, 563-574 and Schwarz et al., 2002, Molecular Cell, 10, 537-568), or 5',3'-diphosphate. In certain embodiments, the siNA molecule of the invention comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der waals interactions, hydrophobic intercations, and/or stacking interactions. In certain embodiments, the siNA molecules of the invention comprise nucleotide sequence that is complementary to nucleotide sequence of a target gene (e.g., IKKi). In another embodiment, the siNA molecule of the invention interacts with nucleotide sequence of a target gene in a manner that causes inhibition of expression of the target gene. As used herein, siNA molecules need not be limited to those molecules containing only RNA, but further encompasses chemically-modified nucleotides and non-nucleotides. In certain embodiments, the short interfering nucleic acid molecules of the invention lack 2'-hydroxy (2'-OH) containing nucleotides. In some embodiments, siNA molecules do not require the presence of nucleotides having a 2'-hydroxy group for mediating RNAi and as such, short interfering nucleic acid molecules of the invention optionally do not include any ribonucleotides (e.g., nucleotides having a 2'-OH group). Such siNA molecules that do not require the presence of ribonucleotides within the siNA molecule to support RNAi can however have an attached linker or linkers or other attached or associated groups, moieties, or chains containing one or more nucleotides with 2'-OH groups. Optionally, siNA molecules can comprise ribonucleotides at about 5, 10, 20, 30, 40, or 50% of the nucleotide positions. The modified short interfering nucleic acid molecules of the invention can also be referred to as short interfering modified oligonucleotides "siMON." As used herein, the term siNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, translational inhibition, or epigenetics. For example, siNA molecules of the invention can be used to epigenetically silence genes at both the post-transcriptional level or the pre-transcriptional level. In a non-limiting example, epigenetic regulation of gene expression by siNA molecules of the invention can result from siNA mediated modification of chromatin structure to alter gene expression (see, e.g., Allshire, 2002, Science, 297, 1818-1819; Volpe et al, 2002, Science, 297, 1833-1837; Jenuwein, 2002, Science, 297, 2215-2218; and Hall et al., 2002, Science, 297, 2232-2237).

By "asymmetric hairpin" as used herein is meant a linear siNA molecule comprising an antisense region, a loop portion that can comprise nucleotides or non-nucleotides, and a sense region that comprises fewer nucleotides than the antisense region to the extent that the sense region has enough complimentary nucleotides to base pair with the antisense region and form a duplex with loop. For example, an asymmetric hairpin siNA molecule of the invention can comprise an antisense region having length sufficient to mediate RNAi in a cell or in vitro system (e.g. about 19 to about 22 nucleotides) and a loop region comprising about 4 to about 8 nucleotides, and a sense region having about 3 to about 18 nucleotides that are complementary to the antisense region. The asymmetric hairpin siNA molecule can also comprise a 5'-terminal phosphate group that can be chemically modified. The loop portion of the asymmetric hairpin siNA molecule can comprise nucleotides, non-nucleotides, linker molecules, or conjugate molecules as described herein.

By "asymmetric duplex" as used herein is meant a siNA molecule having two separate strands comprising a sense region and an antisense region, wherein the sense region comprises fewer nucleotides than the antisense region to the extent that the sense region has enough complimentary nucleotides to base pair with the antisense region and form a duplex. For example, an asymmetric duplex siNA molecule of the invention can comprise an antisense region having length sufficient to mediate RNAi in a cell or in vitro system (e.g. about 19 to about 22 nucleotides) and a sense region having about 3 to about 18 nucleotides that are complementary to the antisense region.

As used herein, the term "nucleic acid molecule" refers to any nucleic acid containing molecule, including but not limited to, DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

A gene, such as the insulin receptor, may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

As used herein, the term "probe" refers to an oligonucleotide (*i.e*., a sequence of nucleotides), whether occurring naturally as in a purified restriction digest or produced synthetically, recombinantly or by PCR amplification, that is capable of hybridizing to at least a portion of another oligonucleotide of interest. A probe may be single-stranded or double-stranded. Probes are useful in the detection, identification and isolation of particular gene sequences. In certain embodiments, a probe used in the present invention will be labeled with a "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

The term "isolated" when used in relation to a nucleic acid, as in "an isolated oligonucleotide" or "isolated polynucleotide" refers to a nucleic acid sequence that is identified and separated from at least one component or contaminant with which it is ordinarily associated in its natural source. Isolated nucleic acid is such present in a form or setting that is different from that in which it is found in nature. In contrast, non-isolated nucleic acids as nucleic acids such as DNA and RNA found in the state they exist in nature. For example, a given DNA sequence (*e.g.*, a gene) is found on the host cell chromosome in proximity to neighboring genes; RNA sequences, such as a specific mRNA sequence encoding a specific protein, are found in the cell as a mixture with numerous other mRNAs that encode a multitude of proteins. However, isolated nucleic acid encoding a given protein includes, by way of example, such nucleic acid in cells ordinarily expressing the given protein where the nucleic acid is in a chromosomal location different from that of natural cells, or is otherwise flanked by a different nucleic acid sequence than that found in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand *(i.e.,* the oligonucleotide or polynucleotide may be single-stranded), but may contain both the sense and anti-sense strands *(i.e.,* the oligonucleotide or polynucleotide may be double-stranded).

As used herein, the term "purified" or "to purify" refers to the removal of components (*e.g*., contaminants) from a sample. For example, antibodies are purified by removal of contaminating non-immunoglobulin proteins; they are also purified by the removal of immunoglobulin that does not bind to the target molecule. The removal of non-immunoglobulin proteins and/or the removal of immunoglobulins that do not bind to the target molecule results in an increase in the percent of target-reactive immunoglobulins in the sample. In another example, recombinant polypeptides are expressed in bacterial host cells and the polypeptides are purified by the removal of host cell proteins; the percent of recombinant polypeptides is thereby increased in the sample.

As used herein, the terms "subject" and "patient" refer to any animal, such as a mammal like a dog, cat, bird, livestock, and preferably a human (e.g. a human with a disease such as obesity, diabetes, or insulin resistance).

As used here, the term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

As used herein, the term "antibody fragments" refers to a portion of an intact antibody. Examples of antibody fragments include, but are not limited to, linear antibodies; single-chain antibody molecules; Fc or Fc' peptides, Fab and Fab fragments, and multispecific antibodies formed from antibody fragments. The antibody fragments preferably retain at least part of the hinge and optionally the CH1 region of an IgG heavy chain. In other preferred embodiments, the antibody fragments comprise at least a portion of the CH2 region or the entire CH2 region.

As used herein, the term "toxic" refers to any detrimental or harmful effects on a subject, a cell, or a tissue as compared to the same cell or tissue prior to the administration of the toxicant.

As used herein, the term "effective amount" refers to the amount of a composition (e.g., inhibitor of IKKi) sufficient to effect beneficial or desired results. An effective amount can be administered in one or more administrations, applications or dosages and is not intended to be limited to a particular formulation or administration route.

As used herein, the term "administration" refers to the act of giving a drug, prodrug, or other agent, or therapeutic treatment (e.g., compositions of the present invention) to a subject (e.g., a subject or *in vivo, in vitro,* or *ex vivo* cells, tissues, and organs). Exemplary routes of administration to the human body can be through the eyes (ophthalmic), mouth (oral), skin(transdermal, topical), nose (nasal), lungs (inhalant), oral mucosa (buccal), ear, by injection (e.g., intravenously, subcutaneously, intratumorally, intraperitoneally, etc.) and the like.

As used herein, the term "co-administration" refers to the administration of at least two agent(s) (e.g., IKKi siRNAs or antibodies and one or more other agents) or therapies to a subject. In some embodiments, the co-administration of two or more agents or therapies is concurrent. In other embodiments, a first agent/therapy is administered prior to a second agent/therapy. Those of skill in the art understand that the formulations and/or routes of administration of the various agents or therapies used may vary. The appropriate dosage for co-administration can be readily determined by one skilled in the art. In some embodiments, when agents or therapies are co-administered, the respective agents or therapies are administered at lower dosages than appropriate for their administration alone. Thus, co-administration is especially desirable in embodiments where the co-administration of the agents or therapies lowers the requisite dosage of a potentially harmful (e.g., toxic) agent(s).

As used herein, the term "pharmaceutical composition" refers to the combination of an active agent (e.g., IKKi antibody or IKKi-inhibiting agent) with a carrier, inert or active, making the composition especially suitable for diagnostic or therapeutic use *in vitro, in vivo* or *ex vivo.*

The terms "pharmaceutically acceptable" or "pharmacologically acceptable," as used herein, refer to compositions that do not substantially produce adverse reactions, e.g., toxic, allergic, or immunological reactions, when administered to a subject.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, crystals and industrial samples. Such examples are not however to be construed as limiting the sample types applicable to the present invention.

The term "homology" refers to a degree of complementarity. There may be partial homology or complete homology (i.e., identity). A partially complementary sequence is one that at least partially inhibits a completely complementary sequence from hybridizing to a target nucleic acid and is referred to using the functional term "substantially homologous." The term "inhibition of binding," when used in reference to nucleic acid binding, refers to inhibition of binding caused by competition of homologous sequences for binding to a target sequence. The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (i.e., the hybridization) of a completely homologous to a target under conditions of low stringency. This is not to say that conditions of low stringency are such that non-specific binding is permitted; low stringency conditions require that the binding of two sequences to one another be a specific (i.e., selective) interaction. The absence of non-specific binding may be tested by the use of a second target that lacks even a partial degree of complementarity (e.g., less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (e.g., the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (e.g., increasing the temperature of the hybridization and/or wash steps, the use of formamide in the hybridization solution, etc.).

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

As used herein, the term "competes for binding" is used in reference to a first polypeptide with an activity which binds to the same substrate as does a second polypeptide with an activity, where the second polypeptide is a variant of the first polypeptide or a related or dissimilar polypeptide. The efficiency (e.g., kinetics or thermodynamics) of binding by the first polypeptide may be the same as or greater than or less than the efficiency substrate binding by the second polypeptide. For example, the equilibrium binding constant (K_{D}) for binding to the substrate may be different for the two polypeptides. The term "Kₘ" as used herein refers to the Michaelis-Menton constant for an enzyme and is defined as the concentration of the specific substrate at which a given enzyme yields one-half its maximum velocity in an enzyme catalyzed reaction.

As used herein, the term "hybridization" is used in reference to the pairing of complementary nucleic acids. Hybridization and the strength of hybridization (i.e., the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "Tₘ" is used in reference to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ =81.5+0.41(% G+C), when a nucleic acid is in aqueous solution at 1 M NaCl (See e.g., Anderson and Young, Quantitative Filter Hybridization, in Nucleic Acid Hybridization [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. Those skilled in the art will recognize that "stringency" conditions may be altered by varying the parameters just described either individually or in concert. With "high stringency" conditions, nucleic acid base pairing will occur only between nucleic acid fragments that have a high frequency of complementary base sequences (e.g., hybridization under "high stringency" conditions may occur between homologs with about 85-100% identity, preferably about 70-100% identity). With medium stringency conditions, nucleic acid base pairing will occur between nucleic acids with an intermediate frequency of complementary base sequences (e.g., hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

"High stringency conditions" when used in reference to nucleic acid hybridization comprise conditions equivalent to binding or hybridization at 42° C. in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5.times. Denhardt's reagent and 100 ug/ml denatured salmon sperm DNA followed by washing in a solution comprising 0.1X SSPE, 1.0% SDS at 42° C. when a probe of about 500 nucleotides in length is employed.

"Medium stringency conditions" when used in reference to nucleic acid hybridization comprise conditions equivalent to binding or hybridization at 42° C. in a solution consisting of 5X.SSPE (43.8 g/l NaCl, 6.9 g/l NaH₂PO₄H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X.SSPE, 1.0% SDS at 42° C. when a probe of about 500 nucleotides in length is employed.

"Low stringency conditions" comprise conditions equivalent to binding or hybridization at 42° C. in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 µg/ml denatured salmon sperm DNA followed by washing in a solution comprising 5X SSPE, 0.1% SDS at 42° C. when a probe of about 500 nucleotides in length is employed.

The present invention is not limited to the hybridization of probes of about 500 nucleotides in length. The present invention contemplates the use of probes between approximately 10 nucleotides up to several thousand (e.g., at least 5000) nucleotides in length. One skilled in the relevant understands that stringency conditions may be altered for probes of other sizes (See e.g., Anderson and Young, Quantitative Filter Hybridization, in Nucleic Acid Hybridization [1985] and Sambrook et al., Molecular Cloning: A Laboratory Manual, Cold Spring Harbor Press, NY [1989]).

The following terms are used to describe the sequence relationships between two or more polynucleotides: "reference sequence", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA sequence given in a sequence listing or may comprise a complete gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman [Smith and Waterman, Adv. Appl. Math. 2: 482 (1981)] by the homology alignment algorithm of Needleman and Wunsch [Needleman and Wunsch, J. Mol. Biol. 48:443 (1970)], by the search for similarity method of Pearson and Lipman [Pearson and Lipman, Proc. Natl. Acad. Sci. (U.S.A.) 85:2444 (1988)], by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (i.e., resulting in the highest percentage of homology over the comparison window) generated by the various methods is selected. The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence, for example, as a segment of the full-length sequences of the compositions claimed in the present invention (e.g., nucleic acid sequences encoding IKKi peptide or a fragment thereof).

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity or more (e.g., 99 percent sequence identity). Preferably, residue positions that are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

As used herein, the term "IKKi" refers to inhibitor of kappa light polypeptide gene enhancer in B-cells, kinase epsilon and all alternative terms used to reference said protein and the gene encoding it, including but not limited to: I kappa-B kinase epsilon, IkBKE, IKKE, IKK-E, IKK-epsilon, IKKI, IKK-i, Inducible I kappa-B kinase, Inhibitor of nuclear factor kappa-B kinase epsilon subunit, Inhibitor of nuclear factor kappa-B kinase subunit epsilon, KIAA0151, MGC125294, MGC125295, MGC125297, AW558201, Ikke, IKKepsilon, Ikki, IKK-i, Inducible I kappa-B kinase, Inhibitor of nuclear factor kappa-B kinase epsilon subunit, Inhibitor of nuclear factor kappa-B kinase subunit epsilon, IKK3, IKKε and IKK 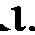 .

As used herein, the term "phospho-specific antibody" refers to an antibody that can differentiate between an antigen that bears a covalent phosphate modification and one that does not. A phospho-specific antibody may be specific for a non-phosphorylated form of an antigen, or it may be specific for a phosphorylated form. In some cases, the epitope recognized by the phospho-specific antibody is known. In some cases, it is not. Methods for the generation and use of phospho-specific antibodies are known in the art (e.g., Taya et al, In: Tumor Suppressor Genes: Vol. 2 Regulation, Function, and Medicinal Applications, Methods Mol. Biol., 223, 17-26, 2003; U.S. Pat. No. 6,309,863; U.S. Pat. No. 6,924,361; Sykes et al, Current Proteomics, 3, 113-117, 2006).

### DETAILED DESCRIPTION

The present invention provides diagnostics, screening methods, and treatment methods related to obesity, insulin resistance, diabetes, weight loss, and related disorders. In particular, the present invention provides methods of treating such conditions with IKKi inhibitors, methods of diagnosing such conditions based on IKKi status, and methods of screening candidate IKKi inhibitors.

Work conducted during the development of embodiments of the present invention demonstrated that activated IKKi phosphosphorylates the insulin receptor, thereby blocking the activity of the insulin receptor in the insulin signaling pathway. Preventing the activation of IKKi (e.g., by using IKKi inhibitors) therefore is useful in reducing body fat, increasing percent lean body mass, as well as treating diseases and conditions such as obesity, insulin resistance, diabetes, and related disorders. While the present invention is not limited to any particular mechanism, and an understanding of the mechanism is not necessary to practice the present invention, it is believed that inhibition of IKKi allows, for example, the natural activity of insulin to promote glucose metabolism, thereby reducing body fat and treating diabetes, obesity, and related conditions. Determining the activity level of IKKi, therefore, is also useful for diagnosing conditions such as obesity, insulin resistance, and related disorders. Again, while the present invention is not limited to any particular mechanism, it is believed that determining the activity level of activated IKKi provides information on if, and how much, the insulin receptor is being inhibited by IKK. This in turn is diagnostic of certain conditions that are involved with improper glucose metabolism.

Insulin resistance is characterized by defects in both insulin-stimulated glucose transport in muscle and fat and insulin-dependent suppression of glucose output in the liver (Saltiel, 2001; Taniguchi et al., Nat. Rev. Mol. Cell Biol. 7, 85-96. 2006; Thirone et al., Trends Endocrinool. Metab. 17, 72-78,2006). Numerous longitudinal studies suggest that insulin resistance is the first step in the development of Type 2 diabetes, particularly in obese patients. Obesity is correlated with increased circulating levels of pro-inflammatory cytokines including TNFα, IL-6, IL-18, IL-1ß, and CRP (Hotamisligil, Nature, 444, 860-867,2006; Shoelson et al., Gastroentrol, 132, 2169-2180, 2007; Wellen et al., J. Clin. Invest., 115, 1111-1119, 2005). Many of these cytokines can block insulin action, indicating a possible inflammatory link between obesity and Type 2 diabetes (Schenk et al., J. Clin. Invest., 118, 2992-3002, 2008). Inflammation has been observed in both liver and adipose tissue from obese rodents and humans (Odegaard et al., Nat. Clin. Pract. Endocrinol. Metab. 11, 212-217, 2008; Schenk et al., J. Clin. Invest., 118, 2992-3002, 2008), and targeted deletion of genes involved in inflammatory processes, including CCR2 (Tamura et al., Arterioscler. Thromb. Vasc. Biol. 28, 2195-2201, 2008; Weisberg et al., J. Clin. Invest., 112, 1796-1808, 2006), MCP1 (Kanda et al., J. Clin. Invest., 116, 1494-1505, 2006), TNFα (Hotamisligil et al., Science, 259, 87-91, 1993; Moller, Trends Endocrinol. Metabl, 11,212-217, 2000), TLR4 (Shi et al., J. Clin. Invest,, 116, 3015-3025, 2006), JNK1 (Hirosumi et al., Nature, 420, 333-336, 2002; Sabio et al., Science, 322, 1539-1543, 2008; Solinas et al., Cell Metab., 6, 386-397, 2007; Tuncman et al., PNAS, 103, 10741-10746, 2006), CAP (Lesniewski et al., Nat. Med., 13,455-462, 2007) and others (Franckhauser et al., Diabetologia, 51, 1306-1316, 2008; Odegaard et al., Nature, 447, 1116-1120, 2007; Wellen et al., Cell, 129, 537-548, 2007), appears to disrupt the link between dietary or genetic obesity and insulin resistance. However, the initial steps in the generation of this inflammatory state, the primary signals involved and the tissues in which insulin action is impaired, remain uncertain.

A number of studies have indicated an important role for NFκB in linking obesity and insulin resistance (Tilg et al., Mol. Med., 14, 222-231, 2008; Wunderlich et al., PNAS, 105, 1297-1302, 2008). This pathway may be activated downstream of the toll-like receptor-4 (TLR4) due to its interactions with dietary fatty acids (Kim et al., Circ. Res., 100, 1589-1596, 2007; Tsukumo et al., Diabetes, 56, 1986-1998, 2007), or as a consequence of hypoxia associated with obesity (Schenk et al., J. Clin. Invest., 118, 2992-3002, 2008; Ye et al., Am. J. Physiol. Endocrinol., Metabl., 293, E1118-1128, 2007). Most studies implicating NFκB have relied on the targeted deletion (Arkan et al., Nat. Med., 11, 191-198, 2005; Cai et al.,Nat. Med., 11, 183-190, 2005; Zhang et al.,Cell, 135, 61-73, 2008) or pharmacological inhibition (Yin et al., Nature, 396, 77-80, 1998; Yuan et al., Science, 293, 1673-1677, 2001) of the kinase IKKβ, which lies upstream of the inhibitory IκB proteins. Upon phosphorylation, IκB undergoes proteasomal degradation, and is released from the associated NFκB transcription factor, permitting its translocation to the nucleus and transcription of numerous inflammatory genes (Akira et al., Nat. Rev. Immunol., 4, 499-511, 2004; Kawai et al., Trends Mol. Med., 13,460-469,2007). Hepatocyte-specific IKKβ knockout mice fed a high fat diet retain liver insulin sensitivity but develop insulin resistance in fat and muscle. In contrast, myeloid-specific IKKβ knockout mice are protected from diet-induced global insulin resistance, but not obesity (Arkan et al., Nat. Med., 11, 191-198, 2005). Additionally, high dose salicylates, which inhibit IKKβ activity, improve glucose tolerance in obese mice (Kim et al., Circ. Res., 100, 1589-1596, 2007; Yin et al., Nature, 396, 77-80, 1998) and in patients with type 2 diabetes (Fleischman et al., Diabetes Care, 31, 289-294, 2008; Grilli et al., Science, 274, 1383-1385, 1996; Kopp et al., Science, 265, 956-959, 1994; Koska et al., Diabetologia, 52, 385-393, 2008).

The IKK (IκB Kinase) family of proteins is comprised of four members, IKKα, IKKβ, IKKi (or ε) and TBK1 (TANK Binding Kinase 1) (Hacker et al., Science STKE, 357, re13,, 2006; Kawai et al., Trends Mol. Med., 13, 460-469, 2007). While IKKα and β activate the canonical NFκB pathway, the roles of IKKi and TBK1 are less well understood. Expression of IKKi is induced in myeloid cells after inflammatory stimuli, partially as a result of NFκB activation (Shimada et al., Int. Immunol., 11, 1357-1362, 1999), whereas TBK1 is more ubiquitously expressed (Pomerantz et al., EMBO J., 18, 6694-6704, 1999). These atypical IKKs can enhance NFκB transcriptional activity through phosphorylation of RelA (Adli et al., J. Biol. Chem., 281, 26976-26984, 2006; Buss et al., J. Biol. Chem., 279, 55633-55643, 2004), but are thought to mainly regulate transcription via the phosphorylation of the transcription factors Interferon Regulatory Factor-3 and 7 (IRF3 and IRF7) (Peters et al., Mol. Cell, 5, 513-522, 2000; Sharma et al., Science, 300, 1148-1151,2003) .

Despite strong evidence for an inflammatory link between obesity and diabetes, the primary site or sites at which the inflammatory response occurs has not yet been established. Adipose tissue responds to overnutrition, perhaps through the generation of endoplasmic reticulum or oxidative stress (Hotamisligil et al., Nat. Rev. Immunol., 8, 923-934, 2008; Wellen et al., J. Clin. Invest., 115, 1111-1119, 2005), by secreting cytokines or chemokines that recruit proinflammatory, M1 polarized macrophages to adipose tissue (Lumeng et al., Diabetes, 56, 16-23, 2007). These in turn secrete more cytokines that attenuate insulin action in adipocytes, resulting in increased lipolysis and free fatty acid release (Feingold et al., Endocrinol., 130, 10-16, 1992; Green et al., Endocrinol., 134, 2581-2588, 1994). Evidence indicates that liver also undergoes an inflammatory response due to genetic or dietary obesity by secreting proinflammatory cytokines (Ramadori et al., J. Physiol., Pharmacol., Suppl. 1, 107-117, 2008). However, the molecular details underlying macrophage recruitment and activation, the subtypes involved, their crosstalk with muscle, fat and liver cells, and the manner by which they regulate energy expenditure and storage remain uncertain. Experiments conducted during the development of embodiments of the present invention show that high fat diet induces the expression of IKKi in both liver and white adipose tissue, and further that mice bearing a targeted deletion of IKKi are surprisingly protected from diet-induced obesity, liver and adipose inflammation, hepatic steatosis, and insulin resistance, providing an appealing therapeutic target for obesity and type 2 diabetes.

### I. Insulin/Insulin-Receptor Signaling Pathway

### Insulin-stimulated glucose transport is mediated by the transporter Glut4.

The links between the innate immune system, inflammation and insulin resistance suggest that numerous mechanisms have emerged to modulate insulin sensitivity, reinforcing the importance of understanding the basic mechanisms of insulin action. Glucose transport is the rate-limiting step by which insulin increases glucose storage and utilization, and is mediated by the facilitative transporter Glut4. Insulin increases glucose uptake in muscle and fat mainly by enriching the concentration of Glut4 proteins at the plasma membrane. This is a process of regulated recycling, in which the endocytosis, sorting, exocytosis, tethering, docking and fusion of the protein are tightly regulated. In the absence of insulin, or after its receptor is inactivated, Glut4 is internalized via classical endocytotic processes. In adipocytes, these vesicles are retained in a perinuclear region in the cell and traffic to discrete sites at the plasma membrane for tethering, docking and fusion in response to insulin.

*Signaling from the Insulin Receptor.*

The insulin receptor (IR) is a heterotetrameric bi-functional complex, composed of two extracellular α subunits that bind insulin and two transmembrane β subunits with tyrosine kinase activity. Insulin binding to the α subunit stimulates the transphosphorylation of one β subunit by another on specific tyrosine residues in an activation loop, resulting in the increased catalytic activity of the kinase, and increased autophosphorylation at other tyrosine residues in the juxtamembrane regions and intracellular tail. The activated IR then phosphorylates intracellular substrates that include the insulin receptor substrate family (IRS1-4), APS and Cbl family members. Some of these proteins, such as IRS-1, are recruited to a juxtamembrane region in the receptor containing an NPXY motif, while APS and IRS-2 bind directly to the activation loop. Upon phosphorylation, IR substrates interact with a series of effector or adapter molecules containing Src homology 2 (SH2) domains that specifically recognize different phosphotyrosine motifs.

### The PI 3-Kinase Pathway and Glucose Uptake.

The IRS family of proteins is the best characterized of receptor substrates. IRS-1-knockout mice are insulin resistant in peripheral tissues with impaired glucose tolerance. IRS-2 knockout mice are insulin resistant in both peripheral tissues and liver, and develop type 2 diabetes due to insulin resistance along with decreased β-cell function.

Upon tyrosine phosphorylation, IRS proteins interact with the p85 regulatory subunit of PI 3-kinase, leading to the activation of the enzyme and its targeting to the plasma membrane. The enzyme generates the lipid product phosphatidylinositol 3,4,5-trisphosphate (PIP₃), which regulates the localization and activity of numerous proteins. Blockade of the enzyme with pharmacological inhibitors completely inhibits the stimulation of glucose uptake. Overexpression of dominant-interfering forms of PI 3-kinase blocks glucose uptake, and overexpression of constitutively active forms partially mimic insulin action. Targeted deletion of the p85 PI 3-kinase regulatory subunit in mice increases insulin sensitivity, enhancing glucose uptake and disposal, presumably due to increased PI kinase activity. Conversely, gene knockout of the catalytic subunit results in insulin resistance and glucose intolerance.

Insulin-stimulated increases in PIP₃ result in the recruitment of pleckstrin homology (PH) domain-containing proteins, including various enzymes, their substrates, adapter molecules, and cytoskeletal proteins. Among these is PDK1, which phosphorylates the kinases Akt1-3, PKCζ/λ and SGK. The protein kinase mTOR, complexed to the regulatory protein Rictor, has been identified as PDK2. PIP₃ mediates the translocation of Akt to the plasma membrane, via its PH domain, for phosphorylation. Overexpression of a membrane-bound form of Akt in 3T3L1 adipocytes increased the localization of Glut4 to the plasma membrane; insulin-stimulated Glut4 translocation was inhibited by expression of a dominant-interfering Akt mutant; and knock down or knockout of Akt blocks insulin action.

Despite the evidence supporting an important role for the PI 3-kinase pathway, activation of this enzyme is not sufficient for insulin-stimulated glucose transport. Stimulation of PI 3-kinase activity by PDGF or interleukin 4 does not increase glucose uptake. Numerous insulin receptor mutants have been identified in which the stimulation of glucose uptake and PI-kinase are differentially regulated. Overexpression of the IRS1 PTB domain decreased IRS1-associated PI 3-kinase activity, but was without effect on insulin-stimulated glucose uptake. Moreover, addition of a membrane-permeable analog of PIP₃ did not stimulate glucose uptake in the absence of insulin. Consistent with this, overexpression of constitutively active PI 3-kinase mutants did not fully mimic insulin-stimulated Glut4 translocation to the plasma membrane. Together these data suggest that PI 3-kinase activation is not sufficient to stimulate glucose uptake.

### Insulin Signaling from Lipid Rafts

Several studies have shown that a separate insulin signaling pathway is localized in lipid raft microdomains, specialized regions of the plasma membrane enriched in cholesterol, sphingolipids, glycolipids, GPI-anchored proteins, and lipid-modified signaling proteins. At least some of the insulin receptor has been shown to reside in these microdomains, perhaps through its interaction with the raft protein caveolin. Activation of the insulin receptor in these plasma membrane subdomains stimulates the tyrosine phosphorylation of the proto-oncogenes c-Cbl and Cbl-b. This phosphorylation step requires recruitment of Cbl to the adapter protein APS (see below). Upon binding to the receptor, APS is phosphorylated on a C-terminal tyrosine, resulting in the recruitment of Cbl via the SH2 domain of the latter protein. Cbl subsequently undergoes phosphorylation on three tyrosines.

The Cbl Associated Protein (CAP) is recruited with Cbl to the insulin receptor:APS complex. CAP is a bi-functional adapter protein with three SH3 domains, and an amino-terminal region similar to the gut peptide Sorbin, called the Sorbin Homology (SoHo) domain. CAP is found in insulin-sensitive tissues, and expression is increased by activation of PPARγ, the receptor for the thiazolidinedione class of insulin-sensitizing drugs.

The carboxyl-terminal SH3 domain of CAP associates with a PXXP motif in Cbl, such that these proteins are constitutively associated. Upon recruitment to the insulin receptor, CAP interacts with the lipid raft domain protein flotillin via its SoHo domain. Overexpression of dominant-interfering CAP mutants that do not bind to Cbl or flotillin blocked translocation of phosphorylated Cbl to lipid rafts, and also prevented insulin-stimulated glucose uptake and Glut4 translocation.

Upon tyrosine phosphorylation, Cbl interacts with the protein CrkII, an SH2/SH3-containing adapter protein. CrkII binds to Cbl via its SH2 domain, and is constitutively associated with the nucleotide exchange factor C3G via its SH3 domain. Thus, insulin stimulates the translocation of both CrkII and C3G to lipid rafts, an effect of the hormone that can be blocked by transfection of cells with CAPΔSH3 or CAPΔSoHo. Upon its translocation, C3G catalyzes activation of the small Rho family G proteins, TC10α and TC10β. SiRNA-mediated knockdown studies indicate that activation of TC10α but not β is required for the stimulation of glucose uptake by insulin. Together, these data indicate that the CAP/Cbl/TC10 pathway is required for insulin-stimulated glucose uptake in parallel with, and independent of the PI 3-kinase signaling cascade. Moreover, numerous studies have demonstrated alterations in the CAP/Cbl/TC10 pathway in states of obesity and insulin resistance.

### II. IKKi

IKKi is a kinase that is related to IKKα and IKKβ (Shimada et al., Int. Immunol., 11: 1357-1362 (1999)). Although IKKi has homology with IKKα and IKKß, the amino acid identity between IKKi and IKKβ is only 24% in the kinase domain. Over- expression of IKKi activates NFκB. IKKi is expressed preferentially in immune cells, and is induced in response to LPS or inflammatory cytokines. The kinase activity can be regulated by IKKi expression levels (Shimada et al.). IKKi phosphorylates the IkB proteins of the complex that inhibits NFκB activity. Phosphorylation of these IkB proteins causes them to be degraded, which allows NFκB to become active.

As described above, during the development of the present invention, it was determined that IKKi is a responsible for phosphorylating the insulin receptor, thereby inhibiting the role of insulin in proper glucose metabolism. IKKi is also known as inducible I Kappa B kinase, as well as IKKε and IKK3. In states of overnutrition, inflammatory macrophages infiltrate adipose tissue, and secrete cytokine that impair insulin action, in particular blocking the anti-lipolytic effects of the hormone, which results in increased lipolysis and fatty acid production. These secreted cytokines and fatty acids can activate the NFκB pathway in both macrophages and adipocytes. The inducible I Kappa B kinase (IKKi) is a protein kinase that lies in this pathway, induced by both fatty acids and cytokines such as TNFa and IL-6, and in turn initiating an inflammatory pathway(s). As described in the Example below, upon activation, IKKi induces insulin resistance by catalyzing phosphorylation of the insulin receptor, thereby blocking its interaction with, and tyrosine phosphorylation of, substrates, in the process of attenuating insulin action. IKKi is induced in both adipocytes and adipose tissue macrophages derived from high fat diet-fed mice compared to those from mice fed a control diet. As described in the Examples below, mice in which the IKKi gene was ablated were resistant to the effects of high fat diet. Unlike their control littermates, these mice do not become obese or insulin resistant upon prolonged high fat feeding. The consensus amino acid and nucleic acid sequences for murine IKKi are shown in Figure 24, while the consensus amino acid and nucleic acid sequences for human IKKi are shown in Figure 25.

### III. IKKi Inhibitors

The present invention is not limited by the type of IKKi inhibitors that are employed for therapeutic treatment of obesity, diabetes, insulin resistance, glucose metabolism disorders and conditions, as well as treatment for reducing body fat and increasing percent lean body mass. Exemplary compounds are discussed below. Additional IKKI inhibitors may be identified by the screening methods described in part IV. In preferred embodiments, the IKKi inhibitors inhibit the insulin receptor phosphorylation activity of IKKi. In certain embodiments, the IKKi inhibitors inhibit the insulin receptor phosphorylation activity of IKKi at the serine in the insulin receptor sequence VKTVNES (SEQ ID NO:15) or at a corresponding serine in non-human insulin receptor sequences (which can be identified using, for example, sequence alignments). In some embodiments, the IKKi inhibitors inhibit the insulin receptor phosphorylation activity of IKKi, but do not inhibit other activities, including other kinase activities, of IKKi.

Exemplary agents that inhibit IKKi expression or activity include small interfering RNAs (siRNAs), ribozymes, antisense nucleic acids, kinase inhibitors, anti-IKKi antibodies, small molecules, peptides, mutant IKKi polypeptides and the like. In some embodiments, the IKKi inhibitor is an nucleic acid sequence that can inhibit the functioning of an IKKi RNA (e.g., such as the sequences shown in Figures 24 and 25). Nucleic acids that can inhibit the function of an IKKi RNA can be generated from coding and non-coding regions of the IKKi gene. However, nucleic acids that can inhibit the function of an IKKi RNA are often selected to be complementary to sequences near the 5' end of the coding region. Hence, in some embodiments, the nucleic acid that can inhibit the functioning of an IKKi RNA can be complementary to sequences near the 5' end of SEQ ID NO: 11 (murine) or SEQ ID NO: 13 (human). In other embodiments, nucleic acids that can inhibit the function of an Ikki RNA from other species (e.g., mouse, rat, cat, dog, goat, pig or a monkey IKKi RNA).

A nucleic acid sequence that can inhibit the functioning of an IKKi RNA need not be 100% complementary to a selected region of SEQ ID NOS: 11 or 13, or closely related sequences. Instead, some variability in the sequence of the nucleic acid that can inhibit the functioning of an IKKi RNA is permitted, as functionality can be determined in the screening assays described below. For example, a nucleic acid that can inhibit the functioning of a human IKKi RNA can be complementary to a nucleic acid encoding a mouse or rat IKKi gene product. Nucleic acids encoding mouse IKKi gene product, for example, can be found in the NCBI database at GenBank Accession No. AB016589, NM 019777, NT 0399180, and CCDS 15269.1; a mouse IKKi polypeptide sequence has GenBank Accession No. NP 062751 or CCDS 15269.1; and a rat IKKi cDNA is GenBank Accession No. XM 344139.

Moreover, nucleic acids that can hybridize under moderately or highly stringent hybridization conditions are sufficiently complementary to inhibit the functioning of an IKK RNA and can be utilized in the compositions of the invention. Generally, stringent hybridization conditions are selected to be about 5°C lower than the thermal melting point (Tm) for the specific sequence at a defined ionic strength and pH. However, stringent conditions encompass temperatures in the range of about 1°C to about 20°C lower than the thermal pointing point of the selected sequence, depending upon the desired degree of stringency as otherwise qualified herein. In some embodiments, the nucleic acids that can inhibit the functioning of IKKi RNA can hybridize to an IKKi RNA under physiological conditions, for example, physiological temperatures and salt concentrations.

Precise complementarity is therefore not required for successful duplex formation between a nucleic acid that can inhibit an IKKi RNA and the complementary coding sequence of an IKKi RNA. Inhibitory nucleic acid molecules that comprise, for example, 2, 3, 4, or 5 or more stretches of contiguous nucleotides that are precisely complementary to an IKKi coding sequence, each separated by a stretch of contiguous nucleotides that are not complementary to adjacent IKKi coding sequences, can inhibit the function of IKKi mRNA.

In general, each stretch of contiguous nucleotides is at least 4, 5, 6, 7, or 8 or more nucleotides in length. Non-complementary intervening sequences are preferably 1, 2, 3, or 4 nucleotides in length. One skilled in the art can use the calculated melting point of a nucleic acid hybridized to a sense nucleic acid to estimate the degree of mismatching that will be tolerated between a particular nucleic acid for inhibiting expression of a particular IKKi RNA.

In some embodiments a nucleic acid that can inhibit the function of an endogenous IKKi RNA is an anti-sense oligonucleotide. The anti-sense oligonucleotide is complementary to at least a portion of the coding sequence of an IKKi gene sequence, such as SEQ ID NO: 11 or 13. Such anti-sense oligonucleotides are generally at least six nucleotides in length, but can be about 8, 12, 15, 20, 25, 30, 35, 40, 45, or 50 nucleotides long. Longer oligonucleotides can also be used. IKKi anti-sense oligonucleotides can be provided in a DNA construct, or expression cassette and introduced into cells whose division is to be decreased, for example, into cells expressing IKKi, such as adipocytes or macrophages.

In one embodiment of the invention, expression of an IKKi gene is decreased using a ribozyme. A ribozyme is an RNA molecule with catalytic activity (See, e.g., Cech, 1987, Science 236: 1532-1539; Cech, 1990, Ann. Rev. Biochem. 59: 543-568; Cech, 1992, Curr. Opin. Struct. Biol. 2: 605-609; and Couture and Stinchcomb, 1996, Trends Genet. 12: 510-515. Ribozymes can be used to inhibit gene function by cleaving an RNA sequence, as is known in the art (see, e. g., Haseloff et al., U.S. Pat. No. 5,641, 673).

IKKi nucleic acids complementary IKKI sequences, such as SEQ ID NOS: 11 or 13 can be used to generate ribozymes that will specifically bind to mRNA transcribed from an IKKi gene. Methods of designing and constructing ribozymes that can cleave other RNA molecules in trans in a highly sequence specific manner have been developed and described in the art (see Haseloff et al. (1988), Nature 334: 585-591). For example, the cleavage activity of ribozymes can be targeted to specific RNAs by engineering a discrete "hybridization" region into the ribozyme. The hybridization region contains a sequence complementary to the target RNA and thus specifically hybridizes with the target. The target sequence can be a segment of about 10, 12, 15, 20, or 50 contiguous nucleotides selected from a nucleotide sequence such as SEQ ID NOS: 11 or 13. Longer complementary sequences can be used to increase the affinity of the hybridization sequence for the target. The hybridizing and cleavage regions of the ribozyme can be integrally related; thus, upon hybridizing to the target RNA through the complementary regions, the catalytic region of the ribozyme can cleave the target.

RNA interference (RNAi) involves post-transcriptional gene silencing (PTGS) induced by the direct introduction of dsRNA. Small interfering RNAs (siRNAs) are generally 21-23 nucleotide dsRNAs that mediate post-transcriptional gene silencing. Introduction of siRNAs can induce post-transcriptional gene silencing in mammalian cells. siRNAs can also be produced in vivo by cleavage of dsRNA introduced directly or via a transgene or virus. Amplification by an RNA-dependent RNA polymerase may occur in some organisms. siRNAs are incorporated into the RNA-induced silencing complex, guiding the complex to the homologous endogenous mRNA where the complex cleaves the transcript.

Rules for designing siRNAs are known in the art (see, e.g., Elbashir et al., 2001, Nature 411: 494-498; J. Harborth, S.). Thus, an effective siRNA can be made by selecting target sites within an IKKi sequence, such as SEQ ID NOS: 11 or 13 that begin with AA, that have 3' UU overhangs for both the sense and antisense siRNA strands, and that have an approximate 50% G/C content. In some embodiments, an siRNA can be a double-stranded RNA having one of the following sequences:
AAUUACCUGU GGCACACAGA UU (SEQ ID NO:14)
AAGGCCCGCA ACAAGAAAUC CUU (SEQ ID NO: 15)
AACAAGAAAU CCGGAGAGCU GUU (SEQ ID NO:16)
AAAUCCGGAG AGCUGGUUGC UU (SEQ ID NO:17)
AAGGUCUUCA ACACUACCAG CU (SEQ ID NO: 18).

In some embodiments, the IKKi inhibitor is small interfering RNAs with one of the following sequences: 5'-GUGAAGGUCUUCAACACUACC-3' (SEQ ID NO:19) and 5'-UAGUGUUGAAGACCUUCACAG-3' (SEQ ID NO: 20).

In certain embodiments, the IKKi inhibitor is a small molecule. For example, in particular embodiments, the IKKi inhibitor is 5-(5,6-Dimethoxy-1H-benzimidazol-1-yl)-3-[[2-(methylsulfonyl)phenyl]methoxy]-2-thiophenecarbonitrile. In particular embodiments, the IKKi inhibitor is a benzimidazol substituted thiopene derivative, such as those described in WO2005/075465. Exemplary IKKi inhibitors are described by the following formula: 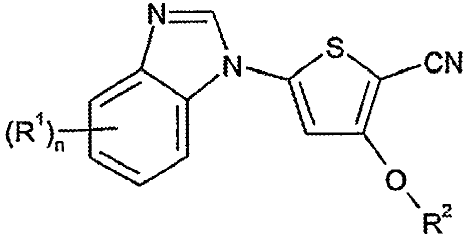
wherein n is 0, 1, 2, 3, or 4;
each R¹ which may be the same or different, independently represents H, halogen or a group (X)a (Y)bZ;
X represents -O- or -CONH-;
a is 0 or 1;
Y represents -C₁₋₆ alkylene-
b is 0 or 1;
Z represents hydroxy, C₁₋₆alkyl, C₁₋₆, haloalkyl, C₅₋₇ heterocyclyl, C₁₋₆ alkoxyalkyl, C₁₋₆ haloalkoxyalkyl;
R² represents a group -(X¹)c(Y¹)dZ¹
Wherein X¹ represents -C₁₋₁₂ alkylene-;
c is 0 or 1;
Y1 represents -O-;
d is 0 or 1;
0Z¹ represents H, aryl or heteroaryl each of which contains 5-14 ring atoms, C₅₋₇ heterocyclyl, C₅₋₇ cycloalkyl, C₅₋₇ cycloalkenyl, (each of which aryl, heteroaryl, heterocyclyl, cycloalkyl, cycloalkenyl may be optionally substituted by one or more substituents independently selected from C₁₋₆ alkyl, C₁₋₆ haloalkyl, halogen, C₁₋₆ alkoxy, C₁₋₆ haloalkoxy, S0₂R³, C₁₋₆ hydroxyalkyl);
R³ represents H or C₁₋₆ alkyl;
or pharmaceutically acceptable salts, solvates or physiologically derivatives thereof.

In other embodiments, the IKKi inhibitor is selected from a compound shown in Table 1 below:

**TABLE 1**

| |
|---|
| 5-[5,6-bis(methyloxy)-1H-benzimidazol-1-yl]-3-({[4-(hydroxymethyl)phenyl]methyl}-oxy)-2-thiophenecarbonitrile |
| 5-[5,6-bis(methyloxy)-1H-benzimidazol-1-yl]-3-{[(1*R*)-1-(2-chlorophenyl)ethyl]oxy}-2-thiophenecarbonitrile |
| 5-(1*H*-benzimidazol-1-yl)-3-{(2-methylphenyl)methyl]oxy}-2-thiophenecarbonitrile |
| 5-(1*H*-benzimidazol-1-yl)-3-[(phenylmethyl)oxy]-2-thiophenecarbonitrile |
| 5-[5,6-bis(methyloxy)-1H-benzimidazol-1-yl]-3-({[1-methyl-1*H*-1,2,3-benzotriazol-6-yl)methyl]oxy}-2-thiophenecarbonitrile) |
| 5-(6-(methyloxy)-5-{[2-(4-morpholinyl)ethyl]oxy}-1H-benzimidazol-1-yl)-3-({[2-(trifluoromethyl)phenyl]methyl}oxy)-2-thiophenecarbonitrile |
| 5-[5,6-bis(methyloxy)-1H-benzimidazol-1-yl]-3-{[(2,5-difluorophenyl)methyl]oxy}-2-thiophenecarbonitrile |
| 5-[5,6-bis(methyloxy)-1H-benzimidazol-1-yl]-3-({[2-(trifluoromethyl)phenyl]methyl}oxy)-2-thiophenecarbonitrile |
| 5-[5,6-bis(methyloxy)-1H-benzimidazol-1-yl]-3-({[2-(methylsulfonyl)phenyl]methyl}oxy)-2-thiophenecarbonitrile |
| 5-(5-chloro-1H-benzimidazol-1-yl)-3-[(phenylmethyl)oxyl-2-thiophenecarbonitrile |
| 5-[5,6-bis(methyloxy)-1H-benzimidazol-1-yl]-3-{[(2,6-difluorophenyl)methyl]oxy}-2- thiophenecarbonitrile |
| 5-[5-[(3-hydroxypropyl)oxy]-6-(methyloxy)-1H-benzimidazol-1-yl]-3-({[2-(trifluoromethyl)phenYllmethyl}oxy)-2-thiophene-carbonitrile |
| 5-[5,6-bis(methyloxy)-1H-benzimidazol-1-yl]-3-{[(3-bromo-4-pyridinyl)methyl]oxy}-2-thiophenecarbonitrile |
| 5-[5,6-bis(methyloxy)-1H-benzimidazol-1-yl]-3-[(cyclohexylmethyl)oxy]-2-thiophenecarbonitrile |
| 1-[5-cyano-4-({[2-(trifluoromethyl)phenyl]methyl}oxy)-2-thienyl]-N-[2-(4-morpholinyl)ethyl]-1H-benzimidazole-5-carboxamide |
| 5-[5,6-bis(methyloxy)-1H-benzimidazol-1-yl]-3-({(1*R*)-*1*-*[2-*(trifluoromethyl)phenyl]ethyl}oxy)-2-thiophenecarbonitrile |
| 5-[5,6-bis(methyloxy)-1H-benzimidazol-1-yl]-3-[(2-phenylethyl)oxy]-2-thiophenecarbonitrile |
| 5-[5,6-bis(methyloxy)-1H-benzimidazol-1-yl]-3-[({2-[(trifluoromethyl)oxy]phenyllmethyl)oxy]-2-thiophenecarbonitrile |
| 5-[5,6-bis(methyloxy)-1H-benzimidazol-1-yl]-3-({[2-(methyloxy)phenyl]methyl}oxy)-2-thiophenecarbonitrile |
| 5-[5,6-bis(methyloxy)-1H-benzimidazol-1-yl]-3-{[2-(4-morpholinyl)ethyl]oxy}-2-thiophenecarbonitrile |
| 5-[5,6-bis(methyloxy)-1H-benzimidazol-1-yl]-3-{[2-(2-oxo-1-pyrrolidinyl)ethyl]oxy}-2-thiophenecarbonitrile |
| 5-[5,6-bis(methyloxy)-1H-benzimidazol-1-yl]-3-[(tetrahydro-2-furanylmethyl)oxy]-2-thiophenecarbonitrile |
| 5-[5,6-bis(methyloxy)-1H-benzimidazol-1-yl]-3-[(tetrahydro-2H-pyran-2-ylmethyl)oxy]-2-thiophenecarbonitrile |
| 5-[5,6-bis(methyloxy)-1H-benzimidazol-1-yl]-3-{[2-(phenyloxy)ethyl]oxy}-2-thiophenecarbonitrile |
| 5-[5,6-bis(methyloxy)-1H-benzimidazol-1-yl]-3-{[(1S)-1-(2-chlorophenyl)butyl]oxy}-2-thiophenecarbonitrile |
| 5-[5,6-bis(methyloxy)-1H-benzimidazol-1-yl]-3-[(3-thienylmethyl)oxy]-2-thiophenecarbonitrile |
| 5-[5,6-bis(methyloxy)-1H-benzimidazol-1-yl]-3-[(2-thienylmethyl)oxyl-2-thiophenecarbonitrile |
| 5-[5,6-bis(methyloxy)-1H-benzimidazol-1-yl]-3-{[(1R)-1-methylpropyl]oxy}-2thiophenecarbonitrile |

In certain embodiments, the IKKi inhibitor is an antibody or antibody fragment, including polyclonal and monoclonal antibodies. Various procedures known in the art may be used for the production of polyclonal antibodies directed against IKKi. For the production of antibody, various host animals can be immunized by injection with a peptide corresponding to an IKKi epitope including but not limited to rabbits, mice, rats, sheep, goats, etc. In a preferred embodiment, the peptide is conjugated to an immunogenic carrier (e.g., diphtheria toxoid, bovine serum albumin (BSA), or keyhole limpet hemocyanin (KLH)). Various adjuvants may be used to increase the immunological response, depending on the host species, including but not limited to Freund's (complete and incomplete), mineral gels (e.g., aluminum hydroxide), surface active substances (e.g., lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum).

For preparation of monoclonal antibodies directed toward IKKi, it is contemplated that any technique that provides for the production of antibody molecules by continuous cell lines in culture will find use with the present invention (See e.g., Harlow and Lane, Antibodies. A Laboratory Manual, Cold Spring Harbor Laboratory Press, Cold Spring Harbor, NY). These include but are not limited to the hybridoma technique originally developed by Kohler and Milstein (Kohler and Milstein, Nature 256: 495-497 [1975]), as well as the trioma technique, the human B-cell hybridoma technique (See e.g., Kozbor et al., Immunol. Tod., 4: 72 [1983]), and the EBV-hybridoma technique to produce human monoclonal antibodies (Cole et al., in Monoclonal Antibodies and Cancer Therapy, Alan R. Liss, Inc., pp. 77-96 [1985]).

Various commercially available anti-IKKi antibodies may be employed with the methods and compositions of the present invention. Exemplary antibodies include, but are not limited to: mouse monoclonal antibody 107A1458 from Abcam; mouse monoclonal antibody 72B587 from Abcam; mouse anti-human monoclonal antibodies 1F7, 2B6, 2F1, and 3D11 from Abnova Corporation; mouse anti-human monoclonal antibody 72B587 from ABR-Affinity BioReagents; rabbit anti-human polyclonal antibody 701-716 from Calbiochem; and mouse monoclonal antibody 107A1458 from Imgenix.

### IV. IKKi Inhibitor Screening Methods

The present invention provides screening methods to identify IKKi inhibitors useful in reducing body fat, increasing percent lean body mass, as well as treating diseases such as obesity, insulin resistance, diabetes, and related disorders. The inhibitors also find use in research and diagnostic applications. In preferred embodiments, the IKKi inhibitors identified inhibit the insulin receptor phosphorylation activity of IKKi. In certain embodiments, the IKKi inhibitors identified inhibit the insulin receptor phosphorylation activity of IKKi at the serine in SEQ ID NO:15 (VKTVNES), which is underlined in SEQ ID NO:14 (consensus human sequence) or corresponding serine in corresponding human or non-human sequences (which can be identified using, for example, sequence alignments). In some embodiments, the IKKi inhibitors identified inhibit the insulin receptor phosphorylation activity of IKKi, but do not inhibit one or more other activities (e.g., other kinase activities) of IKKi.

The present invention provides screening methods to identify agents capable of inhibiting the insulin receptor phosphorylation activity of IKKi. For example, in certain embodiments, cell-free assays are conducted in which IKKi, IR (insulin receptor), ³²P-γ-labeled nucleotides, and a candidate agent are combined under conditions in which IKKi can transfer ³²P from the nucleotide onto the insulin receptor (e.g., at the Serine at position 1062 in SEQ ID NO:14, and as shown in bold in Figure 26). The level of phosphorylation of IR can then be assessed to determine if a candidate agent decreases (or increases) the activity of IKKi relative to a control that was not contacted with the candidate agent. It is noted that certain methods for determining the kinase activity of IKKi are known in the art and are provided in Shimada et al., Internat. Immunol, 11:1357-1362, 1990 and WO2004/097009.

Cell based assays may also be employed to identify agents capable of inhibiting the insulin receptor phosphorylation activity of IKKi. For example, a test cell can be contacted with a candidate agent and then the cells can be lysed to produce a cellular lysate. The IKKi IR kinase activity in the cellular lysate can be assessed with an in vitro kinase assay to determine if the candidate agent increased or decreased the IKKi IR kinase activity within the cell. In certain embodiments, the phosphorylation of IR is determined (e.g., at the serine in the sequence VKTVNES (SEQ ID NO: 15) within the IR). In other embodiments, the phosphorylation state of proteins downstream from IR are determined, including Aps, Cbl, and TC10. In particular embodiments, the test cells is an adipocyte or adipose tissue macrophage. Antibodies may be employed to determine if a candidate agent modulates the activity (e.g., IR kinase activity) of IKKi within a cells. This can be done, for example, by obtaining an antibody that recognizes the IR (or Aps, Cbl, or TC10) when it is phosphorylated (e.g., at the serine at position 1035/1065), and obtaining another antibody that recognizes IR (or Aps, Cbl, or TC10) when it is non-phosphorylated. Such antibodies can be generated, for example, by immunizing with peptides comprising SEQ ID NO:15, where the serine in this sequence is phosphorylated and non-phosphorylated. According to this method, cells are contacted with a candidate agent. A lysate is prepared from the contacted cells. The lysate is then assayed with antibodies that recognize IR (or Aps, Cbl, or TC10) in both the phosphorylated and non-phosphorylated forms. The amount of antibody binding to the phosphorylated and non-phosphorylated forms of IR is then compared to the amount of antibody binding to the phosphorylated and non-phosphorylated form of IR in a lysate prepared from a control cells that was not contacted with the candidate agent. A decrease in the ratio of phosphorylated to non-phosphorylated IR in a treated cell relative to a control cell will indicate that the candidate agent inhibits IKKi IR kinase activity. In some embodiments, the level of phosphorylated IR (or Aps, Cbl, or TC10) is detected using a flow cytometric assay, e.g a bead-based assay such as a Luminex® xMAP® assay. In some embodiments, phospho-specific antibodies are used in the bead-based assay for quantification of phosphorylated IR (or Aps, Cbl, or TC10) relative to un-phosphorylated IR (or Aps, Cbl, or TC10). Methods for quantitative bead-based flow cytometric assays are known in the art and are taught in, for example, U.S. Pat. No. 5,981,180, and U.S. Pat. No. 7,049,151.

The ability of a candidate agent to modulate the kinase activity of IKKi can also be assessed through use of an in vitro kinase assay. For example, a cell lysate can be prepared. A portion of the cell lysate can be contacted with a candidate agent to produce a contacted lysate. The IR kinase activity of IKKi in the contacted lysate can then be compared to the IR kinase activity of IKKi in the lysate that was not contacted with the candidate agent to determine if the candidate agent modulates IKKi IR kinase activity. Conditions under which in vitro kinase assays can be conducted with IKKi are described herein and are known in the art (Shimada et al., Internat. Immunol, 11: 1357-1362 (1999).

In certain embodiments of the cell based and cell lysate based assays, the cells are treated with compounds that will activate IKKi. Such IKKi inducers include, but are not limited to, tumor necrosis factor (TNF), lipopolysaccharide (LPS), interleukin-1 (IL-1), interleukin-6 (IL-6), interferon-gamma, phorbol myristate, and similar agents. In certain embodiments, such IKKi inducers are compounds that activate TLR4, such that TLR4 kinases IKKi, thereby activating IKKi.

In particular embodiments, the present invention provides screening methods for identifying IKKi inhibitors using adipocytes or adipose tissue macrophages that comprise activated IKKi. In certain embodiments, the adipocytes or macrophages are contacted with an IKKi inducer, such as LPS, IL-1, IL-6, interferon-gamma, or phorbol myristate, or other agent in order to activate IKKi. Such activation may be by way of activating TLR4, which activates IKKi. Activated IKKi, as discussed above, phosphorylates the insulin receptor, thereby inhibiting its interaction with insulin (which inhibits glucose metabolism). Activated adipocytes and macrophages are then contacted with a candidate IKKi inhibitor (and a control is not contacted by a candidate IKKi inhibitor) and the effect of the inhibitor is measured. In certain embodiments, the uptake of glucose is monitored (e.g., 2-deoxyglucose is employed to measure uptake as shown in Figure 1). In other embodiments, the amount, or state, of phosphorylation of the insulin receptor is measured (e.g., phosphorylation of the serine in SEQ ID NO:15). In further embodiments, the amount, or state, of phosphorylation of the Aps, Cbl, or TC10 is measured. In further embodiments, the ability of GLUT4 to transport glucose is measured (e.g., labeled GLUT4 is employed as in the Example below to determine the amount of GLUT4 at the plasma membrane). In further embodiments, the size of the adipocytes or macrophages are measured, as the Examples below shows that IKKi inhibition leads to smaller adipocytes (see Figure 19).

In certain embodiments, the present invention provides animal based screening methods for identifying or confirming that a candidate agent is an IKKi inhibitor. Such screening methods may be used either alone or in combination with the above cell-free and cell-based methods. For example, a candidate compound or compound known to inhibit IKKi activity (e.g., IR kinase activity) can be administered to a non-human subject, and lean body mass, fat mass, or fat-free mass of the subject can be compared to that of a control subject (e. g., a corresponding non-human subject to which the test compound was not administered or to the baseline fat mass of the subject). Suitable non-human subjects include, for example, rodents such as rats and mice, rabbits, guinea pigs, farm animals such as pigs, turkeys, cows, sheep, goats, or chickens, or household pets such as dogs or cats. In certain embodiments, the animal is fed a high calorie diet. In other embodiments, the animal employed is an animal model of obesity, diabetes, or insulin resistance.

A candidate agent can be administered to a subject by any route, including, without limitation, oral or parenteral routes of administration such as intravenous, intramuscular, intraperitoneal, subcutaneous, intrathecal, intraarterial, nasal, or pulmonary administration. A test compound can be formulated as, for example, a solution, suspension, or emulsion with pharmaceutically acceptable carriers or excipients suitable for the particular route of administration, including sterile aqueous or non-aqueous carriers. Aqueous carriers include, without limitation, water, alcohol, saline, and buffered solutions. Examples of non-aqueous carriers include, without limitation, propylene glycol, polyethylene glycol, vegetable oils, and injectable organic esters. Preservatives, flavorings, sugars, and other additives such as antimicrobials, antioxidants, chelating agents, inert gases, and the like also may be present.

For oral administration, tablets or capsules can be prepared by conventional means with pharmaceutically acceptable excipients such as binding agents (e.g., pregelatinized maize starch, polyvinylpyrrolidone or hydroxypropyl methylcellulose), fillers (e.g., lactose, microcrystalline cellulose or calcium hydrogen phosphate), lubricants (e.g., magnesium stearate, talc or silica), disintegrants (e.g., potato starch or sodium starch glycolate), or wetting agents (e.g., sodium lauryl sulfate). Tablets can be coated using methods known in the art. Preparations for oral administration also can be formulated to give controlled release of the compound. Nasal preparations can be presented in a liquid form or as a dry product. Nebulised aqueous suspensions or solutions can include carriers or excipients to adjust pH and/or toxicity.

The effect of a candidate agent on a non-human subject can be evaluated using a variety of methods. Fat mass and/or lean mass can be assessed using, for example, DEXA hydrodensitometry weighing (i.e., underwater weighing), anthropometry (i. e., skinfold measurements using calipers, for example), near infrared interactance (NIR), magnetic resonance imaging (MR1), total body electrical conductivity (TOBEC), air displacement (BOD POD), bioelectrical impedance (BIA), or computed tomography. The effect of a test compound on physical activity of a subject also can be monitored to get a sense of "exercise" changes and an initial sense of energy expenditure, since these may change in any animal that has differences in body fat and bone strength. The effect of a test compound on other characteristics related to metabolism also can be determined. These include, for example, characteristics related to food intake (e.g., appetite, taste/smell, pain with eating, satiation, parenteral nutrition, and enteral nutrition), characteristics related to digestion in the gastrointestinal tract (e.g., analyses of villous surfaces of gut, enzymes in gut, or bile salts), characteristics related to absorption, changes in caloric requirements, characteristics related to nutrient loss (e.g., through feces, hemorrhage, urine, fistulas, or loss through barriers such as the gastrointestinal tract, skin, or lung). Energy expenditure also can be monitored. For example, multi-directional motion can be monitored using a Mini Mitter device (Bend, OR). Other characteristics related to energy expenditure that can be monitored include step/walking motion, heart rate, breathing, use of oxygen and output of carbon dioxide, photobeam monitoring, and charting of physical activity.

The candidate agents of the present invention can be obtained using any of the numerous approaches in combinatorial library methods known in the art, including biological libraries; peptoid libraries (libraries of molecules having the functionalities of peptides, but with a novel, non-peptide backbone, which are resistant to enzymatic degradation but which nevertheless remain bioactive; see, *e.g.,* Zuckennann et al., J. Med. Chem. 37: 2678-85 [1994]); spatially addressable parallel solid phase or solution phase libraries; synthetic library methods requiring deconvolution; the 'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 [1993]; Erb et al., Proc. Nad. Acad. Sci. USA 91:11422 [1994]; Zuckermann et al., J. Med. Chem. 37:2678 [1994]; Cho et al., Science 261:1303 [1993]; Carrell et al., Angew. Chem. Int. Ed. Engl. 33.2059 [1994]; Carell et al., Angew. Chem. Int. Ed. Engl. 33:2061 [1994]; and Gallop et al., J. Med. Chem. 37:1233 [1994].

Libraries of compounds may be presented in solution (*e*.*g*., Houghten, Biotechniques 13:412-421 [1992]), or on beads (Lam, Nature 354:82-84 [1991]), chips (Fodor, Nature 364:555-556 [1993]), bacteria or spores (U.S. Patent No. 5,223,409 ), plasmids (Cull et al., Proc. Nad. Acad. Sci. USA 89:18651869 [1992]) or on phage (Scott and Smith, Science 249:386-390 [1990]; Devlin Science 249:404-406 [1990]; Cwirla et al., Proc. NatI. Acad. Sci. 87:6378-6382 [1990]; Felici, J. Mol. Biol. 222:301 [1991]).

### V. Treatment Methods

IKKi inhibitors may be used to treat a variety of disease and conditions, particularly those related to improper insulin-insulin receptor signaling (e.g., those caused by increased phosphorylation of the insulin receptor by IKKi, thereby attenuating the ability of insulin to bind to this receptor for normal glucose metabolism). In particular embodiments, an IKKi inhibitor is administered to a subject in order to reduce body fat or increase lead body mass in the subject. In other embodiments, an IKKi inhibitor is administered to a subject to reduce the symptoms of (or eliminate the symptoms of) obesity, insulin resistance, diabetes, and related disorders. In some embodiments, an IKKi inhibitor is administered to lower cholesterol or lipid in a subject or to prevent elevated cholesterol or lipids in a subject. In preferred embodiments, the IKKi inhibitors are used to treat the symptoms of obesity or type 2 diabetes.

Both Types 1 and 2 diabetes mellitus are disorders of dysregulated energy metabolism, due to inadequate action and/or secretion of insulin. Although it is more common in Type 2, patients with both forms of diabetes exhibit insulin resistance, resulting from a defect in insulin-stimulated glucose transport in muscle and fat and suppression of hepatic glucose output. Obesity is a crucial determinant in the development of most cases of Type 2 diabetes, and is associated with increased circulating levels of pro-inflammatory cytokines that impair glucose tolerance, such as TNFα, IL-6, IL-18, IL-1ß, and CRP. Weight loss decreases the circulating levels of these cytokines, suggesting a direct role of adipose tissue in regulating systemic inflammation. The inflammatory signaling cascade leading to NFκB activation contributes to the development of insulin resistance in obese animal models. Haploinsufficiency of IκB Kinase-B (IKKB) protects mice from high fat diet-induced insulin resistance, but does not protect against obesity. High dose salicylates, which inhibit IKKß activity, improve glucose tolerance in obese mice.

The present invention further provides pharmaceutical compositions comprising an IKKi inhibitor, alone or in combination with at least one other agent, such as a stabilizing compound, and may be administered in any sterile, biocompatible pharmaceutical carrier, including, but not limited to, saline, buffered saline, dextrose, and water.

As is well known in the medical arts, dosages for any one patient depends upon many factors, including the patient's size, body surface area, age, the particular compound to be administered, sex, time and route of administration, general health, and interaction with other drugs being concurrently administered.

Depending on the condition being treated, these pharmaceutical compositions may be formulated and administered systemically or locally. Techniques for formulation and administration may be found in the latest edition of "Remington's Pharmaceutical Sciences" (Mack Publishing Co, Easton Pa.). Suitable routes may, for example, include oral or transmucosal administration; as well as parenteral delivery, including intramuscular, subcutaneous, intramedullary, intrathecal, intraventricular, intravenous, intraperitoneal, or intranasal administration. Administration of expression vectors to express therapeutic proteins or nucleic acids sequences (e.g., siRNA sequences, antisense sequences, etc.) may also be employed.

For injection, the pharmaceutical compositions of the invention may be formulated in aqueous solutions, preferably in physiologically compatible buffers such as Hanks' solution, Ringer's solution, or physiologically buffered saline. For tissue or cellular administration, penetrants appropriate to the particular barrier to be permeated are used in the formulation. Such penetrants are generally known in the art.

In other embodiments, the pharmaceutical compositions of the present invention can be formulated using pharmaceutically acceptable carriers well known in the art in dosages suitable for oral administration. Such carriers enable the pharmaceutical compositions to be formulated as tablets, pills, capsules, liquids, gels, syrups, slurries, suspensions and the like, for oral or nasal ingestion by a patient to be treated.

Pharmaceutical compositions suitable for use in the present invention include compositions wherein the active ingredients are contained in an effective amount to achieve the intended purpose. For example, an effective amount of an IKKi inhibitor may be that amount that restores a normal (non-diseased) rate of insulin mediated glucose metabolism. Determination of effective amounts is well within the capability of those skilled in the art, especially in light of the disclosure provided herein.

In addition to the active ingredients these pharmaceutical compositions may contain suitable pharmaceutically acceptable carriers comprising excipients and auxiliaries that facilitate processing of the active compounds into preparations that can be used pharmaceutically. The preparations formulated for oral administration may be in the form of tablets, dragees, capsules, or solutions. The pharmaceutical compositions of the present invention may be manufactured in a manner that is itself known (e.g., by means of conventional mixing, dissolving, granulating, dragee-making, levigating, emulsifying, encapsulating, entrapping or lyophilizing processes).

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are carbohydrate or protein fillers such as sugars, including lactose, sucrose, mannitol, or sorbitol; starch from corn, wheat, rice, potato, etc; cellulose such as methyl cellulose, hydroxypropylmethyl-cellulose, or sodium carboxymethylcellulose; and gums including arabic and tragacanth; and proteins such as gelatin and collagen. If desired, disintegrating or solubilizing agents may be added, such as the cross-linked polyvinyl pyrrolidone, agar, alginic acid or a salt thereof such as sodium alginate.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, (i.e., dosage).

Pharmaceutical preparations that can be used orally include push-fit capsules made of gelatin, as well as soft, sealed capsules made of gelatin and a coating such as glycerol or sorbitol. The push-fit capsules can contain the active ingredients mixed with a filler or binders such as lactose or starches, lubricants such as talc or magnesium stearate, and, optionally, stabilizers. In soft capsules, the active compounds may be dissolved or suspended in suitable liquids, such as fatty oils, liquid paraffin, or liquid polyethylene glycol with or without stabilizers.

Compositions comprising a compound of the invention formulated in a pharmaceutical acceptable carrier may be prepared, placed in an appropriate container, and labeled for treatment of an indicated condition. Conditions indicated on the label may include treatment of obesity, diabetes, insulin resistance, or weight loss.

The pharmaceutical composition may be provided as a salt and can be formed with many acids, including but not limited to hydrochloric, sulfuric, acetic, lactic, tartaric, malic, succinic, etc. Salts tend to be more soluble in aqueous or other protonic solvents that are the corresponding free base forms. In other cases, the preferred preparation may be a lyophilized powder in 1 mM-50 mM histidine, 0.1%-2% sucrose, 2%-7% mannitol at a pH range of 4.5 to 5.5 that is combined with buffer prior to use.

For any compound used in the method of the invention, the therapeutically effective dose can be estimated initially from cell culture assays. Then, preferably, dosage can be formulated in animal models (particularly murine models). A therapeutically effective dose refers to that amount of an IKKi inhibitor that ameliorates symptoms of the disease state or unwanted condition. Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index, and it can be expressed as the ratio LD₅₀/ED₅₀. Compounds that exhibit large therapeutic indices are preferred.

The data obtained from these cell culture assays and additional animal studies can be used in formulating a range of dosage for human use. The dosage of such compounds lies preferably within a range of circulating concentrations that include the ED₅₀ with little or no toxicity. The dosage varies within this range depending upon the dosage form employed, sensitivity of the patient, and the route of administration.

The exact dosage is chosen by the individual physician in view of the patient to be treated. Dosage and administration are adjusted to provide sufficient levels of the active moiety or to maintain the desired effect. Additional factors which may be taken into account include the severity of the disease state; age, weight, and gender of the patient; diet, time and frequency of administration, drug combination (s), reaction sensitivities, and tolerance/response to therapy. Long acting pharmaceutical compositions might be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Guidance as to particular dosages and methods of delivery is provided in the literature (See, U.S. Pat. Nos. 4,657,760; 5,206,344; 5,225,212; WO2004/097009, or WO2005/075465).

In some embodiments, the composition comprising an inhibitor of IKKi is co-administered with an anti-cancer agent (e.g., chemotherapeutic). The present invention is not limited by type of anti-cancer agent co-administered. Indeed, a variety of anti-cancer agents are contemplated to be useful in the present invention including, but not limited to, Acivicin; Aclarubicin; Acodazole Hydrochloride; Acronine; Adozelesin; Adriamycin; Aldesleukin; Alitretinoin; Allopurinol Sodium; Altretamine; Ambomycin; Ametantrone Acetate; Aminoglutethimide; Amsacrine; Anastrozole; Annonaceous Acetogenins; Anthramycin; Asimicin; Asparaginase; Asperlin; Azacitidine; Azetepa; Azotomycin; Batimastat; Benzodepa; Bexarotene; Bicalutamide; Bisantrene Hydrochloride; Bisnafide Dimesylate; Bizelesin; Bleomycin Sulfate; Brequinar Sodium; Bropirimine; Bullatacin; Busulfan; Cabergoline; Cactinomycin; Calusterone; Caracemide; Carbetimer; Carboplatin; Carmustine; Carubicin Hydrochloride; Carzelesin; Cedefingol; Celecoxib; Chlorambucil; Cirolemycin; Cisplatin; Cladribine; Crisnatol Mesylate; Cyclophosphamide; Cytarabine; Dacarbazine; DACA (N-[2-(Dimethyl-amino)ethyl]acridine-4-carboxamide); Dactinomycin; Daunorubicin Hydrochloride; Daunomycin; Decitabine; Denileukin Diftitox; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; 5-FdUMP; Flurocitabine; Fosquidone; Fostriecin Sodium; FK-317; FK-973; FR-66979; FR-900482; Gemcitabine; Geimcitabine Hydrochloride; Gemtuzumab Ozogamicin; Gold Au 198; Goserelin Acetate; Guanacone; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta-1a; Interferon Gamma-1b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Methoxsalen; Metoprine; Meturedepa; Mitindomide; Mitocarcin; Mitocromin; Mitogillin; Mitomalcin; Mitomycin; Mytomycin C; Mitosper; Mitotane; Mitoxantrone Hydrochloride; Mycophenolic Acid; Nocodazole; Nogalamycin; Oprelvekin; Ormaplatin; Oxisuran; Paclitaxel; Pamidronate Disodium; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rituximab; Rogletimide; Rolliniastatin; Safingol; Safingol Hydrochloride; Samarium/Lexidronam; Semustine; Simtrazene; Sparfosate Sodium; Sparsomycin; Spirogermanium Hydrochloride; Spiromustine; Spiroplatin; Squamocin; Squamotacin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Thymitaq; Tiazofurin; Tirapazamine; Tomudex; TOP-53; Topotecan Hydrochloride; Toremifene Citrate; Trastuzumab; Trestolone Acetate; Triciribine Phosphate; Trimetrexate; Trimetrexate Glucuronate; Triptorelin; Tubulozole Hydrochloride; Uracil Mustard; Uredepa; Valrubicin; Vapreotide; Verteporfin; Vinblastine; Vinblastine Sulfate; Vincristine; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride; 2-Chlorodeoxyadenosine; 2'-Deoxyformycin; 9-aminocamptothecin; raltitrexed; N-propargyl-5,8-dideazafolic acid; 2-chloro-2'-arabino-fluoro-2'-deoxyadenosine; 2-chloro-2'-deoxyadenosine; anisomycin; trichostatin A; hPRL-G129R; CEP-751; linomide; sulfur mustard; nitrogen mustard (mechlorethamine); cyclophosphamide; melphalan; chlorambucil; ifosfamide; busulfan; N-methyl-N-nitrosourea (MNU); N, N'-Bis(2-chloroethyl)-N-nitrosourea (BCNU); N-(2-chloroethyl)-N'-cyclohex- yl-N-nitrosourea (CCNU); N-(2-chloroethyl)-N'-(trans-4-methylcyclohexyl-N-- nitrosourea (MeCCNU); N-(2-chloroethyl)-N'-(diethyl)ethylphosphonate-N-nit- rosourea (fotemustine); streptozotocin; diacarbazine (DTIC); mitozolomide; temozolomide; thiotepa; mitomycin C; AZQ; adozelesin; Cisplatin; Carboplatin; Ormaplatin; Oxaliplatin; C1-973; DWA 2114R; JM216; JM335; Bis (platinum); tomudex; azacitidine; cytarabine; gemcitabine; 6-Mercaptopurine; 6-Thioguanine; Hypoxanthine; teniposide; 9-amino camptothecin; Topotecan; CPT-11; Doxorubicin; Daunomycin; Epirubicin; darubicin; mitoxantrone; losoxantrone; Dactinomycin (Actinomycin D); amsacrine; pyrazoloacridine; all-trans retinol; 14-hydroxy-retro-retinol; all-trans retinoic acid; N-(4-Hydroxyphenyl) retinamide; 13-cis retinoic acid; 3-Methyl TTNEB; 9-cis retinoic acid; fludarabine (2-F-ara-AMP); and 2-chlorodeoxyadenosine (2-Cda).

Other anti-cancer agents include: Antiproliferative agents (e.g., Piritrexim Isothionate), Antiprostatic hypertrophy agent (e.g., Sitogluside), Benign prostatic hypertrophy therapy agents (e.g., Tamsulosin Hydrochloride), Prostate growth inhibitor agents (e.g., Pentomone), and Radioactive agents: Fibrinogen 1 125; Fludeoxyglucose F 18; Fluorodopa F 18; Insulin I 125; Insulin I 131; Iobenguane I 123; Iodipamide Sodium I 131; Iodoantipyrine I 131; Iodocholesterol I 131; Iodohippurate Sodium I 123; Iodohippurate Sodium I 125; Iodohippurate Sodium I 131; Iodopyracet I 125; Iodopyracet I 131; Iofetamine Hydrochloride I 123; Iomethin I 125; Iomethin I 131; Iothalamate Sodium I 125; Iothalamate Sodium I 131; Iotyrosine I 131; Liothyronine I 125; Liothyronine I 131; Merisoprol Acetate Hg 197; Merisoprol Acetate Hg 203; Merisoprol Hg 197; Selenomethionine Se 75; Technetium Tc 99m Antimony Trisulfide Colloid; Technetium Tc 99m Bicisate; Technetium Tc 99m Disofenin; Technetium Tc 99m Etidronate; Technetium Tc 99m Exametazime; Technetium Tc 99m Furifosmin; Technetium Tc 99m Gluceptate; Technetium Tc 99m Lidofenin; Technetium Tc 99m Mebrofenin; Technetium Tc 99m Medronate; Technetium Tc 99m Medronate Disodium; Technetium Tc 99m Mertiatide; Technetium Tc 99m Oxidronate; Technetium Tc 99m Pentetate; Technetium Tc 99m Pentetate Calcium Trisodium; Technetium Tc 99m Sestamibi; Technetium Tc 99m Siboroxime; Technetium Tc 99m Succimer; Technetium Tc 99m Sulfur Colloid; Technetium Tc 99m Teboroxime; Technetium Tc 99m Tetrofosmin; Technetium Tc 99m Tiatide; Thyroxine I 125; Thyroxine I 131; Tolpovidone I 131; Triolein I 125; Triolein I 131.

Another category of anti-cancer agents is anti-cancer Supplementary Potentiating Agents, including: Tricyclic anti-depressant drugs (e.g., imipramine, desipramine, amitryptyline, clomipramine, trimipramine, doxepin, nortriptyline, protriptyline, amoxapine and maprotiline); non-tricyclic anti-depressant drugs (e.g., sertraline, trazodone and citalopram); Ca⁺⁺ antagonists (e.g., verapamil, nifedipine, nitrendipine and caroverine); Calmodulin inhibitors (e.g., prenylamine, trifluoroperazine and clomipramine); Amphotericin B; Triparanol analogues (e.g., tamoxifen); antiarrhythmic drugs (e.g., quinidine); antihypertensive drugs (e.g., reserpine); Thiol depleters (e.g., buthionine and sulfoximine) and Multiple Drug Resistance reducing agents such as Cremaphor EL.

Still other anticancer agents are those selected from the group consisting of: annonaceous acetogenins; asimicin; rolliniastatin; guanacone, squamocin, bullatacin; squamotacin; taxanes; paclitaxel; gemcitabine; methotrexate FR-900482; FK-973; FR-66979; FK-317; 5-FU; FUDR; FdUMP; Hydroxyurea; Docetaxel; discodermolide; epothilones; vincristine; vinblastine; vinorelbine; meta-pac; irinotecan; SN-38; 10-OH campto; topotecan; etoposide; adriamycin; flavopiridol; Cis-Pt; carbo-Pt; bleomycin; mitomycin C; mithramycin; capecitabine; cytarabine; 2-C1-2'deoxyadenosine; Fludarabine-PO₄ mitoxantrone; mitozolomide; Pentostatin; and Tomudex.

One particularly preferred class of anticancer agents are taxanes (e.g., paclitaxel and docetaxel). Another important category of anticancer agent is annonaceous acetogenin. Other cancer therapies include hormonal manipulation. In some embodiments, the anti-cancer agent is tamoxifen or the aromatase inhibitor arimidex (i.e., anastrozole).

In some embodiments, the composition comprising an inhibitor of IKKi is co-administered with an anti-viral agent. The present invention is not limited by type of anti-viral agent co-administered. Indeed, a variety of anti-viral agents are contemplated to be useful in the present invention including, but not limited to, Zanamivir, Oseltamivir, Amantadine, Rimantadine, Acyclovir, Valacyclovir, Famciclovir, Nevirapine [Viramune®]m Delavirdine [Rescriptor®], Efavirenz [Sustiva® and Stocrin®], Zidovudine [AZT, ZDV, azidothymidine, Retrovir®], Didanosine [ddI, Videx® Videx EC®], Zalcitabine [ddC, deoxycytidine, Hivid®], Stavudine [d4T, Zerit®, Zerit XR®], Lamivudine [3TC, Epivir®], Abacavir [ABC, Ziagen®], Emtricitabine [FTC, Emtriva®, Coviracil]), Amprenavir [Agenerase], Fosamprenavir [Lexiva], Indinavir [Crixivan], Iopinavir/ritonavir [Kaletra], Ritonavir [Norvir], Saquinavir [Fortovase], Nelfinavir [Viracept], Tenofovir [tenofovir disoproxil fumarate, Viread®], Adefovir [bis-POM PMPA, Preveon® and Hepsera®]), Denavir, Efavirenz, Epivir, Famvir, Fortovase, Invirase, Nevirapine, Norvir, Oseltamivir, Penciclovir, Preveon, Relenza, Rescriptor, Retrovir, Saquinavir, Sustiva, Symadine, Symmetrel, Tamiflu, Valacyclovir, Valtrex, Viracept, Viramune, Zanamivir, Ziagen, and Zovirax.

In some embodiments, the composition comprising an inhibitor of IKKi is co-administered with an antibiotic agent. Examples of antibiotics include, but are not limited to, penicillins, aminoglycosides, macrolides, monobactams, rifamycins, tetracyclines, chloramphenicol, clindamycin, lincomycin, imipenem, fusidic acid, novobiocin, fosfomycin, fusidate sodium, neomycin, polymyxin, capreomycin, colistimethate, colistin, gramicidin, minocycline, doxycycline, vanomycin, bacitracin, kanamycin, gentamycin, erythromicin and cephalosporins.

In some embodiments, the composition comprising an inhibitor of IKKi is co-administered with an antifungal agent. Examples of antifungal agents include but are not limited to, azoles (e.g., Fluconazole®, Itraconazole®, Ketoconazole®, Miconazole®, Clortrimazole®, Voriconazole ®, Posaconazole®, Rovuconazole®, etc.), polyenes (e.g., natamycin, lucensomycin, nystatin, amphotericin B, etc.), echinocandins (e.g., Cancidas®), pradimicins (e.g., beanomicins, nikkomycins, sordarins, allylamines, etc.) and derivatives and analogs thereof.

In some embodiments, the composition comprising an inhibitor of IKKi is co-administered with a cholesterol-lowering agent and/or a lipid-lowering agent. Examples of cholesterol lowering drugs include, but are not limited to HMG CoA reductase inhibitors, squalene synthetase inhibitors, fibric acid derivatives, probucols, bile acid sequestrants, nicotinic acids and neomycins. Examples of HMG CoA reductase inhibitors includes, but is not limited to, pravastatin, lovastatin, simvastatin, atorvastatin, fluvastatin and cerivastatin. A fibric acid derivative includes, but is not limited to, gemfibrolzil, fenofibrate, clofibrate, bezafibrate, ciprofibrate, and clinofibrate. Other agents include, but are not limited to, dextrothyroxine or its sodium salt, colestipol or its hydrochloride, cholestyramine, nicotinic acid, neomycin, p-aminoaslicylic acid or aspirin. Representative lipid-lowering drugs can be found in The Medical Letter on Drugs and Therapeutics, vol. 43, issue 1105, pp. 43-48, May 28, 2001.

### VI. IKKi Diagnostics

The present invention provides diagnostic assays related to IKKi for diagnosing conditions such as diabetes, obesity, insulin resistance, and related conditions. For example, measurements of IKKi protein and nucleic acid levels and activity in tissues or blood from patients reveal susceptibility to these devastating diseases.

In certain embodiments, the IKKi activity level is measured as a diagnostic. For example, a sample from a patient (e.g., biopsy, blood sample, or other biological fluid) is assayed to determine the level of insulin receptor phosphorylation (e.g., at the serine in the sequence VKTVNES, which is SEQ ID NO:15). Methods of detecting the state of phosphorylation of the insulin receptor are discussed above. In other embodiments, the phosphorylation state of Aps, Cbl, or TC10 is determined in a sample from a patient in order to assess the activity level of IKKi.

In particular embodiments, the diagnostic assay comprises detecting the activation level of IKKi by determining the level of phosphorylation of IKKi itself. As discussed above, the TRL4 protein phosphorylates IKKi, which activates IKKi, causing IKKi to phosphorylate the insulin receptor (thereby inhibiting the action of the insulin receptor with respect to insulin). As such, in certain embodiments, detecting the phosphorylation state of IKKi in a patient sample is diagnostic of conditions such as obesity, diabetes, insulin resistance, and related conditions.

### A. IKKi Protein Detection

The level of IKKi expression in a patient sample may be detected using a variety of techniques known to those of ordinary skill in the art, including but not limited to: protein sequencing; and, immunoassays.

### 1. Sequencing

Illustrative non-limiting examples of protein sequencing techniques include, but are not limited to, mass spectrometry and Edman degradation.

Mass spectrometry can, in principle, sequence any size protein but becomes computationally more difficult as size increases. A protein is digested by an endoprotease, and the resulting solution is passed through a high pressure liquid chromatography column. At the end of this column, the solution is sprayed out of a narrow nozzle charged to a high positive potential into the mass spectrometer. The charge on the droplets causes them to fragment until only single ions remain. The peptides are then fragmented and the mass-charge ratios of the fragments measured. The mass spectrum is analyzed by computer and often compared against a database of previously sequenced proteins in order to determine the sequences of the fragments. The process is then repeated with a different digestion enzyme, and the overlaps in sequences are used to construct a sequence for the protein.

In the Edman degradation reaction, the peptide to be sequenced is adsorbed onto a solid surface (e.g., a glass fiber coated with polybrene). The Edman reagent, phenylisothiocyanate (PTC), is added to the adsorbed peptide, together with a mildly basic buffer solution of 12% trimethylamine, and reacts with the amine group of the N-terminal amino acid. The terminal amino acid derivative can then be selectively detached by the addition of anhydrous acid. The derivative isomerizes to give a substituted phenylthiohydantoin, which can be washed off and identified by chromatography, and the cycle can be repeated. The efficiency of each step is about 98%, which allows about 50 amino acids to be reliably determined.

### 2. Immunoassays

Illustrative non-limiting examples of immunoassays include, but are not limited to: immunoprecipitation; Western blot; ELISA; immunohistochemistry; immunocytochemistry; flow cytometry; and, immuno-PCR. Polyclonal or monoclonal antibodies detectably labeled using various techniques known to those of ordinary skill in the art (*e.g*., colorimetric, fluorescent, chemiluminescent or radioactive) are suitable for use in the immunoassays.

Immunoprecipitation is the technique of precipitating an antigen out of solution using an antibody specific to that antigen. The process can be used to identify protein complexes present in cell extracts by targeting a protein believed to be in the complex. The complexes are brought out of solution by insoluble antibody-binding proteins isolated initially from bacteria, such as Protein A and Protein G. The antibodies can also be coupled to sepharose beads that can easily be isolated out of solution. After washing, the precipitate can be analyzed using mass spectrometry, Western blotting, or any number of other methods for identifying constituents in the complex.

A Western blot, or immunoblot, is a method to detect protein in a given sample of tissue homogenate or extract. It uses gel electrophoresis to separate denatured proteins by mass. The proteins are then transferred out of the gel and onto a membrane, typically polyvinyldiflroride or nitrocellulose, where they are probed using antibodies specific to the protein of interest. As a result, researchers can examine the amount of protein in a given sample and compare levels between several groups.

An ELISA, short for Enzyme-Linked ImmunoSorbent Assay, is a biochemical technique to detect the presence of an antibody or an antigen in a sample. It utilizes a minimum of two antibodies, one of which is specific to the antigen and the other of which is coupled to an enzyme. The second antibody will cause a chromogenic or fluorogenic substrate to produce a signal. Variations of ELISA include sandwich ELISA, competitive ELISA, and ELISPOT. Because the ELISA can be performed to evaluate either the presence of antigen or the presence of antibody in a sample, it is a useful tool both for determining serum antibody concentrations and also for detecting the presence of antigen.

Immunohistochemistry and immunocytochemistry refer to the process of localizing proteins in a tissue section or cell, respectively, via the principle of antigens in tissue or cells binding to their respective antibodies. Visualization is enabled by tagging the antibody with color producing or fluorescent tags. Typical examples of color tags include, but are not limited to, horseradish peroxidase and alkaline phosphatase. Typical examples of fluorophore tags include, but are not limited to, fluorescein isothiocyanate (FITC) or phycoerythrin (PE).

Flow cytometry is a technique for counting, examining and sorting microscopic particles suspended in a stream of fluid. It allows simultaneous multiparametric analysis of the physical and/or chemical characteristics of single cells flowing through an optical/electronic detection apparatus. A beam of light (*e.g*., a laser) of a single frequency or color is directed onto a hydrodynamically focused stream of fluid. A number of detectors are aimed at the point where the stream passes through the light beam; one in line with the light beam (Forward Scatter or FSC) and several perpendicular to it (Side Scatter (SSC) and one or more fluorescent detectors). Each suspended particle passing through the beam scatters the light in some way, and fluorescent chemicals in the particle may be excited into emitting light at a lower frequency than the light source. The combination of scattered and fluorescent light is picked up by the detectors, and by analyzing fluctuations in brightness at each detector, one for each fluorescent emission peak, it is possible to deduce various facts about the physical and chemical structure of each individual particle. FSC correlates with the cell volume and SSC correlates with the density or inner complexity of the particle (*e.g.*, shape of the nucleus, the amount and type of cytoplasmic granules or the membrane roughness).

Immuno-polymerase chain reaction (IPCR) utilizes nucleic acid amplification techniques to increase signal generation in antibody-based immunoassays. Because no protein equivalence of PCR exists, that is, proteins cannot be replicated in the same manner that nucleic acid is replicated during PCR, the only way to increase detection sensitivity is by signal amplification. The target proteins are bound to antibodies which are directly or indirectly conjugated to oligonucleotides. Unbound antibodies are washed away and the remaining bound antibodies have their oligonucleotides amplified. Protein detection occurs via detection of amplified oligonucleotides using standard nucleic acid detection methods, including real-time methods.

### B. DNA and RNA Detection

The level of IKKi mRNA can also be detected using a variety of nucleic acid techniques known to those of ordinary skill in the art, including but not limited to: nucleic acid sequencing; nucleic acid hybridization; and, nucleic acid amplification. The sequence of murine and human Ikki nucleic acid are provided in Figures 24B and 25B to provide guidance for designing primers and probes for detecting IKKi nucleic acid.

### 1. Sequencing

Illustrative non-limiting examples of nucleic acid sequencing techniques include, but are not limited to, chain terminator (Sanger) sequencing and dye terminator sequencing. Those of ordinary skill in the art will recognize that because RNA is less stable in the cell and more prone to nuclease attack experimentally RNA is usually reverse transcribed to DNA before sequencing.

Chain terminator sequencing uses sequence-specific termination of a DNA synthesis reaction using modified nucleotide substrates. Extension is initiated at a specific site on the template DNA by using a short radioactive, or other labeled, oligonucleotide primer complementary to the template at that region. The oligonucleotide primer is extended using a DNA polymerase, standard four deoxynucleotide bases, and a low concentration of one chain terminating nucleotide, most commonly a di-deoxynucleotide. This reaction is repeated in four separate tubes with each of the bases taking turns as the di-deoxynucleotide. Limited incorporation of the chain terminating nucleotide by the DNA polymerase results in a series of related DNA fragments that are terminated only at positions where that particular di-deoxynucleotide is used. For each reaction tube, the fragments are size-separated by electrophoresis in a slab polyacrylamide gel or a capillary tube filled with a viscous polymer. The sequence is determined by reading which lane produces a visualized mark from the labeled primer as you scan from the top of the gel to the bottom.

Dye terminator sequencing alternatively labels the terminators. Complete sequencing can be performed in a single reaction by labeling each of the di-deoxynucleotide chain-terminators with a separate fluorescent dye, which fluoresces at a different wavelength.

### 2. Hybridization

Illustrative non-limiting examples of nucleic acid hybridization techniques include, but are not limited to, *in situ* hybridization (ISH), microarray, and Southern or Northern blot.

*In situ* hybridization (ISH) is a type of hybridization that uses a labeled complementary DNA or RNA strand as a probe to localize a specific DNA or RNA sequence in a portion or section of tissue (*in situ*), or, if the tissue is small enough, the entire tissue (whole mount ISH). DNA ISH can be used to determine the structure of chromosomes. RNA ISH is used to measure and localize mRNAs and other transcripts within tissue sections or whole mounts. Sample cells and tissues are usually treated to fix the target transcripts in place and to increase access of the probe. The probe hybridizes to the target sequence at elevated temperature, and then the excess probe is washed away. The probe that was labeled with either radio-, fluorescent- or antigen-labeled bases is localized and quantitated in the tissue using either autoradiography, fluorescence microscopy or immunohistochemistry, respectively. ISH can also use two or more probes, labeled with radioactivity or the other non-radioactive labels, to simultaneously detect two or more transcripts.

### 2.1 FISH

In some embodiments, IKKi nucleic acid is detected using fluorescence *in situ* hybridization (FISH). The preferred FISH assays for the present invention utilize bacterial artificial chromosomes (BACs). These have been used extensively in the human genome sequencing project (see Nature 409: 953-958 (2001)) and clones containing specific BACs are available through distributors that can be located through many sources, *e.g.,* NCBI. Each BAC clone from the human genome has been given a reference name that unambiguously identifies it. These names can be used to find a corresponding GenBank sequence and to order copies of the clone from a distributor.

The present invention further provides a method of performing a FISH assay on human prostate cells, human prostate tissue or on the fluid surrounding said human prostate cells or human prostate tissue.

Specific protocols are well known in the art and can be readily adapted for the present invention. Guidance regarding methodology may be obtained from many references including: *In situ* Hybridization: Medical Applications (eds. G. R. Coulton and J. de Belleroche), Kluwer Academic Publishers, Boston (1992); *In situ* Hybridization: In Neurobiology; Advances in Methodology (eds. J. H. Eberwine, K. L. Valentino, and J. D. Barchas), Oxford University Press Inc., England (1994); Kuo, et al., Am. J. Hum. Genet. 49:112-119 (1991); Klinger, et al., Am. J. Hum. Genet. 51:55-65 (1992); and Ward, et al., Am. J. Hum. Genet. 52:854-865 (1993)). There are also kits that are commercially available and that provide protocols for performing FISH assays (available from *e.g*., Oncor, Inc., Gaithersburg, MD). Patents providing guidance on methodology include U.S. 5,225,326; 5,545,524; 6,121,489 and 6,573,043.

### 2.2 Microarrays

Different kinds of biological assays are called microarrays including, but not limited to: DNA microarrays (e.g., cDNA microarrays and oligonucleotide microarrays); protein microarrays; tissue microarrays; transfection or cell microarrays; chemical compound microarrays; and, antibody microarrays. A DNA microarray, commonly known as gene chip, DNA chip, or biochip, is a collection of microscopic DNA spots attached to a solid surface (*e.g.*, glass, plastic or silicon chip) forming an array for the purpose of expression profiling or monitoring expression levels for thousands of genes simultaneously. The affixed DNA segments are known as probes, thousands of which can be used in a single DNA microarray. Microarrays can be used to identify disease genes by comparing gene expression in disease and normal cells. Microarrays can be fabricated using a variety of technologies, including but not limiting: printing with fine-pointed pins onto glass slides; photolithography using pre-made masks; photolithography using dynamic micromirror devices; ink-jet printing; or, electrochemistry on microelectrode arrays.

Southern and Northern blotting is used to detect specific DNA or RNA sequences, respectively. DNA or RNA extracted from a sample is fragmented, electrophoretically separated on a matrix gel, and transferred to a membrane filter. The filter bound DNA or RNA is subject to hybridization with a labeled probe complementary to the sequence of interest. Hybridized probe bound to the filter is detected. A variant of the procedure is the reverse Northern blot, in which the substrate nucleic acid that is affixed to the membrane is a collection of isolated DNA fragments and the probe is RNA extracted from a tissue and labeled.

### 3. Amplification

IKKi genomic DNA and mRNA may be amplified prior to or simultaneous with detection. Illustrative non-limiting examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (*e.g*., PCR) require that RNA be reversed transcribed to DNA prior to amplification (*e.g*., RT-PCR), whereas other amplification techniques directly amplify RNA (*e.g*., TMA and NASBA).

The polymerase chain reaction (U.S. Pat. Nos. 4,683,195, 4,683,202, 4,800,159 and 4,965,188), commonly referred to as PCR, uses multiple cycles of denaturation, annealing of primer pairs to opposite strands, and primer extension to exponentially increase copy numbers of a target nucleic acid sequence. In a variation called RT-PCR, reverse transcriptase (RT) is used to make a complementary DNA (cDNA) from mRNA, and the cDNA is then amplified by PCR to produce multiple copies of DNA. For other various permutations of PCR *see, e.g.,* U.S. Pat. Nos. 4,683,195, 4,683,202 and 4,800,159; Mullis et al., Meth. Enzymol. 155: 335 (1987); and, Murakawa et al., DNA 7: 287 (1988).

Transcription mediated amplification (U.S. Pat. Nos. 5,480,784 and 5,399,491), commonly referred to as TMA, synthesizes multiple copies of a target nucleic acid sequence autocatalytically under conditions of substantially constant temperature, ionic strength, and pH in which multiple RNA copies of the target sequence autocatalytically generate additional copies. *See, e.g.,* U.S. Pat. Nos. 5,399,491 and 5,824,518. In a variation described in U.S. Publ. No. 20060046265, TMA optionally incorporates the use of blocking moieties, terminating moieties, and other modifying moieties to improve TMA process sensitivity and accuracy.

The ligase chain reaction (Weiss, R., Science 254: 1292 (1991)), commonly referred to as LCR, uses two sets of complementary DNA oligonucleotides that hybridize to adjacent regions of the target nucleic acid. The DNA oligonucleotides are covalently linked by a DNA ligase in repeated cycles of thermal denaturation, hybridization and ligation to produce a detectable double-stranded ligated oligonucleotide product.

Strand displacement amplification (Walker, G. et al., Proc. Natl. Acad. Sci. USA 89: 392-396 (1992); U.S. Pat. Nos. 5,270,184 and 5,455,166), commonly referred to as SDA, uses cycles of annealing pairs of primer sequences to opposite strands of a target sequence, primer extension in the presence of a dNTPαS to produce a duplex hemiphosphorothioated primer extension product, endonuclease-mediated nicking of a hemimodified restriction endonuclease recognition site, and polymerase-mediated primer extension from the 3' end of the nick to displace an existing strand and produce a strand for the next round of primer annealing, nicking and strand displacement, resulting in geometric amplification of product. Thermophilic SDA (tSDA) uses thermophilic endonucleases and polymerases at higher temperatures in essentially the same method (EP Pat. No. 0 684 315).

Other amplification methods include, for example: nucleic acid sequence based amplification (U.S. Pat. No. 5,130,238), commonly referred to as NASBA; one that uses an RNA replicase to amplify the probe molecule itself (Lizardi et al., BioTechnol. 6: 1197 (1988)), commonly referred to as Qβ replicase; a transcription based amplification method (Kwoh et al., Proc. Natl. Acad. Sci. USA 86:1173 (1989)); and, self-sustained sequence replication (Guatelli et al., Proc. Natl. Acad. Sci. USA 87: 1874 (1990)). For further discussion of known amplification methods *see* Persing, David H., "In Vitro Nucleic Acid Amplification Techniques" in Diagnostic Medical Microbiology: Principles and Applications (Persing et al., Eds.), pp. 51-87 (American Society for Microbiology, Washington, DC (1993)).

### 4. Detection Methods

Non-amplified or amplified nucleic acids can be detected by any conventional means. For example, IKKi nucleic acid can be detected by hybridization with a detectably labeled probe and measurement of the resulting hybrids. Illustrative non-limiting examples of detection methods are described below.

One illustrative detection method, the Hybridization Protection Assay (HPA) involves hybridizing a chemiluminescent oligonucleotide probe (*e.g.*, an acridinium ester-labeled (AE) probe) to the target sequence, selectively hydrolyzing the chemiluminescent label present on unhybridized probe, and measuring the chemiluminescence produced from the remaining probe in a luminometer. *See, e.g.,* U.S. Pat. No. 5,283,174 and Norman C. Nelson et al., Nonisotopic Probing, Blotting, and Sequencing, ch. 17 (Larry J. Kricka ed., 2d ed. 1995).

Another illustrative detection method provides for quantitative evaluation of the amplification process in real-time. Evaluation of an amplification process in "real-time" involves determining the amount of amplicon in the reaction mixture either continuously or periodically during the amplification reaction, and using the determined values to calculate the amount of target sequence initially present in the sample. A variety of methods for determining the amount of initial target sequence present in a sample based on real-time amplification are well known in the art. These include methods disclosed in U.S. Pat. Nos. 6,303,305 and 6,541,205. Another method for determining the quantity of target sequence initially present in a sample, but which is not based on a real-time amplification, is disclosed in U.S. Pat. No. 5,710,029.

Amplification products may be detected in real-time through the use of various self-hybridizing probes, most of which have a stem-loop structure. Such self-hybridizing probes are labeled so that they emit differently detectable signals, depending on whether the probes are in a self-hybridized state or an altered state through hybridization to a target sequence. By way of non-limiting example, "molecular torches" are a type of self-hybridizing probe that includes distinct regions of self-complementarity (referred to as "the target binding domain" and "the target closing domain") which are connected by a joining region (*e.g.*, non-nucleotide linker) and which hybridize to each other under predetermined hybridization assay conditions. In a preferred embodiment, molecular torches contain single-stranded base regions in the target binding domain that are from 1 to about 20 bases in length and are accessible for hybridization to a target sequence present in an amplification reaction under strand displacement conditions. Under strand displacement conditions, hybridization of the two complementary regions, which may be fully or partially complementary, of the molecular torch is favored, except in the presence of the target sequence, which will bind to the single-stranded region present in the target binding domain and displace all or a portion of the target closing domain. The target binding domain and the target closing domain of a molecular torch include a detectable label or a pair of interacting labels (*e.g*., luminescent/quencher) positioned so that a different signal is produced when the molecular torch is self-hybridized than when the molecular torch is hybridized to the target sequence, thereby permitting detection of probe:target duplexes in a test sample in the presence of unhybridized molecular torches. Molecular torches and a variety of types of interacting label pairs are disclosed in U.S. Pat. No. 6,534,274.

Another example of a detection probe having self-complementarity is a "molecular beacon." Molecular beacons include nucleic acid molecules having a target complementary sequence, an affinity pair (or nucleic acid arms) holding the probe in a closed conformation in the absence of a target sequence present in an amplification reaction, and a label pair that interacts when the probe is in a closed conformation. Hybridization of the target sequence and the target complementary sequence separates the members of the affinity pair, thereby shifting the probe to an open conformation. The shift to the open conformation is detectable due to reduced interaction of the label pair, which may be, for example, a fluorophore and a quencher (e.g., DABCYL and EDANS). Molecular beacons are disclosed in U.S. Pat. Nos. 5,925,517 and 6,150,097.

Other self-hybridizing probes are well known to those of ordinary skill in the art. By way of non-limiting example, probe binding pairs having interacting labels, such as those disclosed in U.S. Pat. No. 5,928,862 might be adapted for use in the present invention. Additional detection systems include "molecular switches," as disclosed in U.S. Publ. No. 20050042638. Other probes, such as those comprising intercalating dyes and/or fluorochromes, are also useful for detection of amplification products in the present invention. *See, e.g.,* U.S. Pat. No. 5,814,447.

### EXAMPLES

The following exampled are provided in order to demonstrate and further illustrate certain preferred embodiments and aspects of the present invention and are not to be construed as limiting the scope thereof.

### Example 1

### Activation of IKKi Mediates Phosphorylation of the Insulin Receptor

This Example describes the identification of IKKi as an enzyme responsible for phosphorylation of the insulin receptor - thereby inhibiting the insulin receptor. The activation of IKKi, therefore, was found to attenuate the activity of insulin, which is associated with obesity, diabetes, insulin resistance, and related disorders.

### Methods

*Materials and reagents.* All chemicals were obtained from Sigma-Aldrich unless stated otherwise. LPS from *S. Minnesota* R595(Re) (Calbiochem), *E.coli* 55:B5 and 0111:B4 (Sigma) was dissolved in phosphate buffered saline and subjected to gentle sonication until opaque to generate a homogeneous micellular emulsion. Dissolved 10ug/uL LPS was stored maximally one week at 4°C prior to use as a 1000-fold stock solution. 2-deoxy-D-[¹⁴C]glucose and [³²P]orthophosphate_{(aq)} were obtained from GE Health. Stealth™ duplex siRNA was obtained from Invitrogen, using 5'-UGC CAG UGA UGU GUU UCC AUC UUC U (SEQ ID NO:1) against the mouse InsR (not conserved in human), 5'-CCU CUU CUG GCA AUG GAG UAC UGU U (SEQ ID NO:2) against mouse IKKα, 5'-UGG CAC CCA AUG AUU UGC CAC UGC U (SEQ ID NO:3) against mouse IKKβ, 5'CAG CUC UGA CUU AGA GUC CUC ACU A (SEQ ID NO:4) against mouse IKKi. Primers were designed using the Block-IT™ program from Invitrogen, as a control the scrambled primers suggested by the program were used.

*Antibodies.* Antibodies against InsRβ, IRS-1, p110, APS, GST and Cbl were from Santa Cruz. Phospho-specific antibodies against T308 and S473 of Akt, IKKα/β and IκBα as well as antibodies against Akt and IKKα were obtained from Cell Signalling. 4G10 and IKKβ were from Upstate, caveolin-1 was from Transduction laboratories, IKKi was from ImGenex and Flag antibody was from Sigma.

*Plasmids and mutagenesis.* All IKKi expressing plasmids were kindly provided by Dr. Hiscott and Dr. Maniatis [see, Fitzgerald et al., Nature Immunol, 2003, 4 (5): 491-496, and Sharma et al., Science 2003, May 16;300(5622): 1148-51. Epub 2003 Apr 17 ]. A retroviral construct expressing myc-GLUT4-enhanced green fluorescent protein, containing a myc-tag in the first exofacial loop of GLUT4 (myc-GLUT4-eGFP) was a kind gift of Dr. Lodish [Bogan, et al. Mol Cell Biol. 2001 Jul;21(14):4785-806]. A human InsR expressing plasmid was kindly provided by Dr. Pessin, SUNY Stony Brook, NY, USA. Mutated InsR was generated using Stratagene Quick Change™ mutagenesis kit according to the manufacturer's protocol using 5'-CTC TCG GAG ACT GGC TGC CTC GTT GAC CGT (SEQ ID NO:5) and 5'-ACG GTC AAC GAG GCA GCC AGT CTC CGA GAG (SEQ ID NO:6) as mutagenic primers.

*Cell culture and transfection.* 3T3-L1 fibroblasts (American Type Culture Collection) were cultured and differentiated as described previously [Reed & Lane, Proc Natl Acad Sci USA. 1980 Jan;77(1):285-9]. Cells were routinely used within 7 days upon completion of the differentiation process, with only cultures in which >95% of cells displaying adipocyte morphology being used. CHO-IR and COS-1 cells were maintained in Dulbecco modified Eagle medium (Gibco) containing 10% foetal bovine serum. CHO-IR and 3T3-L1 cells were routinely transfected with plasmids and siRNA by electroporation as described previously (Inoue, M., et al., (2006) Mol Biol. Cell 17, 2303, Baumann, et al., (2000) Nature 407, 202). Cos-1 cells were transfected using FuGene6 (Roche Diagnostics) in accordance with the manufacturer's protocol.

*Animals and animal care*. Male C57BL/6 mice were rendered insulin resistant by feeding an high fat diet consisting of 45% of calories from fat(D12451 Research Diets Inc.) starting at 8 weeks of age for 20 weeks. Control mice were fed a standard diet consisting of 4.5% fat(5002 LabDiet). Animals were housed in a specific pathogen-free facility with a 12-hour light/12-hour dark cycle and given free access to food and water. All animal use was in compliance with the Institute of Laboratory Animal Research Guide for the Care and Use of Laboratory Animals and approved by the University Committee on Use and Care of Animals at the University of Michigan.

*SVF and primary adipocyte isolation.* Epididymal fat pads from male C57BL/6 mice fed a normal or a high fat diet were excised and minced in PBS with calcium chloride and 0.5% BSA. Tissue suspensions were centrifuged at 500g for 5 minutes to remove erythrocytes and free leukocytes. Collagenase (Sigma-Aldrich) was added to 1 mg/ml and incubated at 37°C for 20 minutes with shaking. The cell suspension was filtered through a 100µm filter and then spun at 300*g* for 5 minutes to separate floating adipocytes from SVF pellet. To ensure proper isolation adipocyte fractions were examined by microscopy.

*Glucose uptake. analysis.* 3T3-L1 adipocytes grown in 12-well plates were subjected to a glucose uptake assay as described previously [Van den Berghe et al., Mol Cell Biol. 1994 Apr;14(4):2372-7] using 0.075 uCi/well 2-deoxy-D-[¹⁴C]glucose (GE Health). Samples were counted in an LS6500 Multi-Purpose Scintillation Counter (Beckman Coulter) using BCS Biodegradable Counting Scintillant (GE Health).

*Glut4 translocation.* 3T3-L1 fibroblasts infected with retroviral myc-GLUT4-eGFP were selected by GFP-intensity using flow-cytometry and differentiated into adipocytes as described above. The assay was performed as described [Bogan et al., Mol Cell Biol. 2001 Jul;21(14):4785-806 ] with small modifications. Briefly, cells were seeded on 96-well plates, and treated as indicated. Then cells were fixed for 10 minutes at room-temperature in 10% buffered neutral formalin (VWR International). One well was permeabilised with 0.5% Triton-X100 to measure total myc-signal. After the cells were washed and fixation was quenched using 50 mM glycine in phosphate-buffered saline (pH 7.4) cells were incubated overnight in blocking buffer containing 1% goat serum and 1% bovine serum albumin on phosphate-buffered saline (pH 7.4). Cells were incubated for 1 hour with anti-myc monoclonal antibody in blocking buffer. Cells were then incubated for half an hour with Alexa594 goat-anti-mouse antibody in blocking buffer. After extensive washing with phosphate-buffered saline, fluorescence was measured with a Fluorostar Optima plate reader (BMG Labtech Inc., Durham, NC) using the appropriate filter sets. The percentage of GLUT4 at the plasma membrane was calculated for each condition. GFP fluorescence was used to correct for variation in cell-density in each well. Images of the cells were captured using an FV300 scanning laser confocal microscope (Olympus America Inc., Center Valley, PA)

*Immunoprecipitation and Immunoblotting.* For radio-immunoprecipitation cells were incubated with phosphate-free Dulbecco modified Eagle medium (Gibco) for 16 hours followed by a 3 hour incubation with 1mCi/plate [³²P]orthophosphate. After stimulation, cells were washed twice with ice-cold phosphate-buffered saline and were lysed for 30 minutes at 4°C with buffer containing 1mM Na₃VO₄, 1mM EGTA, 1mM EDTA, 50mM Tris-HCl pH7.4, 1% NonidetP-40, 0.5% sodium deoxycholate, 150mM NaCl, 5mM NaF in the presence of protease inhibitors (Roche Diagnostics). Cell lysate was cleared from cellular debris by spinning at 14,000 x g for 10 minutes at 4°C in a table-top centrifuge after which supernatans was pushed through a Millex-HA 0.45 um filter (Millipore). Per immunoprecipitate 1 mg of lysate was subjected to immunoprecipitation using 5 ug antibody for 1.5 hours at 4°C. Immunocomplexes were harvested by incubation with ProtA/ProtG beads (Roche Diagnostics) for 1.5 hours at 4°C. Samples were washed extensively with lysis buffer before solubilisation in sodium dodecyl sulfate (SDS) sample buffer. Bound proteins were resolved were resolved by SDS-polyacrylamide gel electrophoresis (Invitrogen) and transferred to nitrocellulose membranes (BioRad). Individual proteins were detected with the specific antibodies and visualised on film using horseradish peroxidase-conjucated secondary antibodies (SantaCruz) and Western Lightning Enhanced Chemoluminescence (Perkin Elmer Life Sciences).

In vitro *pull-down assay.* GST fusion proteins containing the SH2 domain of APS were expressed in BL21 *E.coli* and purified as described previously (Liu et al., (2002) Mol. Cell. Biol. 22, 3599). CHO-IR cells were transfected with wild-type or kinase-dead IKKi. Whole-cell lysates were prepared as described above for immunoprecipitation using buffer containing 50mM Tris-HCl pH8, 135 mM NaCl, 1% Triton X-100, 1 mM EDTA, 1 mM sodium orthophosphate, 10 mM NaF and protease inhibitors (Roche Diagnostics). Cells were incubated with GST or GST-APS-SH2 for 1 hour at 4°C. Complexes were harvested with glutathione-Sepharose beads (GE Health) for 1 hour at 4°C and subjected to extensive washing before resolving the complexes by immunoblotting.

*Energy expenditure and respiratory quotient.* Oxygen consumption (VO₂), carbon dioxide production (VCO₂), spontaneous motor activity and food intake were measured using the Comprehensive Laboratory Monitoring System (CLAMS, Columbus Instruments), an integrated open-circuit calorimeter equipped with an optical beam activity monitoring system. Mice were weighed each time before the measurements and individually placed into the sealed chambers (7.9" x 4" x 5") at 4:30∼5:00pm. Animals are allowed to stay at least 12-24 hours in the measuring chamber to adapt to the new environment before the virtual measurements start. The measurements were carried out continuously for 24∼48 hours. During this time, animals were provided with food and water through the equipped feeding and drinking devices located inside the chamber. The amount of food consumed by each animal was monitored through a precision balance attached below the chamber. The system was routinely calibrated each time before the experiment using a standard gas (20.5% O₂ and 0.5% CO₂ in N2). VO₂ and VCO₂ in each chamber were sampled sequentially for 5 seconds in a 10 minutes interval and the motor activity was recorded every second. The air flow rate through the chambers was adjusted at the level to keep the oxygen differential around 0.3% at resting conditions. Respiratory quotient (RQ) was calculated as a ratio of VCO₂ to VO₂. Energy expenditure and substrate oxidation rates can also be calculated based on the values of VO₂, VCO₂, and the protein breakdown estimated from urinary nitrogen excretion.

### Results

### Lipopolysaccharide (LPS) attenuates insulin action in adipocytes.

To model the molecular changes associated with obesity in adipose tissue, the effect of activation of the Toll-like receptor 4 (TLR4) were examined with lipopolysaccharide (LPS) in 3T3L1 adipocytes. Cultured adipocytes were pre-treated with LPS at concentrations known to activate TLR4, and insulin-stimulated 2-deoxyglucose uptake was assessed (Figure 1). LPS produced a 30-40% reduction in glucose uptake stimulated by insulin, while slightly elevating the basal transport.

Insulin-stimulated glucose uptake is mediated by the facilitative glucose transporter Glut4. Insulin increases glucose uptake by stimulating the translocation of Glut4 from intracellular stores to the cell surface. In order to assess the means by which LPS attenuates insulin action, 3T3L1 adipocytes were transfected with a construct containing Glut4 fused to both enhanced green fluorescent protein and a myc epitope tag (Myc-Glut4-eGFP), thus allowing an evaluation of the insulin-dependent translocation of the protein (GFP-signal) and membrane insertion (Myc). As shown in Figure 2, LPS blocked the effect of insulin on both processes.

### Inhibition of insulin action by LPS is mediated by activation of IKKi.

LPS binding to TLR4 leads to activation of a class of protein kinases known as the IKK's, comprised of four members, α, β, 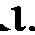 (or ε) and TBK1. To identify which IKK-isoforms are required for the attenuation of insulin action by LPS, IKK activation was analyzed in 3T3L1 adipocytes after treatment with LPS. IKK β and i underwent activation after LPS treatment (as indicated by incorporation of ³²P), whereas TBK1 and IKKα were unaffected (Figure 3a). To determine which of these is involved in the inhibitory effects of LPS, each IKK isoform was knocked down by siRNA prior to assay of glucose uptake.

Protein levels of all IKK isoforms were efficiently reduced by siRNA treatment, while insulin-stimulated Akt phosphorylation was not affected (Figure 3b), indicating that there were no non-specific effects of the knockdowns on insulin receptor activation or signalling. These cells were then assayed for the ability of LPS to block insulin-stimulated glucose uptake. Knockdown of IKKα and IKKβ did not alter the inhibitory effect of LPS on insulin-stimulated glucose uptake, whereas knockdown of IKK 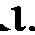 prevented the adverse effects of LPS (Figure 4), indicating that this isoform is required for the effects of LPS on insulin action in adipocytes.

To further evaluate this effect,wild-type IKKi or a kinase-inactive dominant-negative IKKi mutant was ectopically exprssed in 3T3L1 adipocytes (Figure 5). In the face of ectopic overexpression of wild-type IKKi, LPS reduced insulin-stimulated glucose uptake, while overexpression of the kinase-dead IKKi mutant fully prevented the adverse effects of LPS. As a final method to evaluate the importance of IKKi in the deleterious effects of LPS on insulin action, cells were treated with a specific IKKi inhibitor prior to treatment with LPS and insulin. 3T3L1 adipocytes were treated with 50nM of the IKKi inhibitor 5-(5,6-Dimethoxy-1*H*-benzimidazol-1-yl)-3-[[2-(methylsulfonyl)phenyl]methoxy]-2-thiophenecarbonitrile (Figure 6a), and then treated with LPS, followed by insulin (Figure 6b). The IKKi inhibitor prevented the inhibitory effects of LPS on insulin-stimulated glucose uptake, while having little direct effect on insulin-stimulated or basal transport.

### LPS inhibits insulin-stimulated APS and Cbl tyrosine phosphorylation.

Having established that acute LPS treatment interferes with insulin-stimulated Glut4 translocation in adipocytes, it was then determined where in the signalling pathway insulin action is adversely affected by LPS-induced TLR4 activation. Treatment of adipocytes with LPS had no effect on insulin-stimulated tyrosine phosphorylation of the insulin receptor (InsR) or IRS-1 (Figure 7A), as detected by anti-phosphotyrosine immunoblotting, nor was there a reduction in the amount of PI-3' kinase that co-immunoprecipitated with IRS-1 after insulin stimulation (Figure 7B). Likewise, activation of the protein kinase Akt by insulin was not affected by LPS pre-treatment of cells (Figure 8). In addition, there was no effect of LPS on insulin-stimulated activation of Erk-1/2 or PKCλ or on tyrosine phosphorylation of caveolin. However, insulin-stimulated tyrosine phosphorylation of both APS and Cbl were markedly reduced by LPS-treatment (Figure 9). Consistent with these observations, LPS also reduced insulin-induced activation of TC10, a downstream effector of APS/Cbl (Figure 10).

### IKKi catalyzes the phosphorylation of the insulin receptor to selectively block signaling pathways.

Since LPS reduces the insulin-stimulated tyrosine phosphorylation of APS, it was hypothesized that TLR4 activation influences insulin signalling at the insulin receptor-APS signalling node. To identify the targets of IKKi after LPS activation, *in vivo* orthophosphate labelling of LPS-treated adipocytes was performed followed by immunoprecipitation of either the receptor or APS. LPS stimulated the incorporation of radioactive phosphate into the InsR, but not APS, suggesting that IKKi catalyzes the direct phosphorylation of the InsR (Figure 11a). The LPS-stimulated incorporation of radioactive phosphate into the InsR was completely blocked by siRNA-dependent reduction of IKKi, but not any of the other IKK isoforms (Figure 11b).

The primary sequence of the InsR was compared to primary amino acid sequences of known IKKi targets, including p65 and STAT1 (Figure 12). While not necessary to understand to practice the present invention, Ser¹⁰³⁵ was identified in the sequence VKTVNES¹⁰³⁵AS (SEQ ID NO:9) of the InsR as a potential IKKi phosphorylation site. Ser¹⁰³⁵ is fully solvent-exposed in the crystal structure of the insulin receptor tyrosine kinase domain (Hubbard et al., EMBO J. 1997 Sep 15;16(18):5572-81), and resembles the known IKK 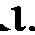 target sequences: VFTDLAS⁴⁶⁸VD (SEQ ID NO:7) in p65 (Mattioli et al., J Biol Chem. 2006 Mar 10;281(10):6175-83) and IKTELIS⁷¹¹VS (SEQ ID NO:8) in STAT1 (TenOever et al., Science. 2007 Mar 2;315(5816):1274-8). To test the hypothesis that this is the site of IKKi-catalyzed phosphorylation, a human InsR mutant was generated by replacing Ser¹⁰³⁵ with alanine, and ectopically expressed in COS-cells in conjunction with wild-type IKKi. Lysates from these cells were subjected to a pull-down assay with a GST-APS fusion protein (Figure 13). In cells expressing the wildtype InsR, IKKi overexpression reduced the interaction of the receptor with GST-APS. In contrast, the Ser¹⁰³⁵Ala InsR mutant was resistant to the negative effects of IKKi on the interaction of the receptor with GST-APS. Furthermore, the Ser¹⁰³⁵Ala substitution blocked the incorporation of radioactive phosphate relative to the wild-type receptor when co-expressed with IKKi (Figure 14A).

To establish whether Ser¹⁰³⁵ is a physiologically important site for negative regulation of insulin action, the endogenous mouse insulin receptor was knocked down in 3T3L1 adipocytes using siRNA, subsequently ectopically expressed the LPS-resistant, siRNA-resistant human Ser¹⁰³⁵Ala mutant, and assayed insulin-stimulated glucose uptake. Cells subject to the murine InsR knockdown did not respond to insulin, while reexpression of the wildtype or Ser¹⁰³⁵Ala mutant human receptor restored responsiveness. Interestingly, the inhibitory effects of LPS were eliminated in cells expressing the mutant receptor, confirming that the phosphorylation of this site was responsible for the inhibitory effects of IKKi (Figure 14B).

### IKKi expression is increased in adipose tissue after high fat feeding.

To evaluate the role of IKKi in obesity and diabetes, its expression was evaluated in mice subject to a normal or high fat diet. After 8 weeks on a high fat diet, male mice were sacrificed and epididymal fat pads were excised and centrifuged to separate adipocytes from the stromal vascular fraction that is highly enriched in adipose tissue macrophages. Cells were lysed and IKKi levels were determined by western blotting (Figure 15). High fat feeding increased IKKi levels in both adipocytes and adipose tissue macrophages.

### Genetic ablation of IKKi prevents obesity and insulin resistance.

The IKKi gene was deleted in mice by homologous recombination (Tenoever et al., Science, 2007 Mar 2;315(5816):1274-8). Mice were placed on a high fat or normal chow diet for 8 weeks. As is seen in Figure 16, IKKi knockout mice (IKKiKO) gained significantly less weight than did their wildtype littermates on this diet. Quantitation of this data revealed that this reduction in weight gain was statistically significant, with a P<0.01 (Figure 17). Most of the reduction in weight resulted from significantly smaller adipose depots, as shown in Figure 18. Further investigation revealed that this difference was due to smaller fat cells (Figure 19).

To evaluate the mechanisms underlying the reduction in weight gain of IKKiKO mice, food intake, energy expenditure and respiratory quotient were evaluated. While there was no significant difference in food intake between the KO and WT mice, IKKiKO mice exhibited increased VO₂ (Figure 20) and VCO₂ (Figure 21) during the dark and light cycles, indicative of increased energy expenditure.

To evaluate the impact of the changes in energy metabolism on glucose homeostasis, glucose and insulin tolerance tests were performed on wildtype and IKKi knockout mice after high fat feeding. After 8 weeks on a high fat diet, wildtype and IKKiKO mice were fasted overnight, and injected with a bolus of glucose. Blood was sampled every 30 minutes, and glucose and insulin levels were assayed (Figure 22). While the wildtype mice were glucose intolerant, IKKiKO mice showed much improved glucose tolerance, with an approximate 25% reduction in the area under the curve. Insulin tolerance was also evaluated in the same mice after a 3 hour fast. While wildtype mice fed a high fat diet were resistant to the actions of insulin, IKKiKO mice remained insulin sensitive (Figure 23).

### IKKi enzymatic activity is markedly increased in mice fed a high fat diet

To assess the effect of high fat diet on the enzymatic activity of IKKi in adipose tissue, fat tissue was excised from mice in either normal diet or high fat fed mice, lysed, and immunoprecipitated with antibodies to IKKi. Following immunoprecipitation, the activity of IKKi was assayed by incubation with myelin basic protein and ³²P-ATP. Enzyme activity was determined by SDS polyacrylamide gel electrophoresis, followed by autoradiography, The results, shown in Figure 27, show that IKKi activity was markedly increased in mice fed a high fat diet.

### Example 2

### IKKi Regulates Energy Expenditure, Insulin Sensitivity and Chronic Inflammation in Obese Mice

This Example shows that high fat diet can increase NFκB activation in mice, which leads to a sustained elevation in level of the Inducible IκB kinase (IKKi) in liver, adipocytes and adipose tissue macrophages. In experiments conducted during the course of the development of embodiments of the present invention it is shown that IKKi knockout mice are protected from high fat diet-induced obesity, chronic inflammation in liver and fat, hepatic steatosis and whole-body insulin resistance. These mice show increased energy expenditure and thermogenesis on high fat diet compared to wild type mice. They maintain insulin sensitivity in liver and fat, without activation of the proinflammatory JNK pathway associated with obesity. Gene expression analyses indicate that targeted deletion of IKKi increases expression of the uncoupling protein UCP1, reduces expression of inflammatory cytokines, and changes expression of certain regulatory proteins and enzymes involved in glucose and lipid metabolism. Thus, IKKi is an unexpected new therapeutic target for obesity, insulin resistance, diabetes and other complications associated with these disorders.

### Methods

### Reagents

All chemicals were obtained from Sigma-Aldrich unless stated otherwise. Anti-IKKi, anti-TBK1, anti-phospho-IKKβ, anti-ERK, anti-Akt, anti-phospho-Akt (ser473), anti-phospho JNK, anti-JNK, anti-IKB, anti-phospho IKB (ser32), anti-phospho IKB (ser32/36) were purchased from Cell Signaling. Anti-IRβ, anti-NFκB (p65), and anti-Rab5B were obtained from Santa Cruz Biotechnology. Anti-MGL1 antibodies purchased from eBioscience. Anti-F4/80 antibodies were from Abcam. Isolectin-Alexa 568, anti-Akt1 anti-caveolin 3 and anti-caveolin 1 antibodies were purchased from BD Biosciences. Anti-luciferase antibody from Cortex Biochem. MBP, anti-IRS1 monoclonal antibody, CAP polyclonal and phospho-tyrosine (4G10) antibodies were purchased from Upstate Biotechnology. Anti-GLUT4 and anti-UCP-1 were purchased from Alpha Diagnostic. Anti-actin and anti-FLAG were purchased from Sigma. Total rodent OXPHOS antibody was purchased from MitoSciences. Anti-Lipin1 was kindly provided by Dr. Thurl Harris at University of Virginia. Enhanced chemiluminesence (ECL) reagents were purchased from NEN, Inc. EDTA-free protease inhibitor tablet was purchased from Roche, Inc.

### Plasmids and mutagenesis

All IKKi expressing plasmids were as previously described (Fitzgerald et al., Nat. Immunol., 4, 491-496, 2003; Sharma et al., Science, 300, 1148-1151, 2003).

### Animals and animal care

Wild type or IKKi knockout male C57BL/6 mice were fed a high fat diet consisting of 45% of calories from fat (D12451 Research Diets Inc.) starting at 8 weeks of age for 14-18 weeks. Control mice were fed a standard diet consisting of 4.5% fat (5002 Lab Diet). Unless mentioned in the Figure legends, most of the experiments were performed 6 hours after withdraw of food. Animals were housed in a specific pathogen-free facility with a 12-hour light/12-hour dark cycle and given free access to food and water. All animal use was in compliance with the Institute of Laboratory Animal Research Guide for the Care and Use of Laboratory Animals and approved by the University Committee on Use and Care of Animals at the University of Michigan.

### Energy expenditure and respiratory quotient

WT and IKKi knockout mice (N = 8 per genotype) were placed in standard metabolic cages both on ND and after 16 weeks of HFD. Body composition was measured by NMR analyzer conducted by the University of Michigan Animal Metabolic Phenotyping Core. Food consumption, spontaneous cage activity, VO₂, VCO₂ and RER were measured during 3 consecutive days (3 dark cycles and 2 light cycles). The mean values for light and dark cycles were used in the analyses.

Oxygen consumption (VO2), carbon dioxide production (VCO2), spontaneous motor activity and food intake were measured using the Comprehensive Laboratory Monitoring System (CLAMS, Columbus Instruments), an integrated open-circuit calorimeter equipped with an optical beam activity monitoring system (Lesniewski et al., Nat. Med., 13, 455-462, 2007). These studies were conducted by University of Michigan Animal Metabolic Phenotyping Core. Respiratory quotient (RQ) was calculated as a ratio of VCO₂ to VO₂. Energy expenditure and substrate oxidation rates can also be calculated based on the values of VO₂, VCO₂, and the protein breakdown estimated from urinary nitrogen excretion.

### Rectal temperature measurement

Rectal temperature recordings were determined with YSI 4600 Precision thermometer (YSI, Inc., Yellow Springs, OH) around noon.

### Whole blood and plasma measurements

Whole blood was collected into heparin tubes. Plasma insulin concentrations were measured by insulin ELISA kit (Crystal Chem Inc.). Blood glucose was measured by OneTouch Ultra Glucometer. NEFA and cholesterol were measured by colorimetric assay (Wako). Triglyceride level was measure by Triglyceride Reagent kit purchased from Sigma. Adiponectin, and leptin concentration was measured by ELISA kits purchased from Cayman Chem Inc. Plasma MCP-1, TNFa, Rantes and IL-6 were measured by ELISA kits purchased from R&D Systems.

### Glucose, pyruvate and insulin tolerance tests

For glucose or pyruvate tolerance tests, mice were injected intraperitoneally with 1.5mg glucose/g body weight or 2mg sodium pyruvate/g body weight after 12 hrs of fasting. Blood glucose was measured at basal, 15, 30, 45, 60, 90, 120 and 180 min from tail blood using the One Touch Ultra glucometer (Lifescan). For insulin tolerance tests, mice were given an intraperitoneal injection of 0.75 unit insulin/kg body weight after 3 hrs of fasting. Blood glucose concentrations were determined as described above.

### Primary adipocytes and SVF isolation

3-5 male mice per genotype were fasted for 12 hrs before dissection. Epididymal fat pads were collected and minced with scissors. Fat pads were then digested with 1mg/mg of type II collagenase in KRBH buffer (10mM Hepes, ph7.4, 15mM NaHCO₃, 120mM NaCl, 4mM KH₂PO₄, 1mM MgSO₄, 1mM CaCl₂ and 2mM sodium pyruvate) for 10 minutes with vigorous shaking at 37°C. The cell suspension was filtered through a 70µm filter and then spun at 300g for 5 minutes to separate floating adipocytes from SVF pellet. To ensure proper isolation adipocyte fractions were examined by microscopy. Isolated cells were resuspended in appropriate buffer for RNA or protein analysis.

### RNA extraction and Real-time RT-PCR analysis

Mouse tissues were isolated, rinsed in Phosphate Buffered Saline (PBS), frozen in liquid nitrogen and stored at -80 °C until extraction. Total RNA was extracted from tissues using the RNeasy Lipid Tissue Kit (Qiagen) according to the manufacturer's instructions with the inclusion of a DNase digestion step. Total RNA was extracted from primary adipocytes and SVF using the RNeasy Kit (Qiagen) with a DNase step. The Superscript First-Strand Synthesis System for RT-PCR (Invitrogen) was used with random primers for reverse transcription. Real-time PCR amplification of the cDNA was performed on samples in triplicate with Power SYBR Green PCR Master Mix (Applied Biosystems) using the Applied Biosystems 7900HT Fast Real-time PCR System. *Rplp0* was chosen as the internal control for normalization after screening several candidate genes; its expression was not significantly affected by experimental conditions. Sequences of all primers used in this study are listed in Supplementary Figure 8. Data was analyzed using the 2^{-ΔΔCT} method (Livak and Schmittgen, 2001), and statistical significance was determined using the unpaired heterocedastic Student's t-test with one averaged sample value per mouse. For qPCR with tissue samples, WT fed with ND was set as 1.

### Immunoprecipitation

Tissues were resuspended in lysis buffer (50mM Tris, pH7.5, 5mM EDTA, 250mM sucrose, 1% NP40, 2mM DTT, 1mM sodium vanadate, 100mM NaF, 10mM Na₄P₂O₇, and freshly added protease inhibitor tablet), grinded and rocked for 1 hr in cold room (Li et al., 2006). Crude lysates were then centrifuged at 14,000 x g for 15 minutes twice and the protein concentration was determined using BioRad Protein Assay Reagent. Immunoprecipitations were performed with the indicated amount of lysates. Supernatants were incubated with 5 µg of polyclonal antibody overnight and then incubated with protein A beads for another 1 h at 4°C. Samples were washed extensively with lysis buffer before solubilisation in sodium dodecyl sulfate (SDS) sample buffer. Bound proteins were resolved by SDS-polyacrylamide gel electrophoresis and transferred to nitrocellulose membranes (BioRad). Individual proteins were detected with the specific antibodies and visualised on film using horseradish peroxidase-conjugated secondary antibodies (BioRad) and Western Lightning Enhanced Chemoluminescence (Perkin Elmer Life Sciences).

### Insulin signaling analysis

Epididymal fat, liver and quadriceps muscle tissues were collected from mice in the basal state or 10 min after an IP injection of insulin (0.85 U/kg), and quickly frozen in liquid nitrogen. Frozen tissues were homogenized on ice in lysis buffer, grinded and rocked for 1 hr in cold room. 40 µg proteins were resolved by a 4-12% or 4-20% SDS-polyacrylamide gel electrophoresis and transferred to nitrocellulose membranes (BioRad). Individual proteins were detected with the specific antibodies and visualised on film using horseradish peroxidase-conjugated secondary antibodies (BioRad) and Western Lightning Enhanced Chemoluminescence (Perkin Elmer Life Sciences).

### Immunohistochemistry and confocal microscopy

IHC and confocal microscopy were performed as described (Lumeng et al., 2007a; Lumeng et al., 2008). Adipocyte cross-sectional area from caveolin stained adipose tissue images (150-200 adipocytes/mouse, 3 mice/genotype) was calculated using CellProfiler image analysis software. Crown-like structures were identified with F4/80 immunostaining for quantitation. Adipocyte number was calculated from adipocyte diameters using established formulas (Hirsch et al., Clin. Endrocrinol. Metab., 5, 299-311, 1976).

### In vivo bioluminescence

HLL mice were shaved to expose the skin and injected IP with 150mg/kg luciferin prior to imaging with a Xenogen IVIS System 100 under sedation. Serial images were taken and luminescence quantitated at the plateau of the signal (∼15-20 minutes after injection). Tissue were harvested after luciferin injection and imaged at the plateau of the luminescent signal.

### Ex vivo lipogenesis assay

3-5 male mice per genotype were fasted for 3 hours before dissection. Epididymal fat pads were collected and minced with scissors. Fat pads were then digested with 1mg/mg of type I collagenase in KRBH buffer (10mM Hepes, pH7.4, 15mM NaHCO₃, 120mM NaCl, 4mM KH₂PO₄, 1mM MgSO₄, 1mM CaCl₂ and 2mM sodium pyruvate) for 10 minutes with vigorous shaking at 37 degrees. Adipocytes were isolated by removing supernatants that contains preadipocytes, macrophage and erythrocytes with centrifugation at 200rpm for 2min. Isolated cells were resuspended in 1.5ml of KRBH buffer. 50µl of cells were mixed with 1µCi of ¹⁴C-glucose and 950µl ofKRBH in the absence or presence of various concentrations of insulin, followed by shaking at 37 degree for 1 hour. 500µl of suspension from each sample was mixed with 500µl of PBS and 5ml of non-aqueous scintillant Ecoscint O (National Diagnostics) in a scintillation vial. All experiments were performed at a glucose concentration of 4 µM glucose, a concentration at which glucose uptake is rate limiting to the assay, effectively measuring glucose uptake in the isolated adipocytes. Vials were vortexed vigorously, and allowed to settle down for separation. The separation procedure was repeated 3 times to completely separate ¹⁴C-glucose from non-aqueous phase. Non-aqueous phase was transferred to a new vial for counting. Experiments were performed in triplicate and normalized to both protein amount and cell number. Results were similar regardless of normalization; data are presented as normalized to protein amount. Comparisons between groups were made using analysis of student t-test.

### Cell culture and transfection

3T3-L1 fibroblasts (American Type Culture Collection) were cultured and differentiated as described previously (Reed and Lane, PNAS, 77, 285-289, 1980). Cells were routinely used within 7 days upon completion of the differentiation process, with only cultures in which >95% of cells displaying adipocyte morphology being used. 3T3-L1 adipocytes were transfected by electroporation as previously described (Min et al., Mol. Cell, 3, 751-760, 1999). Cos-1 cells and H2.35 hepatoma cells were transfected using Lipofectamine 2000 (Invitrogen) in accordance with manufacturer's protocol.

### Glucose uptake in 3T3-L1 adipocytes

Insulin stimulated glucose uptake was performed following as previously described (Baumann et al., Nature, 407, 202-207, 2000).

### Results

### High fat diet produces the activation of NFkB in transgenic mice

While NFκB activation has been implicated in obesity, the range of tissues involved in this activation is unknown. To evaluate how obesity regulates NFκB activation in living animals *in vivo,* the effect of diet-induced obesity on transgenic mice engineered was analyzed with a luciferase construct driven by an NFκB responsive promoter (HLL mice) (Sadikot et al., Am. J. Respir. Crit. Care. Med., 164, 873-878, 2001). After injection with a luciferin substrate, high fat diet-fed HLL mice demonstrated an approximate 2-fold increase in abdominal luminescence compared to chow-fed controls (Figure 28). To assess which tissues were responsible for this increased signal, organs were dissected and imaged individually (Figure 28). The luciferase reporter was activated approximately 5-fold in visceral adipose tissue after high fat diet; this activation persisted after correction for tissue weight (Figure 28 and Figure 52). Less pronounced transgene activation was seen in the liver, kidney and quadriceps muscle.

It has been proposed that obesity-induced inflammation is chronic and low-grade compared to other inflammatory stimuli (Hotamisligil, Nature, 444, 860-867, 2006; Shoelson et al., Gastroenterol., 132, 2169-2180, 2007; Wellen et al., J. Clin. Invest., 115, 1111-1119, 2005). To evaluate this, the degree of NFκB activation in normal chow and high fat diet-fed HLL mice was compared before and after injection with lipopolysaccharide (LPS) (Figure 52). For all tissues examined (except for muscle), LPS injection activated the transgene far above basal levels. While the present invention is not limited to any particular mechanism, and an understanding of the mechanism is not necessary to practice the present invention, this supports the notion that the signaling pathways leading to NFκB activation are sub-maximally, but chronically activated in obesity.

To determine the cell types within adipose tissue in which NFκB is activated, immunohistochemical analyses were performed on adipose tissue of HLL mice on control and high fat diets. The luciferase reporter was specifically enriched in adipose tissue macrophage (ATM) clusters and adipocytes in epididymal fat pads from high fat, but not chow-fed mice. NFκB expression (RelA/p65) was also more concentrated in F4/80⁺ ATM clusters by immunofluorescence in C57B1/6 mice fed with high fat diet (Figure 29).

### High fat diet increases IKKi expression in white adipose tissue and liver

To investigate the mechanism and consequences of NFκB activation by high fat diet, the expression of genes encoding IKK family members in liver and white adipose tissue was measured by real-time PCR. As shown in Figure 30, high fat feeding produced a small but significant increase in the expression of IKKα, β and TBK1 in liver. However, mRNA encoding IKKi increased 2.6 fold in mice fed a high-fat diet, compared to control diet-fed mice. In white adipose tissue (WAT), IKKα was unaffected, whereas high-fat diet increased the expression of IKKβ 1.7 fold. Interestingly, IKKi and TBK1 were increased by 12 and 9 fold, respectively. To determine the cell types in adipose tissue responsible for these changes, adipocytes were separated from the SVF by centrifugation. Mice fed a high fat diet exhibited a 2 to 3-fold increase in expression of IKKα, IKKβ and TBK1 mRNA in adipocytes, whereas IKKi expression was increased up to 28-fold compared to control mice (Figure 30). However, among the IKK genes, only the expression of IKKi was up regulated in SVF isolated from white adipose tissue (1.5 fold), although the number of macrophages in adipose tissue from these mice was also significantly increased after high fat feeding (Weisberg et al., J. Clin. Invest., 112, 1796-1808, 2003; Xu et al., Diabetes, 55, 3429-3438, 2003), thus resulting in a major overall increase in IKKi.

To assess the cell type specificity of IKKi protein expression in white adipose tissue from control and high fat fed mice, immunohistochemistry was performed by confocal microscopy (Lumeng et al., Diabetes, 56, 16-23, 2007), using an antibody specific for IKKi. As previously reported, high fat diet produced increased adipocyte size. Adipose tissue from control diet-fed mice exhibited the presence of M2 polarized, MGL1⁺ adipose tissue macrophages (ATMs) (Lumeng et al., J. Clin. Invest., 117, 175-184, 2007), whereas high fat diet produced increased infiltration of M1 polarized macrophages, detected in crown-like structures. While IKKi was barely detected in adipose tissue from mice fed a control diet, the protein was observed in both adipocytes and MGL⁻, F4/80⁺ ATMs in adipose tissue from mice fed a high fat diet, but not in MGL⁺ cells. These data indicate that high fat diet induction of IKKi occurred mainly in M1 polarized ATMs, and was not detected in M2 polarized cells.

IKKi protein levels were also monitored by western blotting (Figure 31). Mice were fed a control or high fat diet, and epididymal adipose tissue and liver were removed, lysed and immunoblotted with anti-IKKi antibodies. IKKi knockout mice (Tenoever et al., Science, 315, 1274-1278, 2007; herein incorporated by reference in its entirety) were also examined as a control. IKKi expression was low in liver or white adipose tissue from wild type mice on a chow diet, but was markedly increased in both tissues from mice fed a high fat diet, correlating well with RNA levels reported in Figure 30. As a control, no IKKi was seen in tissues from knockout mice. Interestingly, none of the other IKK isoforms were up regulated in IKKi knockout mice, suggesting that no compensation occurred (Table 2).

**TABLE 2**

| genes | Tissues | ND-WT | ND-KO | HFD-WT | HFD-KO |
|---|---|---|---|---|---|
| **IK K^{α}** | WAT | 1±0.038 | 0.893±0.697 | 0.756±0.018 | 0.697±0.031 |
| | Liver | 1+0.055 | 1.065+0.051 | 1.188±0.358 | 1.351+0.077 |
| **IK K^{β}** | WAT | 1±.0042 | 0.965±0.051 | 1.699±0.059 | 1.673±0.062 |
| | Liver | 1±0.043 | 1.265±0.044 | 1.410±0.068 | 1.563±0.053 |
| **TBK1** | WAT | 1±0.097 | 1.303±0.381 | 9.122±0.692 | 10.55±0.650 |
| | Liver | 1±0.050 | 0.951±0.097 | 1.394±0.068 | 1.250±0.053 |

The induction of IKKi by high-fat diet prompted the evaluation of IKKi protein kinase activity in tissues from mice fed control and high fat diet. In experiments conducted during the course of the present invention, an *in vitro* immune complex kinase assay for IKKi was developed. To confirm that the antibody is specific for IKKi but not its related protein TBK1, FLAG-tagged IKKi and TBK1 were expressed in Cos cells individually, and detected the proteins with anti-IKKi or anti-FLAG antibody. As shown in Figure 53, anti-FLAG antibody detected similar expression of both proteins in the lysates. While the anti-TBK1 antibody specifically detected FLAG-TBK1, the anti-IKKi antibody specifically detected FLAG-IKKi, without cross-reacting with FLAG-TBK1. A comparision was conducted of the kinase activity of IKKi immunoprecipitated from lysates prepared from both adipose tissue and liver of mice fed with either chow or high fat diet. IKKi kinase activity increased by 3.7 fold and 1.5 fold in liver and WAT from mice fed a high fat versus chow diet, although there was no apparent increase in the specific activity of the enzyme (Figure 31).

### IKKi knockout mice display decreased weight gain and increased energy expenditure on a high fat diet

While the present invention is not limited to any particular mechanism, and an understanding of the mechanism is not necessary to practice the present invention, the profound increase in expression of the IKKi gene and protein after high fat diet led to the contemplation that this protein represented a link between obesity and insulin resistance. The role of IKKi in the regulation of energy balance was investigated by evaluating mice with a targeted deletion in the IKKi gene (Tenoever et al., Science, 315, 1274-1278, 2007; herein incorporated by reference in its entirety). On a normal chow diet, IKKi knockout mice did not differ significantly from their wild type counterparts (Table 3). Their weights were similar, and they had roughly similar circulating levels of glucose, insulin, and non-esterified fatty acids, although triglycerides were slightly lower in IKKi knockout mice. However, after exposure for 3 months to a high fat diet, wild type controls gained near 20 grams, whereas IKKi knockout mice gained significantly less (approximately 12 grams), implying that loss of IKKi protected mice from diet-induced obesity.

**TABLE 3 - Metabolic parameters of WT and IKKi KO mice in normal diet.**

| Parameters | Wild-type | IKKi KO | p value |
|---|---|---|---|
| Body weight (g) | 32.02±0.90 | 32.71±0.58 | 0.525 |
| Glucose (mg/dL) | 159.3±5.2 | 144.0±11.2 | 0.26 |
| Insulin (pq/ml) | 619.3±89.2 | 916.4±96.2 | 0.05 |
| NEFA (mM) | 0.969±0.077 | 0.840±0.037 | 0.175 |
| Triglyceride (mg/dL) | 50.39±3.13 | 40.49±2.31 | 0.03* |
| Cholesterol (mg/dL) | 82.5±3.24 | 75.2±2.02 | 0.292 |

The body composition of the mice was next examined using an NMR analyzer (Table 4). The percentage of lean and fat mass was similar between wild type and IKKi knockout mice on a chow diet. Exposure to 3 months of high fat diet increased the percentage of fat mass and decreased that of lean mass in both control and knockout mice. Tissue weights of white adipose tissue (WAT) and gastrocnemius/quadricep muscle were also measured. As shown in Figure 54, no significant differences were observed in the tissue weight per body weight between wild type and knockout mice on normal chow or high fat diet. In contrast, while high fat diet produced a large increase in liver weight in control mice, this diet-induced increase was not observed in knockout mice.

**TABLE 4**

| mass per body weigth (%) | | | | |
|---|---|---|---|---|
| | Fat | | Lean | |
| | ND | HFD | ND | HFD |
| WT | 9.487±0.810 | 31.84±0.579 | 70.29±0.440 | 53.19±0.652 |
| IKKi KO | 6.621±1.351 | 30.17±1.249 | 72.50±1.189 | 55.18±1.011 |
| p value | 0.14990767 | 0.280264615 | 0.17033973 | 0.15233962 |

Adipocyte size was next compared between wild type controls and knockout mice fed a high fat diet. Cell size was visualized and quantified in white adipose sections from same the area of the fat pad to avoid size variation between locations. As shown in Figure 32, adipocytes from IKKi KO mice were significantly smaller than those from wild type mice on a high fat diet. Interestingly, while adipocytes were smaller, there was a consistent 10-15% increase in the number of cells in the epididymal fat pad from mice on a high fat diet (Figure 33).

Serum adipokine levels were measured, looking first at adiponectin (Figure 33). While no differences were detected in mice on a chow diet, the serum adiponectin levels were significantly higher in knockout mice fed a high fat diet compared to wild type controls. As previously reported (Kadowaki et al., J. Clin. Invest., 116, 1784-1792, 2006), adiponectin levels per body weight were decreased in wild type mice after high fat feeding by approximately 33%. Surprisingly, this high fat diet-induced decrease was almost completely prevented in IKKi knockout mice. High fat diet increased serum leptin levels by 8.5-fold in wild type mice (Figure 33), while leptin levels were approximately 40% lower in IKKi knockout mice exposed to normal chow or high fat diet compared to wild type mice, probably reflecting smaller adipocyte size and increased leptin sensitivity.

Body weight represents a net balance of food intake and energy expenditure. IKKi knockout mice showed higher daily food intake per body weight compared to wild type mice, either on chow or a high-fat diet (Figure 33). While the present invention is not limited to any particular mechanism, and an understanding of the mechanism is not necessary to practice the present invention, this may be related to the lower circulating leptin levels in the knockout mice. Energy expenditure was then examined via indirect calorimetry. O₂ consumption was similar in both wild type control and IKKi knockout mice on a chow diet during the 72 hrs examined (Figure 20). On a high fat diet, wild type mice showed little change in O₂ consumption, whereas IKKi knockout mice demonstrated a significant increase under these conditions. This difference was consistent throughout light and dark phases, indicating an increase in energy expenditure for IKKi knockout mice on high fat diet. The respiratory quotient (RQ=VCO₂NO₂) was also compared, as a measure of fuel-partitioning patterns. RQ fluctuated between 0.85 and 1.0 in mice on a chow diet, and fluctuated between 0.8 and 0.9 in mice on high fat diet for both genotypes. No differences in respiratory quotient were in found in WT and KO mice on either normal or high fat diet (Figures 20 and 21). Taken together, these data suggest that IKKi KO mice are protected from diet-induced obesity, likely due to increased energy expenditure.

The lack of effect of IKKi knockout on RQ suggested that there was no difference in fuel selection between carbohydrates and lipids, leading us to explore whether the increase in energy expenditure might occur secondarily to increased thermogenesis. To this end, the expression of uncoupling proteins was evaluated in white adipose tissue from wild type and IKKi knock out mice on a normal chow or high fat diet (Figure 34). In chow-fed mice, UCP1 mRNA was barely detectable in WAT in both wild type and IKKi knock out mice. High fat diet produced an approximate 2-fold increase in UCP1 mRNA in wild type mice, but generated a 10-fold increase in IKKi knock out mice. The expression of UCP2 mRNA was not changed. Levels of UCP1 mRNA and protein were also evaluated in brown fat from these animals, and no discernible difference was detected in IKKi knock out mice compared to control animals (Figure 55). In order to determine the physiological effects of increased UCP1 expression in white adipose tissue, the rectal temperature of these mice were measured (Figure 34). A significant increase in body temperature was found in IKKi knockout mice compared to control mice in both diets. IKKi knockout mice were 1.5 °C warmer than their wild type counterparts on a high fat diet, with a smaller 0.5 °C increase seen in normal chow-fed mice. However, there was no apparent increase in mitochondrial biogenesis in muscle, WAT or BAT in IKKi knock out mice, based upon western blotting of subunits of OXPHOS complexes (Figure 55).

### Genetic ablation of IKKi improves glucose and lipid homeostasis

Because IKKi knockout mice were protected from diet-induced obesity, whether this gene might play a role in glucose homeostasis was considered. Fasting glucose and insulin levels were examined. As mentioned above, fasting glucose and insulin levels were similar between wild type and IKKi knockout mice fed a normal diet. Chronic exposure to high fat diet increased fasting glucose and insulin levels in wild type mice (Figure 35). In contrast, both glucose and insulin levels were significantly reduced in IKKi knockout mice compared to wild type mice fed a high fat diet.

Obesity is commonly associated with hyperlipidemia. High fat diet produced a major increase in total cholesterol levels along with a slight increase in triglycerides in wild type mice. IKKi knockout mice exhibited reduced fasting serum free fatty acid levels on high fat diet relative to wild type mice (Figure 35), but were similar to wild types regarding fasting serum triglyceride levels. Surprisingly and unexpectedly, the loss of IKKi also resulted in markedly reduced cholesterol levels in high fat diet-fed mice.

To assess the impact of IKKi on systemic glucose homeostasis in more detail, intraperitoneal (IP) glucose and insulin tolerance tests (GTT) were performed after 3 months of chow or high fat diet (Figure 36). Both wild type and knockout mice exhibited normal glucose tolerance on chow diet. While wild type mice were glucose intolerant on a high fat diet, IKKi knockout mice maintained normal glucose tolerance. Insulin levels were also examined at 0, 30, 60 and 180 min during the GTT. Insulin levels were lower in IKKi knockout mice at all time points compared to wild type controls (Figure 36). Insulin tolerance tests also revealed differences between the mice (Figure 23). Although there were no differences detected between the genotypes on a normal diet, high fat-fed IKKi knockout mice were more sensitive to IP injection of insulin compared to wild type controls.

A pyruvate tolerance test (PTT) was also performed on these mice after 3 months of high fat diet (Figure 37). Pyruvate is a precursor for gluconeogenesis, a process suppressed by insulin. Blood glucose was measured at several time points after IP administration of pyruvate in wild type and IKKi knock out mice fed a high fat diet. Blood glucose levels were significantly lower in IKKi knockout mice on high fat diet, compared to the wild type controls. While the present invention is not limited to any particular mechanism, and an understanding of the mechanism is not necessary to practice the present invention, taken together, these results indicate that loss of IKKi protected mice from high fat diet-induced glucose and pyruvate intolerance and insulin resistance.

### IKKi knock out preserves insulin signaling in liver and adipose cells in mice fed a high fat diet

To investigate the mechanisms by which targeted disruption of the IKKi gene protects mice from the deleterious effects of high fat feeding, insulin signaling pathways in liver, fat and muscle *ex vivo* were investigated. Wild type and IKKi knock out mice fed normal chow or high fat diets were injected IP with insulin or saline. Ten minutes later, liver, skeletal muscle and adipose tissue was removed for analysis of insulin-stimulated phosphorylation events by immunoblotting with phospho-Akt antibody. In liver from both wild type and IKKi knockout mice fed a normal chow diet, insulin injection stimulated Akt phosphorylation (Figure 38). While high fat diet feeding of mice resulted in a blunted insulin response in wild type mice, as previously reported (Khamzina et al., Endocrinol., 146, 1473-1481, 2005), the IKKi knock out mice exhibited normal insulin-stimulated Akt phosphorylation. Insulin-stimulated Akt phosphorylation was similarly reduced in white adipose tissue after high fat feeding in wild type but not IKKi knockout mice (Figure 38). Despite these differences, insulin-stimulated Akt phosphorylation was similar between genotypes in muscle from mice on either control or high fat diets (Figure 38). While the present invention is not limited to any particular mechanism, and an understanding of the mechanism is not necessary to practice the present invention,these data suggest that IKKi may be a local negative regulator of insulin signaling. Indeed, the levels of IKKi found in muscle were quite low and high fat diet had no effect on the expression of IKKi in muscle.

To evaluate downstream pathways that might account for the increased insulin sensitivity in liver from IKKi knock out mice, hepatic gene expression was next examined by microarray, with changes confirmed by real-time PCR. Liver mRNA was prepared from two groups of fasting mice: wild type and IKKi knock out mice on high fat diet, and subject these to microarray analyses. Results are presented in Table 5. These studies revealed significant differences between IKKi knockout and wild type controls on a high fat diet in levels of mRNA encoding several proteins. Major changes included two genes involved in glucose homeostasis, pyruvate dehydrogenase kinase isoform 4 (PDK4) and glucokinase, with smaller changes in fructose 1,6 bisphosphatase and malate dehydrogenase. Interestingly, there was little if any change observed in pyruvate kinase, PEPCK or glucose-6-phosphatase mRNAs. The lack of effects on these genes was validated by RT-PCR, and is shown in Figure 56. Real-time PCR data confirmed that PDK4 mRNA expression was reduced 66% in IKKi knockout mice compared to controls (Figure 39). PDK4 phosphorylates and inhibits the activity of the pyruvate dehydrogenase complex (PDC), which catalyzes decarboxylation of pyruvate, thus linking glycolysis to the TCA cycle and fatty acid synthesis (Sugden et al., Am. J. Physiol. Endocrinol. Metab. 284, E855-862, 2003). Increased PDC activity increases the substrates available for oxidation but limits those for gluconeogenesis, suggesting that reducing PDK4 activity would increase glucose and fatty acid oxidation but prevent gluconeogenesis. Moreover, insulin suppresses PDK4 expression in skeletal muscle and hepatoma cells (Kwon et al., Diabetes, 53, 899-910, 2004), and PDK4 knockout mice have lower blood glucose but higher circulating free fatty acid and triglyceride levels (Jeoung et al., Biochem. J. 397, 417-425, 2006). Liver glucokinase converts glucose to glucose-6-phosphate, and is the rate-limiting enzyme controlling glycolysis. Real-time PCR data confirmed that the expression of glucokinase mRNA was 2-fold increased in IKKi knockout mice on high fat diet (Figure 39). Thus, a reduction in PDK4 and increase in glucokinase would produce increased flux of glucose through glycolysis, and may account for the improvement in pyruvate tolerance and the reduced level of free fatty acids observed in IKKi KO mice.

**TABLE 5**

| **Metabolic Genes** | | | | |
|---|---|---|---|---|
| symbol | gene name | Accession No. | fold change (KO/WT) | Potential function |
| **fatty acid binding** | | | | |
| Fabp4 | fatty acid binding protein 4, adipocyte | NM_024406 | 0.71 | FA binding |
| Lcn13 | lipocalin 13 | NM_153558 | 0.56 | cytosolic fatty acid binding protein |
| Cd36 | CD36 antigen | NM_007643 | 0.50 | fatty acid transporter |
| **Lipid metabolism** | | | | |
| Cidea | cell death-inducing DNA fragmentation factor alpha s | NM_007702 | 0.61 | lipholysis |
| Cidec | cell death-inducing DFFA-like effector c | NM_178373 | 0.69 | lipophilis/Fsp27 |
| Mel | malic enzyme, 1, NADP(+)-dependent cytosolic | NM_008615 | 0.64 | lipogenesis |
| Lipg | lipase, endothelial | NM_010720 | 1.55 | Lipg |
| **glucose metabolism** | | | | |
| Pdk4 | pyruvate dehydrogenase kinase, isoenzyme 4 | NM_013743 | 0.35 | mediate glucose oxidation |
| Fbpl | fructose bisphosphatase 1 | NM_019395 | 0.64 | gluconeogenesis |
| Gck | glucokinase | NM_010292 | 1.9 | glucolysis |
| Mdh1 | malate dehydrogenase 1, NAD (soluble) | NM_008618 | 0.69 | TCA cycle |
| **metabolism** | | | | |
| Akr1c19 | aldo-keto reductase family 1, member C19 | NM_001013785 | 0.45 | NADPH oxidoreductase |
| Cbr3 | carbonyl reductase 3 | NM_173047 | 0.49 | NADPH oxidoreductase |
| Dpys | dihydropyridimidase | NM_022722 | 0.23 | pyrimidine metabolism |
| Tpmt | thiopurine methyltransferase | NM_016785 | 0.56 | nucleotide metabolism |
| **Cholesterol metabolism** | | | | |
| Amacr | alpha-methylacyl-CoA racemase | NM_008537 | 0.47 | bile acid synthesis |
| Hmgcs1 | 3-hydroxy-3-methylglutaryl-Coenzyme A synthase I | NM_145942 | 0.75 | cholesterol synthesis |
| Apoa4 | apoliproprotein A-IV | NM 007468 | 0.68 | cholesterol transport |
| **ROS production** | | | | |
| Aox1 | aldehyde oxidase I | NM_009676 | 0.76 | ROS production |
| Cyb5d2 | cytochrome b5 domain containing 2 | NM_001024926 | 0.70 | ROS production |
| Cybb | cytochrome b-245, beta polypeptide | NM_007807 | 0.60 | ROS production |
| Cvb4a14 | cytochrome P450, family 4, subfamily a, polypeptide 1 | NM_007822 | 0.67 | ROS production |
| ***Detoxification*** | | | | |
| Gsta2 | glutathione S-transferase, alpha 2 (Yc2) | NM_008182 | 0.42 | detoxification |
| Gsta4 | glutathione S-transferase, alpha 4 | NM_010357 | 0.60 | detoxification |
| Gstm3 | glutathione S-transferase, mu 3 | NM_010359 | 0.32 | detoxification |
| | | | | |

| **Inflammatory genes** | | | | |
|---|---|---|---|---|
| symbol | gene name | Accession No. | fold change (KO/WT) | |
| Anxal | annexin A1 | NM_100730 | 0.68 | |
| Anxa2 | annexin A2 | NM_007585 | 0.39 | |
| Cc19 | chemokine (C-C motif) ligand 9 | NM_011338 | 0.64 | |
| Ccr2 | chemokine (C-C motif) receptor 2 | NM_009915 | 0.62 | |
| Cd14 | CD14 antigen | NM_009841 | 0.62 | |
| Cd68 | CD68 antigen | NM_009853 | 0.72 | |
| Chi313 | chitinase 3-like 3 | NM_009892 | 0.56 | |
| Cish | cytokine inducible SH2-containing protein | NM_009895 | 1.71 | |
| Cxcl1 | chemokine (C-X-C motif) ligand 1 | NM_008176 | 0.72 | |
| Cxcl10 | chemokine (C-X-C motif) ligand 10 | NM_021274 | 0.59 | |
| Cxcl14 | chemokine (C-X-C motif) ligand 14 | NM_019568 | 0.73 | |
| Dub2 | deubiquitinating enzyme 2 | NM_010089 | 0.54 | |
| Cish | cytokine inducible SH2-containing protein | NM_008995 | 1.72 | |
| Homer2 | homer homolog 2 (Drosophila) | NM_011983 | 1.53 | |
| Ifi205 | interferon activated gene 205 | NM_172648 | 0.67 | |
| Ifi27 | interferon, alpha-inducible protein 27 | NM_029803 | 0.63 | |
| Ifi44 | interferon-induced protein 44 | NM_133871 | 0.70 | |
| Ifit2 | interferon-induced protein with tetratricopeptide r | NM_008332 | 0.68 | |
| Ifit3 | interferon-induced protein with tetratricopeptide r | NM_010501 | 0.68 | |
| Il1rn | interleukin 1 receptor antagonist | NM_001039701 | 0.62 | |
| Il2rg | interleukin 2 receptor, gamma chain | NM_013563 | 0.68 | |
| Mpa21 | macrophage activation 2 like | NM_194336 | 0.54 | |
| Mpeg1 | macrophage expressed gene I | NM_610821 | 0.64 | |
| Socs2 | suppressor of cytokine signaling 2 | NM_007706//NP | 1.66 | |
| Tlr1 | toll-like receptor 1 | NM_030682 | 0.73 | |
| Tlr2 | toll-like receptor 2 | NM_011905 | 0.68 | |
| Tlr4 | toll-like receptor 4 | NM_021297 | 0.75 | |
| Tnfaip2 | tumor necrosis factor, alpha-induced protein 2 | NM_009396 | 0.59 | |
| Tnfrsf12a | tumor necrosis factor receptor superfamily, mem | NM_013749 | 0.75 | |

| **Transcription factors** | | | | |
|---|---|---|---|---|
| symbol | gene name | Accession No. | fold change (KO/WT) | other name |
| Onecut1 | one cut domain, family member 1 | NM_008262 | 2.97 | HNF6 |
| Thrsp | thyroid hormone responsive SPOT14 | NM_009381 | 1.69 | spot14 |
| Cebpb | CCAAT/enhancer binding protein (C/E | NM_009883 | 1.66 | |
| Tle1 | transducin-like enhancer of split 1, ho | NM_011599 | 1.59 | |
| Hes6 | hairy and enhancer of split 6 (Drosoph | NM_019479 | 1.53 | |
| Nr1 h4 | nuclear receptor subfamily 1, group H, | NM_009108 | 1.50 | FXR |

In addition to direct effects on hepatic gene expression, the increased hepatic insulin sensitivity in IKKi knock out mice might also occur in part secondarily to increased circulating levels of adiponectin. This adipokine is mainly produced by adipocytes and circulates at high concentrations in serum; its mRNA expression in adipocytes correlates inversely with insulin resistance (Kadowaki et al., J. Clin. Invest., 116, 1784-1792, 2006). As described above, circulating levels of this adipokine were elevated in IKKi knock out mice compared to wild types on a high fat diet (Figure 33). Adiponectin mRNA expression was examined in white adipose tissue by real-time PCR. As shown in Figure 39, the expression of adiponectin mRNA was reduced in white adipose tissue derived from high fat diet-fed wild type mice. This reduction was prevented in IKKi knockout mice compared to wild type controls, correlating well with circulating levels of adiponectin (Figure 33).

Because adiponectin is regulated by the activity of PPARγ (Semple et al., J. Clin. Invest., 116, 581-589, 2006), the expression of PPARγ was determined to assess whether it is induced in absence of IKKi. PPARγ mRNA levels in white adipose tissue were also reduced after high fat feeding, and elevated in IKKi knockout mice compared with wild type controls (Figure 39). The PPARγ-regulated genes CD36, CAP and GLUT4 were also up regulated in adipocytes from these mice, as were the encoded proteins (Figure 40), further indicating that PPARγ activity is increased in adipose tissue from these mice. Recent studies showed that lipin1 directly interacts with PPARγ and increases its transcriptional activity (Koh et al., J. Biol. Chem., 283, 34896-34906, 2008). Overexpression of lipinl in 3T3-L1 cells increased the mRNA levels of adipogenic genes, including C/EBPα, PPARγ, GLUT4 and aP2. Moreover, mutation of lipin1 in mice (*fld*) produces a lipodystrophic phenotype and insulin resistance, suggesting an indispensable role of lipin1 in maintaining insulin sensitivity (Koh et al., J. Biol. Chem., 283, 34896-34906, 2008; Phan et al., J. Biol. Chem., 279, 29558-29564, 2004). IKKi knockout mice expressed nearly 2-fold greater levels of lipin1 mRNA and protein compared to control mice on a high fat diet (Figure 41), providing a clue to the normal insulin responsiveness in adipose tissue of the knockout mice on high fat diet.

The insulin sensitivity of isolated adipocytes *in vitro* was measured by assaying rates of lipogenesis at low concentrations of glucose, as a surrogate for glucose transport (Lesniewski et al., Nat. Med., 13, 455-462, 2007). Adipocytes from epididymal fat pads were isolated from wild type and knockout mice after control chow or high fat diet feeding. Cells were incubated with [¹⁴C]glucose in the presence or absence of insulin, and incorporation into lipid was determined after solvent extraction. As shown in Figure 42, adipocytes derived from wild type mice on a control diet responded to insulin with a two-fold increase in lipogenesis. However, adipocytes derived from wildtype mice on a high fat diet were almost completely unresponsive to insulin. In contrast, insulin-stimulated lipogenesis assayed in adipocytes isolated from IKKi knout mice on a high fat diet remained insulin responsive, demonstrating a near two-fold stimulation after treatment with insulin.

To determine whether the resistance of IKKi knockout mice to the deleterious effects of high fat diet is cell autonomous, an effort was made to mimic the increased expression of IKKi produced by high fat diet by overexpressing the enzyme in 3T3-L1 adipocytes. Cells were transfected by electroporation with constructs expressing wildtype or a kinase-inactive mutant of IKKi, and then assayed for insulin-stimulated glucose uptake (Min et al., Mol. Cell, 3, 751-760, 1999) (Figure 42). Insulin treatment produced a 10-fold increase in glucose transport in these cells. Transfection of the kinase-dead IKKi mutant had no effect, whereas expression of wild type IKKi produced a 50% reduction in insulin-stimulated glucose uptake, along with a small increase in basal activity. While the present invention is not limited to any particular mechanism, and an understanding of the mechanism is not necessary to practice the present invention, these data, along with the changes in the expression of CAP (Ribon et al., PNAS, 95, 14751-14756, 1998) and GLUT4 (Armoni et al., J. Biol. Chem., 281, 19881-19891, 2006) *in vivo,* suggest that increased expression of IKKi in adipocytes produces a direct, cell-autonomous reduction in insulin sensitivity.

### IKKi knockout mice are protected from diet-induced hepatic steatosis

Chronic exposure of mice to high fat diet causes enlarged liver mass and accumulation of lipids, leading to fatty liver (steatosis) (Bradbury, Am. J. Physiol. Gastrointest. Liver Physiol., 290, G194-198, 2006; Postic et al., J. Clin. Invest., 118, 829-838, 2008). An analysis was conducted to determine whether IKKi knock out might protect mice from high fat diet-induced steatosis. High fat diet increased liver mass in wild type controls, as shown in Figure 43. Unexpectedly, the liver mass of IKKi KO mice was significantly less than that of wild type mice fed a high fat diet. The absence of steatosis in IKKi KO mice was apparent from examination of the liver, which was considerably darker than that of the wild type counterparts (Figure 43). Triglyceride accumulation was evaluated in livers of wild type and IKKi knockout mice on a high fat diet, and examined morphology by H-E staining. The triglyceride content of livers from IKKi knockout mice was significantly lower than those of wild type mice after feeding with a high fat diet, either in fed or fasted conditions (Figure 44). No differences in liver triglycerides were found between wild type and knockout mice fed a normal diet. Additionally, high fat diet caused abundant macrosteatosis in wild type livers, as visualized by H-E staining. In contrast, IKKi knockout liver accumulated significantly less lipid within hepatocytes (Figure 44), correlating well with reduced hepatic triglycerides. While the present invention is not limited to any particular mechanism, and an understanding of the mechanism is not necessary to practice the present invention, these data suggest that IKKi knockout mice are protected from diet-induced hepatosteatosis.

Recent studies have indicated a relationship between hepatic steatosis and insulin resistance (Postic et al., J. Clin. Invest., 118, 829-838, 2008; Sanyal, Nat. Clin. Pract. Gastroenterol. Hepatol., 2, 46-53, 2005). Excessive fat accumulation in the liver can occur as a result of increased fat delivery, increased synthesis, reduced oxidation, and/or reduced fat export in the form of VLDL (Postic et al., J. Clin. Invest., 118, 829-838, 2008). To explore in more detail the mechanisms underlying the resistance of IKKi KO mice to the development of steatosis, the expression of genes in liver involved in lipid metabolism was investigated. Interestingly, no significant differences were detected between wild type and knockout mice in the expression of lipogenic enzymes, including FAS, ACC1, and Scd1, or those involved in beta-oxidation, including Acox1, Acad1, CPT1a, and MCAD, although all of these genes showed the expected response to a high-fat diet (Figure 57). Lipin1 expression reduces VLDL-triglyceride release from liver (Chen et al.,Arterioscler. Thromb. Vasc. Biol. 28, 1738-1744, 2008), and its deficiency is associated with fatty liver and insulin resistance (Xu et al., Diabetes, 55, 3429-3438, 2006). Interestingly, both mRNA and protein levels of lipin1 were increased in IKKi knockout mice on both control and high fat diets (Figure 45).

CD36 is a plasma membrane fatty acid transporter (Ibrahimi et al., Curr. Opin. Clin. Nutr. Metab. Care, 5, 139-145, 2002). Recent studies have shown that activation of LXRαcan induce CD36 expression, thereby contributing to hepatic steatosis, whereas LXRα-induced steatosis was abolished in CD36 knockout mice (Zhou et al., Gastroenterol., 134, 556-567, 2008). CD36 expression increased in response to high fat feeding in wild type mice. Expression of this mRNA was greatly reduced in IKKi knockout mice compared to wild types on normal chow and high fat diets, either in the fed or fasted state (Figure 45). Additionally, the high fat diet-induced increase in the expression of both hepatic FABP4 and PPARγ was partially prevented in IKKi knock out mice (Figure 45). Thus, although it is not possible to determine which effects were primary or secondary to reduced lipid accumulation, while the present invention is not limited to any particular mechanism, and an understanding of the mechanism is not necessary to practice the present invention, it is contemplated that IKKi knockout mice were protected from diet-induced hepatic steatosis partially due to direct inhibition of the expression of CD36, PPARγ and FABP4, and increased expression of Lipid1.

To determine whether increased levels of IKKi in liver cells can reproduce the effects of high fat feeding on gene expression in a cell-autonomous fashion, H2-35 hepatoma cells were transfected with wild type or kinase inactive IKKi, followed by assaying of mRNA levels of selected genes by RT-PCR. Approximately 3-fold overexpression of both wildtype and kinase dead IKKi was achieved in these cells, with approximately 20% efficiency (Figure 46). Interestingly, expression of the wild type kinase produced an approximate 2-fold increase in the expression of PDK4, with a 5-fold increase in Rantes mRNA expression. In contrast, overexpression of a kinase-dead enzyme was without effect. These data suggest that the IKKi-dependent changes in hepatic gene expression are likely to be direct and cell autonomous.

### IKKi knockout mice are protected from chronic, diet-induced, but not acute inflammation

Recent studies have shown that obesity is associated with a state of chronic, low-grade inflammation, characterized by infiltration of proinflammatory M1-polarized macrophages into fat tissue (Lumeng et al., J. Clin. Invest., 117, 175-184, 2007), and elevated levels of proinflammatory cytokines such as TNFα, MIP-1α and IL-6 secreted from these adipose tissue macrophages (Hotamisligil et al., Nat. Rev. Immunol., 8, 923-964, 2008; Wellen et al., J. Clin. Invest., 115, 1111-1119, 2005). To investigate the role of IKKi in the innate immune response in chronic high fat feeding, serum cytokine levels were measured in wild type and knockout mice by ELISA. As shown in Figure 47, serum levels of TNFα, MCP-1, and Rantes were similar between wild type controls and knockout mice on chow diet. Exposure of wild type mice to a high fat diet elevated the secretion of all three proinflammatory cytokines; TNFα was elevated up to 3-fold, MCP-1 was elevated 3.7 fold and Rantes levels were up 2.2 fold. Interestingly, the circulating levels of all three cytokines were at near normal levels in the IKKi knockout mice.

Macrophage infiltration in adipose tissue was also examined with cell surface markers (Lumeng et al., Diabetes, 56, 16-23, 2007). Immunofluorescence staining was performed in adipose tissue sections from wild type and IKKi knockout mice on control and high fat diets. Adipose tissue from IKKi knockout mice fed a high fat diet exhibited significantly less ATM infiltration compared to wild type controls, as detected with F4/80 antibody (Figure). Quantification of the macrophages stained positive with the antibody showed that ATM infiltration was attenuated by 90% in IKKi knockout adipose tissue (Figure 47). Chemokine and cytokine mRNA expression in adipose tissue was also significantly decreased, correlating well with decreased ATM infiltration (Figure 48). Levels of mRNAs encoding TNFα, Rantes, and MIP1α were significantly reduced in IKKi knock out mice compared to wild type mice on a high fat diet, although levels of MCP-1α and IP-10 mRNA were unaffected.

While it is clear that obesity can induce inflammation in adipose tissue, it is possible that inflammation occurs in liver as well (Sanyal, Nat. Clin. Pract. Gastroenterol. Hepatol., 2, 46-53, 2005; Schwabe et al. Am. J. Gastrointest. Liver Physiol., 290, G583-589, 2006). RNA ws isolated from liver and real-time PCR was performed to measure the levels of mRNAs encoding cytokines and other inflammatory genes (Figure 49). Chronic high fat diet increased the mRNA levels of TNFα, MCP-1, MIP-1αRantes and IP-10 in livers of wild type controls. The diet-induced increase in mRNA expression of all these proinflammatory proteins was markedly reduced in livers of IKKi knockout mice compared with wild type controls. As another marker of inflammation, iNOS expression was examined in livers from these mice (Figure 49). iNOS expression was markedly elevated upon high fat feeding of wild type mice, and this increase was almost completely blocked in livers from IKKi knock out mice.

Because inflammation appeared to be reduced in IKKi knock out mice, signaling pathways in liver, WAT and muscle tissues thought to be associated with chronic inflammation were assayed. Numerous studies have shown that high fat diet stimulates the activity of the JNK pathway (Hirosumi et al., Nature, 420, 333-336, 2002; Todoric et al., Diabetologia, 49, 2109-2119, 2006; Tuncman et al., PNAS, 103, 10741-10746, 2006), which is thought to play a crucial role in linking obesity to insulin resistance (Nakatani et al., J. Biol., Chem., 279, 45803-45809, 2004; Singh et al., Hepatology, 49, 87-96, 2009; Solinas et al., Cell Metab., 6, 386-397, 2007). To assess the role of this pathway in the resistance of IKKi knock out mice to the deleterious effects of high fat feeding, lysates from wild type and IKKi knockout mice fed a normal chow or high fat diet were immunoblotted and analyzed with a phospho-JNK antibody, as a surrogate to assay activation of the kinase. As shown in Figure 50, feeding wild type mice a chronic high fat diet produced increased JNK phosphorylation. This increase was seen in liver, gastrocnemius muscle, and white adipose tissue. Interestingly, IKKi knock out mice exhibited reduced levels of JNK phosphorylation in all three tissues comparable to control-fed wild type mice. Similar results were observed regarding JNK phosphorylation in isolated adipocytes and cells derived from the SVF, suggesting that loss of IKKi affects the inflammatory response in both cell types (Figure 58). However, overexpression of IKKi in various cell lines did not lead to increased phosphorylation of JNK,suggesting that the reduction in this pathway in knock out mice was likely to be secondary to reduced fat accumulation, leading to the conclusion that the JNK pathway is not a direct target of IKKi. Moreover, while the present invention is not limited to any particular mechanism, and an understanding of the mechanism is not necessary to practice the present invention, the level of IκB in these tissues was not changed by the loss of IKKi, indicating that IKKi may not play an important role in maintaining the stability of I B.

Taken together, while the present invention is not limited to any particular mechanism, and an understanding of the mechanism is not necessary to practice the present invention, these data suggest that targeted deletion of the IKKi gene prevents the generation of low-grade inflammation in response to high fat feeding. Because IKKi is known to catalyze the phosphorylation of IRF3 and 7 that are involved in directly regulating expression of certain inflammatory genes, the hypothesis was considered that IKKi knockout mice might be unresponsive to acute inflammatory signals as well. Endotoxin, due to its bacterial cell wall component, lipopolysaccharide (LPS), stimulates a strong host immune response via TLR4 activation, inducing proinflammatory cytokines such as TNFα, MCP-1, Rantes and IL-6. To test the role of IKKi in acute inflammatory responses, wild type and IKKi knockout mice were injected with LPS. LPS injection stimulated both IKKβ and IκB phosphorylation in liver and WAT of both wild type and knockout mice, and also led to a profound elevation in circulating levels of MCP-1 and Rantes within 2.5 hours (Figure 51). However, the levels of these cytokines were not changed in IKKi knockout mice, compared with wild type controls. These data are consistent with previous findings (Tenoever et al., Science, 315, 1274-1278, 2007; herein incorporated by reference in its entirety). While the present invention is not limited to any particular mechanism, and an understanding of the mechanism is not necessary to practice the present invention, this suggests that IKKi is not involved in the acute immune response, but may play a role in sustaining a state of chronic, low-grade inflammation in obesity.

## Claims

1. An IKKi-inhibiting agent in a therapeutically effective dose for use in the treatment of a subject experiencing or at risk of overweight or obese body composition, wherein said use results in reduction of or prevention of increase in body fat in said subject.

2. The IKKi-inhibiting agent of claim 1 for use according to claim 1 wherein said use results in an outcome selected from the group consisting of increased glucose metabolism, reduction in body fat, lack of increase in body fat, increased insulin receptor signaling, decreased level of insulin receptor phosphorylation, reduction in or prevention of chronic inflammation in liver, reduction in or prevention of chronic inflammation in adipose tissue, reduction in or prevention of hepatic steatosis, promotion of metabolic energy expenditure, reduction in circulating free fatty acids, and reduction in cholesterol.

3. The IKKi-inhibiting agent of claim 1 for use according to claim 1 wherein IKKi-mediated phosphorylation of IKB in said subject is unaffected by said IKKi-inhibiting agent.

4. The IKKi-inhibiting agent of claim 1 for use according to claim 1 selected from the group consisting of a benzimidazol-substituted thiopene, an siRNA, an antisense oligonucleotide, a non-phospho-specific anti-IKKi antibody, and a phospho-specific anti-IKKi antibody.

5. A diagnostic method comprising:
a) providing a sample from a subject; and
b) measuring the level in said sample of a molecule selected from among the group consisting of IKKi protein, IKKi transcript, phosphorylated insulin receptor, and phosphorylated IKKi wherein said IKKi phosphorylation is mediated by TLR4; and
c) determining if the subject has,or has an elevated risk for, a condition associated with impaired insulin receptor signaling, wherein an elevated level of said molecule measured in step b indicates that said subject has, or is at elevated risk for, said condition.

6. The method of claim 5, wherein said measuring comprises the use of an agent specific to said molecule, said agent selected from the group consisting of a nucleic acid probe, a non-phospho-specific antibody, and a phospho-specific antibody.

7. The method of claim 5, wherein said level of said molecule is compared to a standard, wherein said standard is either known to be associated with said condition or is from a healthy individual without said condition.

8. A method of identifying an IKKi-inhibiting agent comprising:
a) combining a polypeptide comprising IKKi, a polypeptide comprising an insulin receptor, labeled phosphorous atoms, and a candidate IKKi inhibiting agent under conditions sufficient to promote phosphorylation of said insulin receptor by said IKKi polypeptide in absence of said candidate inhibitor; and
b) determining the activity of said IKKi polypeptide with regard to phosphorylation of said insulin receptor.

9. A method of identifying an IKKi-inhibiting agent comprising:
a) providing a cell or cell lysate comprising insulin receptors; and
b) contacting said cell with a candidate IKKi inhibitor; and
c) determining whether said candidate IKKi inhibitor affected a property selected from the group consisting of the rate of glucose metabolism and the level of phosphorylation of said insulin receptors.

10. The method of claim 9, wherein said determination of whether said IKKi inhibitor affects said rate of glucose metabolism comprises measuring a feature selected from the group consisting of uptake of glucose by said cell, the phosphorylation state of insulin receptors, the phosphorylation state of APS, the phosphorylation state of Cb1, the phosphorylation state of TC10, the ability of GLUT4 to transport glucose, the translocation of GLUT4 to the plasma membrane, and the size of said cell relative to a control cell.

11. The method of claim 9, wherein said cell is treated with an IKKi-inducing agent prior to said contacting step.

12. The method of claim 5, wherein said impaired insulin signaling occurs in a location selected from the group consisting of adipocyte cells, adipose tissue macrophage cells, adipose tissue, liver cells, and liver tissue.

13. The method of claim 5, wherein said subject is experiencing or is at risk of experiencing a condition selected from the group consisting of obesity, diabetes, and insulin resistance.

## Patentansprüche

1. IKKi-inhibierendes Agens in einer therapeutisch wirksamen Dosis zur Verwendung in der Behandlung eines Subjektes, das von Übergewicht oder einer adipösen Körperzusammensetzung betroffen ist oder hierfür ein Risiko aufweist, wobei die Verwendung in einer Reduzierung von oder einer Prävention einer Zunahme an Körperfett in dem Subjekt resultiert.

2. IKKi-inhibierendes Agens nach Anspruch 1 zur Verwendung gemäß Anspruch 1, wobei die Verwendung ein Ergebnis zur Folge hat, das ausgewählt ist aus der Gruppe bestehend aus einer Erhöhung des Glukosemetabolismus, Reduzierung von Körperfett, einem Ausbleiben einer Zunahme an Körperfett, einer Erhöhung der Insulinrezeptorsignalgebung, einem erniedrigten Spiegel von Insulinrezeptorphosphorylierung, einer Reduzierung der oder Prävention von chronischer Entzündung in der Leber, Reduzierung der oder Prävention von chronischer Entzündung in adipösem Gewebe, Reduzierung der oder Prävention von Lebersteatose, Förderung des metabolischen Energieverbrauchs, Reduzierung der Zirkulation von freien Fettsäuren, und Reduzierung von Cholesterin.

3. IKKi-inhibierendes Agens nach Anspruch 1 zur Verwendung gemäß Anspruch 1, wobei die IKKi-vermittelte Phosphorylierung von IKB in dem Subjekt durch das IKKi-inhibierende Agens unbeeinflusst ist.

4. IKKi-inhibierendes Agens nach Anspruch 1 zur Verwendung gemäß Anspruch 1, das ausgewählt ist aus der Gruppe bestehend aus Benzimidazol-substituierten Thiophen, einer siRNA, einem Gegensinn-Oligonukleotid, einem nicht-phosphospezifischen Anti-IKKi-Antikörper, und einem phosphospezifischen Anti-IKKi-Antikörper.

5. Diagnostisches Verfahren, das Folgendes aufweist:
a) Bereitstellen einer Probe von einem Subjekt; und
b) Messen des Spiegels von einem Molekül in der Probe, das ausgewählt ist aus der Gruppe bestehend aus IKKi-Protein, IKKi-Transkript, phosphoryliertem Insulinrezeptor und phosphoryliertem IKKi, wobei die IKKi-Phosphorylierung durch TLR4 vermittelt wird; und
c) Bestimmen, ob das Subjekt von einem Zustand betroffen ist oder hierfür ein erhöhtes Risiko aufweist, der mit einer beeinträchtigten Insulinrezeptorsignalgebung assoziiert ist, wobei ein in Schritt b gemessener erhöhter Spiegel des Moleküls indiziert, dass das Subjekt den Zustand hat oder ein erhöhtes Risiko hierfür aufweist.

6. Verfahren nach Anspruch 5, wobei das Messen die Verwendung eines Agens umfasst, das spezifisch ist für das Molekül, wobei das Agens ausgewählt ist aus der Gruppe bestehend aus einer Nukleinsäuresonde, einem nicht-phosphorspezifischen Antikörper und einem phosphorspezifischen Antikörper.

7. Verfahren nach Anspruch 5, wobei der Spiegel des Moleküls mit einem Standard verglichen wird, wobei für den Standard bekannt ist, dass dieser mit dem Zustand assoziiert ist oder aus einem gesunden Individuum ohne diesen Zustand stammt.

8. Verfahren zum Identifizieren eines IKKi-inhibierenden Agens, das Folgendes aufweist:
a) Kombinieren eines Polypeptids, das IKKi aufweist, eines Polypeptids, das einen Insulinrezeptor aufweist, markierten Phosphoratomen und einem Kandidaten für ein IKKi-inhibierendes Agens unter Bedingungen, die ausreichend sind, um die Phosphorylierung des Insulinrezeptors durch das IKKi-Polypeptid in Abwesenheit des Inhibitorkandidaten zu fördern; und
b) Bestimmen der Aktivität des IKKi-Polypeptids in Bezug auf die Phosphorylierung des Insulinrezeptors.

9. Verfahren zum Identifizieren eines IKKi-inhibierenden Agens, das Folgendes aufweist:
a) Bereitstellen einer Zelle oder eines Zelllysats, welche(s) Insulinrezeptoren aufweist; und
b) In-Kontakt-Bringen der Zelle mit einem Kandidaten für einen IKKi-Inhibitor; und
c) Feststellen, ob der Kandidat für einen IKKi-Inhibitor eine Eigenschaft beeinflusst, die ausgewählt ist aus der Gruppe bestehend aus der Rate des Glukosemetabolismus und dem Spiegel der Phosphorylierung der Insulinrezeptoren.

10. Verfahren nach Anspruch 9, wobei die Bestimmung, ob der IKKi-Inhibitor die Rate des Glukosemetabolismus beeinflusst, das Messen eines Merkmals umfasst, das ausgewählt ist aus der Gruppe bestehend aus der Aufnahme von Glukose durch die Zelle, dem Phosphorylierungsstatus des Insulinrezeptors, dem Phosphorylierungsstatus von APS, dem Phosphorylierungsstatus von Cb1, dem Phosphorylierungsstatus von TC10, der Fähigkeit von GLUT4, Glukose zu transportieren, der Translokation von GLUT4 zu der Plasmamembran, und der Größe der Zelle relativ zu einer Kontrollzelle.

11. Verfahren nach Anspruch 9, wobei die Zelle mit einem IKKi-induzierenden Agens vor dem Schritt des In-Kontakt-Bringens behandelt wird.

12. Verfahren nach Anspruch 5, wobei die beeinträchtigte Insulinsignalgebung in einem Bereich erfolgt, der ausgewählt ist aus der Gruppe bestehend aus Adipozytenzellen, Makrophagenzellen, adipösem Gewebe, Leberzellen und Lebergewebe.

13. Verfahren nach Anspruch 5, wobei das Subjekt von einem Zustand betroffen ist oder hierfür ein Risiko aufweist, der ausgewählt ist aus der Gruppe bestehend aus Fettleibigkeit, Diabetes und Insulinresistenz.

## Revendications

1. Agent inhibiteur d'IKKi à une dose thérapeutiquement efficace pour une utilisation dans le traitement d'un sujet souffrant ou à risque d'une composition corporelle en surpoids ou obèse, où ladite utilisation conduit à une réduction de la graisse corporelle ou une prévention de l'augmentation de celle-ci chez ledit sujet.

2. Agent inhibiteur d'IKKi de la revendication 1 pour une utilisation selon la revendication 1, dans lequel ladite utilisation conduit à un résultat choisi dans le groupe constitué de l'augmentation de métabolisme du glucose, de la réduction de la graisse corporelle, du manque d'augmentation de la graisse corporelle, de l'augmentation de la signalisation de récepteur de l'insuline, de la diminution de niveau de phosphorylation de récepteur de l'insuline, de la réduction ou de la prévention de l'inflammation chronique dans le foie, de la réduction ou de la prévention de l'inflammation chronique dans le tissu adipeux, de la réduction ou de la prévention de la stéatose hépatique, de la dépense d'énergie métabolique favorisée, de la réduction de circulation des acides gras libres, et de la réduction du cholestérol.

3. Agent inhibiteur d'IKKi de la revendication 1 pour une utilisation selon la revendication 1, dans lequel la phosphorylation médiée par IKKi de IKB chez ledit sujet n'est pas affectée par ledit agent inhibiteur d'IKKi.

4. Agent inhibiteur d'IKKi de la revendication 1 pour une utilisation selon la revendication 1 choisi dans le groupe constitué d'un thiophène à substitution benzimidazole, d'un ARNsi, d'un oligonucléotide antisens, d'un anticorps anti-IKKi non phospho-spécifique, et d'un anticorps anti-IKKi phospho-spécifique.

5. Procédé de diagnostic comprenant le fait :
a) de fournir un échantillon à partir d'un sujet ; et
b) de mesurer le niveau dans ledit échantillon d'une molécule choisie parmi le groupe constitué d'une protéine IKKi, d'un produit de transcription d'IKKi, d'un récepteur de l'insuline phosphorylé, et de l'IKKi phosphorylé où ladite phosphorylation d'IKKi est médiée par TLR4 ; et
c) de déterminer si le sujet présente une affection associée à une signalisation de récepteur de l'insuline altérée ou présente un risque élevé de souffrir de celle-ci, où un niveau élevé de ladite molécule mesuré à l'étape b indique que ledit sujet présente ladite affection, ou présente un risque élevé de souffrir de celle-ci.

6. Procédé de la revendication 5, dans lequel ladite mesure comprend l'utilisation d'un agent spécifique de ladite molécule, ledit agent étant choisi dans le groupe constitué d'une sonde d'acide nucléique, d'un anticorps non phospho-spécifique, et d'un anticorps phospho-spécifique.

7. Procédé de la revendication 5, dans lequel ledit niveau de ladite molécule est comparé à un standard, où ledit standard est soit connu pour être associé à ladite affection ou provient d'un individu sain sans ladite affection.

8. Procédé d'identification d'un agent inhibiteur d'IKKi comprenant le fait :
a) de combiner un polypeptide comprenant IKKi, un polypeptide comprenant un récepteur de l'insuline, des atomes de phosphore marqués, et un agent inhibiteur d'IKKi candidat dans des conditions suffisantes pour favoriser la phosphorylation dudit récepteur de l'insuline par ledit polypeptide d'IKKi en l'absence dudit inhibiteur candidat ; et
b) de déterminer l'activité dudit polypeptide d'IKKi par rapport à la phosphorylation dudit récepteur de l'insuline.

9. Procédé d'identification d'un agent inhibiteur d'IKKi comprenant le fait :
a) de fournir une cellule ou un lysat cellulaire comprenant des récepteurs de l'insuline ; et
b) de mettre ladite cellule en contact avec un inhibiteur d'IKKi candidat ; et
c) de déterminer si ledit inhibiteur d'IKKi candidat a affecté une propriété choisie dans le groupe constitué de la vitesse de métabolisme du glucose et du niveau de phosphorylation desdits récepteurs de l'insuline.

10. Procédé de la revendication 9, dans lequel ladite détermination quant à savoir si ledit inhibiteur d'IKKi affecte ladite vitesse de métabolisme du glucose comprend le fait de mesurer une caractéristique choisie dans le groupe constitué de l'absorption de glucose par ladite cellule, de l'état de phosphorylation de récepteurs de l'insuline, de l'état de phosphorylation d'APS, de l'état de phosphorylation de Cb1, de l'état de phosphorylation de TC10, de la capacité de GLUT4 à transporter le glucose, de la translocation de GLUT4 vers la membrane plasmique, et de la taille de ladite cellule par rapport à une cellule témoin.

11. Procédé de la revendication 9, dans lequel ladite cellule est traitée avec un agent inducteur d'IKKi avant ladite étape de mise en contact.

12. Procédé de la revendication 5, dans lequel ladite signalisation de l'insuline altérée se produit dans un emplacement choisi dans le groupe constitué de cellules adipocytes, de cellules macrophages du tissu adipeux, du tissu adipeux, de cellules hépatiques et de tissu hépatique.

13. Procédé de la revendication 5, dans lequel ledit sujet souffre d'une affection choisie dans le groupe constitué de l'obésité, du diabète et de la résistance à l'insuline ou présente un risque de souffrir de celle-ci.
